# EUROPÄISCHE PATENTSCHRIFT

(11) **EP 3 975 982 B1**
(45) Veröffentlichungstag und Bekanntmachung des Hinweises auf die Patenterteilung: **31.05.2023**
(21) Anmeldenummer: 20715848.6
(22) Anmeldetag: 30.03.2020
(51) Int. Cl.: A61K 8/34, A61K 8/58, A61Q 5/06

(54) **ERHÖHUNG DER STABILITÄT VON MITTELN ZUR BEHANDLUNG VON KERATINMATERIAL**
INCREASING THE STABILITY OF AGENTS FOR TREATING KERATIN MATERIAL
RENFORCEMENT DE LA STABILITÉ D'AGENTS UTILISÉS POUR TRAITER DE LA MATIÈRE KÉRATINIQUE

(30) Priorität: 29.05.2019 DE 102019207896
(43) Veröffentlichungstag der Anmeldung: 06.04.2022
(73) Patentinhaber: Henkel AG & Co. KGaA, 40589 Düsseldorf (DE)
(72) Erfinder: SCHUMACHER, Ulrike, 40589 Düsseldorf (DE); SCHOEPGENS, Juergen, 41366 Schwalmtal (DE); NOWOTTNY, Marc, 41069 Mönchengladbach (DE); KRIENER, Caroline, 40229 Düsseldorf (DE); KOLONKO, Claudia, 42857 Remscheid (DE); JAISER, Phillip, 40764 Langenfeld (DE); MATHIASZYK, Carsten, 45277 Essen (DE); LECHNER, Torsten, 40764 Langenfeld (DE); WESER, Gabriele, 41472 Neuss (DE)
(86) Internationale Anmeldenummer: PCT/EP2020/058933
(87) Internationale Veröffentlichungsnummer: WO 2020/239291

(56) Entgegenhaltungen:
- FR-B1- 2 936 413
- US-A1- 2005 214 236
- US-A1- 2010 297 049

## Beschreibung

Die vorliegende Anmeldung liegt auf dem Gebiet der Kosmetik und betrifft ein Verfahren zur Behandlung von keratinischem Material, insbesondere menschlichen Haaren, welches die Anwendung von zwei Zusammsetzungen (A) und (B) umfasst. Bei der Zusammensetzung (A) handelt es sich um eine wasserarme Zubereitung, die mindestens ein organisches C1-C₆-Alkoxysilan enthält, und die Zusammensetzung (B) beinhaltet neben Wasser mindestens einen Fettbestandteil sowie mindestens eine farbgebende Verbindung. Das erfindungsgemäße Verfahren ist gekennzeichnet durch die Herstellung der Zubereitungen (A) und (B), ihre Lagerung über einen bestimmten Mindestzeitraum, ihr Vermischen und die Anwendung dieser Abmischung auf dem Keratin material.

Die Veränderung von Form und Farbe von keratinischen Fasern, insbesondere von Haaren, stellt einen wichtigen Bereich der modernen Kosmetik dar. Zur Veränderung der Haarfarbe kennt der Fachmann je nach Anforderung an die Färbung diverse Färbesysteme. Für permanente, intensive Färbungen mit guten Echtheitseigenschaften und guter Grauabdeckung werden üblicherweise Oxidationsfärbemittel verwendet. Solche Färbemittel enthalten üblicherweise Oxidationsfarbstoffvorprodukte, sogenannte Entwicklerkomponenten und Kupplerkomponenten, die unter dem Einfluss von Oxidationsmitteln wie beispielsweise Wasserstoffperoxid untereinander die eigentlichen Farbstoffe ausbilden. Oxidationsfärbemittel zeichnen sich durch sehr langanhaltende Färbeergebnisse aus.

Bei dem Einsatz von direktziehenden Farbstoffen diffundieren bereits fertig ausgebildete Farbstoffe aus dem Färbemittel in die Haarfaser hinein. Im Vergleich zur oxidativen Haarfärbung weisen die mit direktziehenden Farbstoffen erhaltenen Färbungen eine geringere Haltbarkeit und schnellere Auswaschbarkeit auf. Färbungen mit direktziehenden Farbstoffen verbleiben üblicherweise für einen Zeitraum zwischen 5 und 20 Haarwäschen auf dem Haar.

Für kurzzeitige Farbveränderungen auf dem Haar und/oder der Haut ist der Einsatz von Farbpigmenten bekannt. Unter Farbpigmenten werden im Allgemeinen unlösliche, farbgebende Substanzen verstanden. Diese liegen ungelöst in Form kleiner Partikel in der Färbeformulierung vor und lagern sich lediglich von außen auf den Haarfasern und/oder der Hautoberfläche ab. Daher lassen sie sich in der Regel durch einige Wäschen mit tensidhaltigen Reinigungsmitteln wieder rückstandslos entfernen. Unter dem Namen Haar-Mascara sind verschiedene Produkte dieses Typs auf dem Markt erhältlich.

Wünscht sich der Anwender besonders langanhaltende Färbungen, so ist die Verwendung von oxidativen Färbemitteln bislang seine einzige Option. Doch trotz vielfacher Optimierungsversuche lässt sich bei der oxidativen Haarfärbung ein unangenehmer Ammoniakgeruch bzw. Amingeruch nicht vollständig vermeiden. Auch die mit dem Einsatz der oxidativen Färbemittel nach wie vor verbundene Haarschädigung wirkt sich auf das Haar des Anwenders nachteilig aus.

EP 2168633 B1 beschäftigt sich mit der Aufgabenstellung, langanhaltende Haarfärbungen unter Einsatz von Pigmenten zu erzeugen. Die Schrift lehrt, dass sich bei Verwendung einer Kombination aus Pigment, organischer Silicium-Verbindung, hydrophobem Polymer und einem Lösungsmittel auf Haaren Färbungen erzeugen lassen, die gegenüber Schamponierungen besonders widerstandsfähig sind.

Bei den in der EP 2168633 B1 eingesetzten organischen Silicium-Verbindungen handelt es sich um reaktive Verbindungen aus der Klasse der Alkoxy-Silane. Diese Alkoxy-Silane hydrolysieren in Gegenwart von Wasser mit hoher Geschwindigkeit und bilden - abhängig von den jeweils eingesetzten Mengen an Alkoxy-Silan und Wasser - Hydrolyseprodukte und/oder Kondensationsprodukte aus. Der Einfluss der in dieser Reaktion eingesetzten Wassermenge auf die Eigenschaften des Hydrolyse- bzw. Kondensationsproduktes werden beispielsweise in WO 2013068979 A2 beschrieben.

FR 2936413 B1 beschreibt die Verwendung einer kosmetischen Zusammensetzung, die ein hydrolysierbares Silan enthält, zur Vorbehandlung einer Zusammensetzung mit hydrophobem, filmbildendem Polymer, Pigment und flüchtigem Lösungsmittel.

Gegenstand der US 2010/297049 A1 ist ein Verfahren zum Färben bzw. Aufhellen von Keratinfasern, umfassend das in Kontakt bringen der Fasern mit einer ersten Zusammensetzung enthaltend weniger als 10 Gew.-% Wasser und ein Aminosilan und einer zweiten Zusammensetzung enthaltend ein Oxidationsmittel.

US 2005/214236 A1 betrifft Perlglanzpigmente, deren Oberfläche mit einem Aminosilan behandelt wird.

Wenn diese Alkoxy-Silane bzw. ihre Hydrolyse- bzw. Kondensationsprodukte auf keratinischem Material angewendet werden, bildet sich auf dem Keratinmaterial ein Film oder auch ein Coating aus, welches das Keratinmaterial vollständig umhüllt und auf diese Weise die Eigenschaften des Keratinmaterials stark beeinflusst. Mögliche Anwendungsbereiche sind beispielsweise das permanente Styling oder auch die permanente Formveränderung von Keratinfasern. Hierbei werden die Keratinfasern mechanisch in die gewünschte Form gebracht und in dieser Form dann durch Ausbildung des vorbeschriebenen Coatings fixiert. Eine weitere ganz besonders gut geeignete Anwendungsmöglichkeit ist die Färbung von Keratinmaterial; im Rahmen dieser Anwendung wird das Coating bzw. der Film in Gegenwart einer farbgebenden Verbindung, zum Beispiel eines Pigments, erzeugt. Der durch das Pigment gefärbte Film verbleibt auf dem Keratinmaterial bzw. den Keratinfasern und resultiert in überraschend waschbeständigen Färbungen.

Der große Vorteil des auf Alkoxy-Silanen basierenden Färbeprinzips liegt darin, dass die hohe Reaktivität dieser Verbindungsklasse ein sehr schnelles Coating ermöglicht. So können bereits nach sehr kurzen Anwendungszeiträumen von nur wenigen Minuten extrem gute Färbeergebnisse erzielt werden. Neben diesen Vorteilen birgt die hohe Reaktivität der Alkoxy-Silane jedoch auch einige Nachteile.

Aufgrund ihrer hohen Reaktivität können die organischen Alkoxy-Silane nicht zusammen mit größeren Wassermengen konfektioniert werden, da ein großer Überschuss an Wasser die sofortige Hydrolyse und im Anschluss daran eine Polymerisation initiiert. Die bei Lagerung der Alkoxy-Silane in wässrigem Medium stattfindende Polymerisierung äußert sich in einer Verdickung oder Gelierung der wässrigen Zubereitung. Hierdurch werden die Zubereitungen so hochviskos, gelig bzw. gallertartig, dass sie nicht mehr gleichmäßig auf dem Keratinmaterial aufgetragen werden können. Zudem ist die Lagerung der Alkoxy-Silane in Gegenwart hoher Wassermengen mit einem Verlust ihrer Reaktivität verbunden, so dass auch die Ausbildung eines widerstandsfähigen Coatings auf dem Keratinmaterial nicht mehr möglich ist.

Aus diesen Gründen ist es notwendig, die organischen Alkoxy-Silane in wasserfreiem bzw. wasserarmem Milieu zu lagern und die entsprechenden Zubereitungen in einem separaten Container zu konfektionieren. Aufgrund ihrer hohen Reaktivität können die Alkoxy-Silane nicht nur mit Wasser, sondern auch mit anderen kosmetischen Inhaltsstoffen reagieren. Zur Vermeidung aller unerwünschten Reaktionen enthalten die Zubereitungen mit Alkoxy-Silanen deshalb bevorzugt keine weiteren Inhaltsstoffe oder nur die ausgewählten Inhaltsstoffe, die sich gegenüber den Alkoxy-Silanen als chemisch inert herausgestellt haben. Die Konzentration der Alkoxy-Silane in der Zubereitung wird dementsprechend bevorzugt verhältnismäßig hoch gewählt. Die wasserarmen Zubereitungen, welche die Alkoxy-Silane in relativ hohen Konzentrationen enthalten, können auch als "Silan-Blend" bezeichnet werden.

Für die Applikation auf dem Keratinmaterial muss der Anwender nun diesen verhältnismäßig hoch konzentrierten Silan-Blend in eine anwendungsbereite Mischung überführen. In dieser anwendungsbereiten Mischung ist zum einen die Konzentration der organischen Alkoxy-Silane erniedrigt, und zum anderen enthält die Anwendungsmischung auch einen höheren Anteil an Wasser (bzw. einem alternativen protischen Inhaltsstoff), durch welchen die zum Coating führende Polymerisation ausgelöst wird.

Es hat sich als extrem große Herausforderung herausgestellt, die Polymerisationsgeschwindigkeit, d.h. die Geschwindigkeit, mit der sich das Coating auf dem Keratinmaterial ausbildet, optimal an die Anwendungsbedingungen anzupassen.

Bei Anwendung auf menschlichen Haaren führt eine zu schnelle Polymerisationsgeschwindigkeit beispielsweise dazu, dass die Polymerisation bereits abgeschlossen ist, bevor alle Haarpartien behandelt werden konnten. Eine zu schnelle Polymerisation macht deshalb die Ganzkopfbehandlung unmöglich. Im Färbeprozess äußert sich die zu schnelle Polymerisation in einem extrem ungleichmäßigen Farbergebnis, so dass die Haarpartien, die zuletzt behandelt wurden, nur mangelhaft gefärbt sind.

Bei einer zu langsam ablaufenden Polymerisation können andererseits zwar alle Areale des Haares ohne Zeitdruck behandelt werden, jedoch steigt hierdurch die Anwendungsdauer. Bei zu langsamer Polymerisation kommt daher der große Vorteil dieser Färbetechnologie, die Ausbildung von waschechten Färbungen innerhalb kürzester Anwendungszeiträume, nicht zum Tragen.

Eine zentrale Aufgabe der vorliegenden Anmeldung war es, ein Verfahren zum Behandeln von keratinischem Material aufzufinden, mittels welchem die Polymerisationsgeschwindigkeit der organischen Alkoxy-silane an die Anwendungsbedingungen, insbesondere an die bei Anwendung auf dem menschlichen Kopf herrschenden Bedingungen, angepasst werden konnte. Mit anderen Worten wurde nach einem Verfahren gesucht, durch welches die organischen Alkoxy-silane so lange reaktiv bleiben, dass eine Ganzkopfbehandlung ermöglicht wird, ohne den Anwendungszeiträum über Gebühr zu verlängern.

In ersten Versuchsreihen hat sich überraschenderweise herausgestellt, dass diese Aufgabe sehr gut gelöst werden kann, wenn das Keratinmaterial in einem Verfahren behandelt wird, bei dem zwei Zusammensetzungen (A) und (B) auf dem Keratinmaterial angewendet werden. Bei der ersten Zusammensetzung (A) handelt es sich um den zuvor beschriebenen wasserarmen Silan-Blend. Die zweite Zusammensetzung (B) ist eine wasserhaltige Emulsion und enthält neben einem Fettbestandteil mindestens eine farbgebende Verbindung. Werden die beiden Zusammensetzungen (A) und (B) miteinander vermischt und in dieser Abmischung auf das keratinische Material aufgetragen, so konnte die Reaktivität des Silan-Blends optimal an die bei einem Ganzkopf-Haarfärbeprozess herrschenden Anwendungsbedingungen angepasst werden. Auch kompliziertere bzw. zeitaufwändigere Färbetechniken wie beispielsweise die Färbung von speziell auf dem Kopf angeordneten Strähnchen, konnten bei Anwendung des erfindungsgemäßen Verfahrens realisiert werden, so dass auf diese Weise Färbungen mit besonders hoher Gleichmäßigkeit erzeugt werden konnten.

Bei weiteren Versuchen hat sich herausgestellt, dass dies zuvor beschriebene Verfahren neben seinen Vorteilen jedoch auch mit einem Nachteil behaftet ist. So hat sich gezeigt, dass der Einsatz des Silan-Blends (A) in Kombination mit einer emulsionsförmigen Zusammensetzung (B) im Vergleich zum Einsatz einer gelförmigen Zusammensetzung (B) wieder zu Färberesultaten mit verschlechterter Waschechtheit führen kann.

Die zweiter wesentliche Aufgabe der vorliegenden Erfindung bestand deshalb in der Bereitstellung eines auf organischen Alkoxy-silanen basierenden Färbesystems, dessen Reaktivität an die bei einer Färbung herrschenden Bedingungen optimal angepasst ist, ohne hierbei Einbußen hinsichtlich seiner Echtheitseigenschaften, insbesondere seiner Waschechtheit, zu erleiden. Im Optimalfall sollte dieses Färbesystem sowohl eine optimale Reaktivität als auch eine verbesserte Waschechtheit besitzen.

In für den Fachmann nicht vorhersehbarer Weise konnte nun herausgefunden werden, dass die Waschechtheit der mit Anwendung der beiden Zusammensetzungen (A) und (B) erhaltenen Färbungen sich dann signifikant verbessern ließ, wenn mindestens eine der Zusammensetzungen (A) und/oder (B) vor ihrer Anwendung auf dem Keratinmaterial eine bestimmte Lagerzeit oder auch Reifezeit durchliefen.

Ein erster Gegenstand der vorliegenden Erfindung ist ein Verfahren zur Herstellung und Anwendung eines Mittels zur Färbung von keratinischem Material, insbesondere menschlichen Haaren, umfassend die folgenden Schritte:
(1) Herstellung einer ersten Zusammensetzung (A), enthaltend
   (A1) weniger als 10 Gew.-% Wasser und
   (A2) ein oder mehrere organische C₁-C₆-Alkoxy-Silane und/oder deren Kondensationsprodukte, und
(2) Herstellung einer zweiten Zusammensetzung (B), enthaltend
   (B1) Wasser und
   (B2) ein oder mehrere Fettbestandteile und
   (B3) ein oder mehrere farbgebende Verbindungen aus der Gruppe der Pigmente und/oder der direktziehenden Farbstoffe,
(3) Lagerung der Zusammensetzung (A) und/oder (B) für einen Zeitraum von mindestens 24 Stunden,
(4) Vermischen der Zusammensetzungen (A) und (B),
(5) Anwendung der Mischung aus (A) und (B) auf dem keratinischen Material.

Überraschenderweise konnte die Reaktivität der organische C₁-C₆-Alkoxy-Silane (A2) so optimal an die bei einem Ganzkopf-Haarfärbeprozess herrschenden Anwendungsbedingungen angepasst werden. Auch kompliziertere bzw. zeitaufwändigere Färbetechniken wie beispielsweise die Färbung von speziell auf dem Kopf angeordneten Strähnchen, konnten bei Anwendung des erfindungsgemäßen Verfahrens realisiert werden. Bei Einsatz der beiden Zusammensetzungen (A) und (B) in dem o.g. Färbeverfahren konnte auf Keratinmaterial, insbesondere auf menschlichen Haaren, auf diese Weise Färbungen mit besonders hoher Gleichmäßigkeit erzeugt werden. Die auf diese Weise erzeugten Färbungen zeichneten sich überraschenderweise ebenfalls durch eine sehr gute Waschechtheit aus.

### Färbung von keratinischem Material

Unter keratinischem Material sind Haare, die Haut, die Nägel (wie beispielsweise Fingernägel und/oder Fußnägel) zu verstehen. Weiterhin fallen auch Wolle, Pelze und Federn unter die Definition des keratinischen Materials.

Bevorzugt werden unter keratinischem Material das menschliche Haar, die menschliche Haut und menschliche Nägel, insbesondere Finger- und Fußnägel, verstanden. Ganz besonders bevorzugt wird unter keratinischem Material das menschliche Haar verstanden.

Unter Mitteln zur Behandlung von keratinischem Material werden beispielsweise Mittel zur Färbung des Keratinmaterials, Mittel zur Umformung oder Formgebung von keratinischem Material, insbesondere keratinischen Fasern, oder auch Mittel zur Konditionierung bzw. zur Pflege des keratinischen Materials verstanden. Besonders gute Eignung zeige die über das erfindungsgemäße Verfahren hergestellten Mittel zur Färbung von keratinischem Material, insbesondere zur Färbung von keratinischen Fasern, bei denen es sich besonders bevorzugt um menschliche Haare handelt.

Der Begriff "Mittel zur Färbung" wird im Rahmen dieser Erfindung für eine durch Einsatz von farbgebenden Verbindungen, wie beispielsweise Pigmenten, Mica, direktziehenden Farbstoffen und/oder thermochromen und photochromen Farbstoffen hervorgerufene Farbgebung des Keratinmaterials, insbesondere des Haares, verwendet. Bei dieser Färbung lagern sich die vorgenannten farbgebenden Verbindungen in einem besonders homogenen und glatten Film an der Oberfläche des Keratinmaterials ab oder diffundieren in die Keratinfaser hinein. Der Film bildet sich *in situ* durch Oligomerisierung bzw. Polymerisierung des oder der organischen Alkoxy-Silane, und durch die Wechselwirkung von farbgebender Verbindung und organischer Siliciumverbindung und optional weiteren Bestandteilen, wie beispielsweise einem filmbildenden, Polymer.

### Zusammensetzung (A)

Schritt (1) des erfindungsgemäßen Verfahrens ist gekennzeichnet durch die Herstellung der ersten Zusammensetzung (A). Die Zusammensetzung (A) enthält weniger als 10 Gew.-% Wasser (A1 und ein oder mehrere organische C₁-C₆-Alkoxy-Silane und/oder deren Kondensationsprodukte (A2).

### Wassergehalt (A1) in der Zusammensetzung (A)

Zur Gewährleistung einer ausreichend hohen Lagerstabilität ist die Zusammensetzung (A) dadurch gekennzeichnet, dass die wasserarm, bevorzugt im wesentlichen wasserfrei, ist. Deshalb enthält die Zusammensetzung (A) - bezogen auf das Gesamtgewicht der Zusammensetzung (A) - weniger als 10 Gew.-% Wasser.

Bei einem Wassergehalt von knapp unter 10 Gew.-% sind die Zusammensetzungen (A) über längere Zeiträume lagerstabil. Um die Lagerstabilität noch weiter zu verbessern und um eine ausreichend hohe Reaktivität der organische C₁-C₆-Alkoxy-Silane (A2) zu gewährleisten, hat es sich jedoch als besonders bevorzugt herausgestellt, den Wassergehalt in der Zusammensetzung (A) noch weiter herabzusenken. Aus diesem Grund enthält erste Zusammensetzung (A) - bezogen auf das Gesamtgewicht der Zusammensetzung (A) - bevorzugt 0,01 bis 9,5 Gew.-%, weiter bevorzugt 0,01 bis 8,0 Gew.-%, noch weiter bevorzugt 0,01 bis 6,0 und ganz besonders bevorzugt 0,01 bis 4,0 Gew.-% Wasser (A1).

Im Rahmen einer ganz besonders bevorzugten Ausführungsform ist ein erfindungsgemäßes Verfahren dadurch gekennzeichnet, dass die erste Zusammensetzung (A) - bezogen auf das Gesamtgewicht der Zusammensetzung (A) - 0,01 bis 9,5 Gew.-%, bevorzugt 0,01 bis 8,0 Gew.-%, weiter bevorzugt 0,01 bis 6,0 und ganz besonders bevorzugt 0,01 bis 4,0 Gew.-% Wasser (A1) enthält.

### Organische C₁-C₆-Alkoxy-Silane (A2) und/oder deren Kondensationsprodukte in der Zusammensetzung (A)

Als zweiten erfindungswesentlichen Bestandteil enthält die Zusammensetzung (A) ein oder mehrere organische C₁-C₆-Alkoxy-Silane (A2) und/oder deren Kondensationsprodukte.

Bei dem oder den organischen C₁-C₆-Alkoxy-Silanen handelt es sich um organische, nicht polymere Siliciumverbindungen, die bevorzugt aus der Gruppe der Silane mit einem, zwei oder drei Siliciumatomen ausgewählt sind

Organische Siliciumverbindungen, die alternativ auch als siliciumorganische Verbindungen bezeichnet werden, sind Verbindungen, die entweder eine direkte Silicium-Kohlenstoff-Bindung (Si-C) aufweisen oder in denen der Kohlenstoff über ein Sauerstoff-, Stickstoff- oder Schwefel-Atom an das Silicium-Atom geknüpft ist. Die erfindungsgemäßen organische Siliciumverbindungen sind bevorzugt Verbindungen, die ein bis drei Silicumatome enthalten. Besonders bevorzugt enthalten die organische Siliciumverbindungen ein oder zwei Siliciumatome.

Die Bezeichnung Silan steht nach den IUPAC-RegeIn für eine Stoffgruppe chemischer Verbindungen, die auf einem Silicium-Grundgerüst und Wasserstoff basieren. Bei organischen Silanen sind die Wasserstoff-Atome ganz oder teilweise durch organische Gruppen wie beispielsweise (substituierte) Alkylgruppen und/oder Alkoxygruppen ersetzt.

Kennzeichnend für die erfindungsgemäßen C₁-C₆-Alkoxy-Silane ist, dass mindestens eine C₁-C₆-Alkoxygruppe direkt an ein Siliciumatom gebunden vorliegt. Die erfindungsgemäßen C₁-C₆-Alkoxy-Silane umfassen damit mindestens eine Struktureinheit R'R"R‴Si-O-(C₁-C₆-Alkyl) wobei die Reste R', R" und R‴ für die drei übrigen Bindungsvalenzen des Siliciumatoms stehen.

Das oder diese an das Siliciumatom gebundenen C₁-C₆-Alkoxygruppen sind sehr reaktiv und werden in Anwesenheit von Wasser mit hoher Geschwindigkeit hydrolysiert, wobei die Reaktionsgeschwindigkeit unter anderem auch von der Anzahl der hydrolysierbaren Gruppen pro Molekül abhängt. Handelt es sich bei der hydrolysierbaren C₁-C₆-Alkoxy-Gruppe um eine Ethoxygruppe, so enthält die organische Siliciumverbindung bevorzugt eine Struktureinheit R'R"R‴Si-O-CH2-CH3. Die Reste R', R" und R‴ stellen wieder die drei übrigen freien Valenzen des Siliciumatoms dar.

Bereits der Zusatz geringer Wassermengen führt zunächst zur Hydrolyse und dann zu einer Kondensationsreaktion der organischen Alkoxy-silane untereinander. Aus diesem Grund können sowohl die organischen Alkoxy-silane (A2) als auch deren Kondensationsprodukte in der Zusammensetzung enthalten sein.

Unter einem Kondensationsprodukt wird ein Produkt verstanden, dass durch Reaktion von mindestens zwei organischen C₁-C₆-Alkoxy-Silanen unter Abspaltung von Wasser und/oder unter Abspaltung von einem C₁-C₆-Alkanol entsteht.

Die Kondensationsprodukte können beispielsweise Dimere, aber auch Trimere oder Oligomere sein, wobei die Kondensationsprodukte mit den Monomeren im Gleichgewicht stehen.

Abhängig von der eingesetzten bzw. in der Hydrolyse verbrauchten Wassermenge verschiebt sich das Gleichgewicht von monomerem C₁-C₆-Alkoxysilan zu Kondensationsprodukt.

Im Rahmen einer ganz besonders bevorzugten Ausführungsform ist ein erfindungsgemäßes Verfahren dadurch gekennzeichnet, dass die Zusammensetzung (A) ein oder mehrere organische C₁-C₆-Alkoxy-Silane (A2) enthält, die aus Silanen mit einem, zwei oder drei Siliciumatomen ausgewählt sind, wobei die organische Siliciumverbindung außerdem eine oder mehrere basische chemische Funktionen umfasst.

Bei dieser basischen Gruppe kann es sich beispielsweise um eine Aminogruppe, eine Alkylaminogruppe oder um eine Dialkylaminogruppe handeln, die bevorzugt über einen Linker mit einem Siliciumatom verbunden ist. Bevorzugt handelt es sich bei der basischen Gruppe um eine Aminogruppe, eine C₁-C₆-Alkylaminogruppe oder um eine Di(C₁-C₆)alkylaminogruppe.

Ein ganz besonders bevorzugtes erfindungsgemäßes Verfahren ist dadurch gekennzeichnet, dass die Zusammensetzung (A) ein oder mehrere organische C₁-C₆-Alkoxy-Silane (A2) enthält, die ausgewählt sind aus der Gruppe der Silane mit einem, zwei oder drei Siliciumatomen, und wobei die C₁-C₆-Alkoxy-Silane weiterhin eine oder mehrere basische chemische Funktionen umfassen.

Ganz besonders gute Ergebnisse konnten erhalten werden, wenn im erfindungsgemäßen Verfahren C₁-C₆-Alkoxy-Silane der Formel (S-I) und/oder (S-II) eingesetzt wurden. Da wie bereits zuvor beschrieben bereits bei Spuren von Feuchtigkeit eine Hydrolyse/Kondensation einsetzt, sind auch die Kondensationsprodukte der C₁-C₆-Alkoxy-Silane der Formel (S-I) und/oder (S-II) von dieser Ausführungsform mit umfasst.

In einer weiteren ganz besonders bevorzugten Ausführungsform ist ein erfindungsgemäßes Verfahren dadurch gekennzeichnet, dass die erste Zusammensetzung (A) ein oder mehrerer organische C₁-C₆-Alkoxy-Silane (A2) der Formel (S-I) und/oder (S-II) enthält,

R₁R₂N-L-Si(OR₃)ₐ(R₄)_{b} (S-I)

wobei
- R₁, R₂ unabhängig voneinander für ein Wasserstoffatom oder eine C₁-C₆-Alkylgruppe stehen,
- L für eine lineare oder verzweigte, zweiwertige C₁-C₂₀-Alkylengruppe steht,
- R₃, R₄ unabhängig voneinander für eine C₁-C₆-Alkylgruppe stehen,
- a, für eine ganze Zahl von 1 bis 3 steht, und
- b für die ganze Zahl 3 - a steht, und

   (R₅O)_{c}(R₆)_{d}Si-(A)ₑ-[NR₇-(A')]_{f}-[O-(A")]_{g}-[NR₈-(A‴)]ₕ-Si(R₆')_{d'}(OR₅')_{c'} (S-II),

   wobei
- R5, R5', R5", R6, R6' und R6" unabhängig voneinander für eine C₁-C₆-Alkylgruppe stehen,
- A, A', A", A‴ und A"" unabhängig voneinander für eine lineare oder verzweigte, zweiwertige C₁-C₂₀-Alkylengruppe stehen,
- R₇ und R₈ unabhängig voneinander für ein Wasserstoffatom, eine C₁-C₆-Alkylgruppe, eine Hydroxy-C₁-C₆-alkylgruppe, eine C₂-C₆-Alkenylgruppe, eine Amino-C₁-C₆-alkylgruppe oder eine Gruppierung der Formel (S-III) stehen,
- (Aʺʺ)-Si(R₆")_{d}"(OR₅")_{c}" (S-III),
- c, für eine ganze Zahl von 1 bis 3 steht,
- d für die ganze Zahl 3 - c steht,
- c' für eine ganze Zahl von 1 bis 3 steht,
- d' für die ganze Zahl 3 - c` steht,
- c" für eine ganze Zahl von 1 bis 3 steht,
- d" für die ganze Zahl 3 - c" steht,
- e für 0 oder 1 steht,
- f für 0 oder 1 steht,
- g für 0 oder 1 steht,
- h für 0 oder 1 steht,
- mit der Maßgabe, dass mindestens einer der Reste aus e, f, g und h von 0 verschieden ist,
und/oder deren Kondensationsprodukte.

Die Substituenten R₁, R₂, R₃, R₄, R₅, R₅', R₅", R₆, R₆', R₆", R₇, Rs, L, A, A', A", A‴ und Aʺʺ in den Verbindungen der Formel (S-I) und (S-II) sind nachstehend beispielhaft erläutert:
Beispiele für eine C₁-C₆-Alkylgruppe sind die Gruppen Methyl, Ethyl, Propyl, Isopropyl, n-Butyl, s-Butyl und t-Butyl, n-Pentyl und n-Hexyl. Propyl, Ethyl und Methyl sind bevorzugte Alkylreste. Beispiele für eine C₂-C₆-Alkenylgruppe sind Vinyl, Allyl, But-2-enyl, But-3-enyl sowie Isobutenyl, bevorzugte C₂-C₆-Alkenylreste sind Vinyl und Allyl. Bevorzugte Beispiele für eine Hydroxy-C₁-C₆-alkylgruppe sind eine Hydroxymethyl-, eine 2-Hydroxyethyl-, eine 2-Hydroxypropyl, eine 3-Hydroxypropyl-, eine 4-Hydroxybutylgruppe, eine 5-Hydroxypentyl- und eine 6-Hydroxyhexylgruppe; eine 2-Hydroxyethylgruppe ist besonders bevorzugt. Beispiele für eine Amino-C₁-C₆-alkyl-gruppe sind die Aminomethylgruppe, die 2-Aminoethylgruppe, die 3-Aminopropylgruppe. Die 2-Aminoethylgruppe ist besonders bevorzugt. Beispiele für eine lineare zweiwertige C₁-C₂₀-Alkylengruppe sind beispielsweise die Methylen-gruppe (-CH₂-), die Ethylengruppe (-CH₂-CH₂-), die Propylengruppe (-CH₂-CH₂-CH₂-) und die Butylengruppe (-CH₂-CH₂-CH₂-CH₂-). Die Propylengruppe (-CH₂-CH₂-CH₂-) ist besonders bevorzugt. Ab einer Kettenlänge von 3 C-Atomen können zweiwertige Alkylengruppen auch verzweigt sein. Beispiele für verzweigte, zweiwertige C₃-C₂₀-Alkylengruppen sind (-CH₂-CH(CH₃)-) und (-CH₂-CH(CH₃)-CH₂-).

In den organischen Siliciumverbindungen der Formel (S-I)

R₁R₂N-L-Si(OR₃)ₐ(R₄)_{b} (S-I),

stehen die Reste R₁ und R₂ unabhängig voneinander für ein Wasserstoffatom oder eine C₁-C₆-Alkylgruppe. Ganz besonders bevorzugt stehen die Reste R₁ und R₂ beide für ein Wasserstoffatom.

Im Mittelteil der organischen Siliciumverbindung befindet sich die Struktureinheit oder der Linker -L-der für eine lineare oder verzweigte, zweiwertige C₁-C₂₀-Alkylengruppe steht. Die zweiwertige C₁-C₂₀-Alkylengruppe kann alternativ auch als eine divalente oder zweibindige C₁-C₂₀-Alkylengruppe bezeichnet werden, womit gemeint ist, dass jede Gruppierung -L- zwei Bindungen eingehen kann.

Bevorzugt steht -L- für eine lineare, zweiwertige C₁-C₂₀-Alkylengruppe. Weiter bevorzugt steht -L- für eine lineare zweiwertige C₁-C₆-Alkylengruppe. Besonders bevorzugt steht -L- für eine Methylengruppe (-CH₂-), eine Ethylengruppe (-CH₂-CH₂-), eine Propylengruppe (-CH₂-CH₂-CH₂-) oder eine Butylengruppe (-CH₂-CH₂-CH₂-CH₂-). Ganz besonders bevorzugt steht L für eine Propylengruppe (-CH₂-CH₂-CH₂-).

Die erfindungsgemäßen organischen Siliciumverbindungen der Formel (S-I)

R₁R₂N-L-Si(OR₃)ₐ(R₄)_{b} (S-I),

tragen jeweils ein einem Ende die Silicium-haltige Gruppierung -Si(OR₃)ₐ(R₄)_{b}.

In der endständigen Struktureinheit -Si(OR₃)ₐ(R₄)_{b} steht die Reste R3 und R4 unabhängig voneinander für eine C₁-C₆-Alkylgruppe, Besonders bevorzugt stehen R₃ und R₄ unabhängig voneinander für eine Methylgruppe oder eine Ethylgruppe.

Hierbei steht a für eine ganze Zahl von 1 bis 3, und b steht für die ganze Zahl 3 - a. Wenn a für die Zahl 3 steht, dann ist b gleich 0. Wenn a für die Zahl 2 steht, dann ist b gleich 1. Wenn a für die Zahl 1 steht, dann ist b gleich 2.

Keratinbehandlungsmittel mit besonders guten Eigenschaften konnten hergestellt werden, wenn die Zusammensetzung (A) mindestens ein organisches C₁-C₆-Alkoxy-Silan der Formel (S-I) enthält, bei welchem die Reste R₃, R₄ unabhängig voneinander für eine Methylgruppe oder für eine Ethylgruppe stehen.

Weiterhin konnten Färbungen mit den besten Waschechtheiten erhalten werden, wenn die Zusammensetzung (A) mindestens ein organisches C₁-C₆-Alkoxy-Silan der Formel (S-I) enthält, bei welchem der Rest a für die Zahl 3 steht. In diesem Fall steht der Rest b für die Zahl 0.

In einer weiteren bevorzugten Ausführungsform ist ein erfindungsgemäßes Verfahren dadurch gekennzeichnet, dass die Zusammensetzung (A) ein oder mehrere organische C₁-C₆-Alkoxy-Silane der Formel (S-I) enthält,
wobei
- R₃, R₄ unabhängig voneinander für eine Methylgruppe oder für eine Ethylgruppe stehen und
- a für die Zahl 3 steht und
- b für die Zahl 0 steht.

In einer weiteren bevorzugten Ausführungsform ist ein erfindungsgemäßes Verfahren dadurch gekennzeichnet, dass die Zusammensetzung (A) mindestens ein oder mehrere organische C₁-C₆-Alkoxy-Silane der Formel (S-I) enthält,

R₁R₂N-L-Si(OR₃)ₐ(R₄)_{b} (S-I),

wobei
- R₁, R₂ beide für ein Wasserstoffatom stehen, und
- L für eine lineare, zweiwertige C₁-C₆-Alkylengruppe, bevorzugt für eine Propylengruppe (-CH₂-CH₂-CH₂-) oder für eine Ethylengruppe (-CH₂-CH₂-), steht,
- R₃ für eine Ethylgruppe oder eine Methylgruppe steht,
- R₄ für eine Methylgruppe oder für eine Ethylgruppe steht,
- a für die Zahl 3 steht und
- b für die Zahl 0 steht.

Zur Lösung der erfindungsgemäßen Aufgabenstellung besonders gut geeignete organische Silicumverbindungen der Formel (I) sind
- (3-Aminopropyl)triethoxysilan 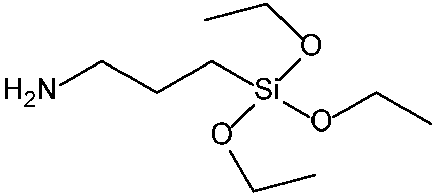
- (3-Aminopropyl)trimethoxysilan 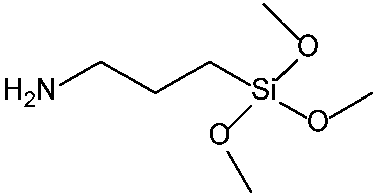
- (2-Aminoethyl)triethoxysilan 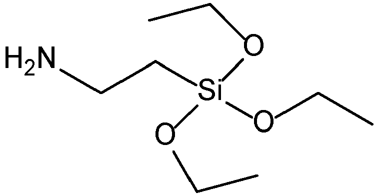
- (2-Aminoethyl)trimethoxysilan 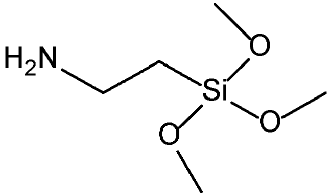
- (3-Dimethylaminopropyl)triethoxysilan 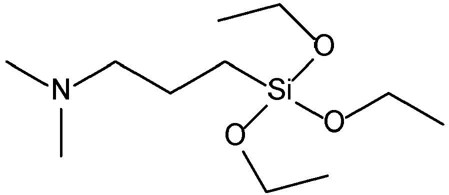
- (3-Dimethylaminopropyl)trimethoxysilan 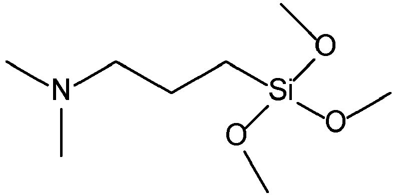
- (2-Dimethylaminoethyl)triethoxysilan. 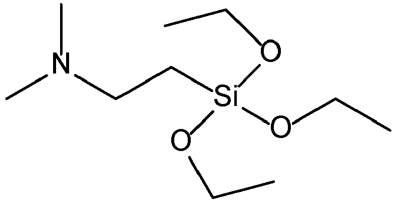
- (2-Dimethylaminoethyl)trimethoxysilan und/oder

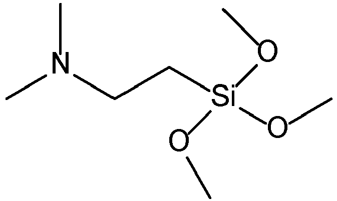

In einer weiteren bevorzugten Ausführungsform ist ein erfindungsgemäßes Verfahren dadurch gekennzeichnet, dass das die erste Zusammensetzung (A) mindestens ein organisches C₁-C₆-Alkoxysilan (A2) der Formel (S-I) enthält, das ausgewählt ist aus der Gruppe aus
- (3-Aminopropyl)triethoxysilan
- (3-Aminopropyl)trimethoxysilan
- (2-Aminoethyl)triethoxysilan
- (2-Aminoethyl)trimethoxysilan
- (3-Dimethylaminopropyl)triethoxysilan
- (3-Dimethylaminopropyl)trimethoxysilan
- (2-Dimethylaminoethyl)triethoxysilan,
- (2-Dimethylaminoethyl)trimethoxysilan
und/oder deren Kondensationsprodukten.

Die vorgenannten organische Siliciumverbindung der Formel (I) sind kommerziell erhältlich. (3-Aminopropyl)trimethoxysilan kann beispielsweise von Sigma-Aldrich käuflich erworben werden. Auch (3-Aminopropyl)triethoxysilan ist kommerziell bei der Firma Sigma-Aldrich erwerblich.

Im Rahmen einer weiteren Ausführungsform des erfindungsgemäßen Verfahrens kann die Zusammensetzung (A) auch ein oder mehrere organische C₁-C₆-Alkoxy-Silane der Formel (S-II) enthalten,

(R₅O)_{c}(R₆)_{d}Si-(A)ₑ-[NR₇-(A')]_{f}-[O-(A")]g-[NR₈-(A‴)]ₕ-Si(R₆')_{d'}(OR₅')_{c'} (S-II).

Die erfindungsgemäßen siliciumorganischen Verbindungen der Formel (S-II) tragen jeweils an ihren beiden Enden die Silicium-haltigen Gruppierungen (R₅O)_{c}(R₆)_{d}Si- und -Si(R₆')_{d'}(OR₅')_{c'}.

Im Mittelteil des Moleküls der Formel (S-II) befinden sich die Gruppierungen -(A)ₑ- und -[NR₇-(A')]_{f}- und -[O-(A")]_{g}- und -[NR₈-(A‴)]ₕ-. Hierbei kann jeder der Reste e, f, g und h unabhängig voneinander für die Zahl 0 oder 1 stehen, wobei die Maßgabe besteht, dass mindestens einer der Reste e, f, g und h von 0 verschieden ist. Mit anderen Worten enthält eine erfindungsgemäße organischen Siliciumverbindung der Formel (II) mindestens eine Gruppierung aus der Gruppe aus -(A)- und - [NR₇-(A')]- und -[O-(A")]- und -[NR₈-(A‴)]-.

In den beiden endständigen Struktureinheiten (R₅O)_{c}(R₆)_{d}Si- und - Si(R₆')_{d'}(OR₅')_{c'}stehen die Reste R5, R5`, R5" unabhängig voneinander für eine C₁-C₆-Alkylgruppe. Die Reste R6, R6' und R6" stehen unabhängig voneinander für eine C₁-C₆-Alkylgruppe.

Hierbei steht c für eine ganze Zahl von 1 bis 3, und d steht für die ganze Zahl 3 - c. Wenn c für die Zahl 3 steht, dann ist d gleich 0. Wenn c für die Zahl 2 steht, dann ist d gleich 1. Wenn c für die Zahl 1 steht, dann ist d gleich 2.

Analog steht c' für eine ganze Zahl von 1 bis 3, und d' steht für die ganze Zahl 3 - c'. Wenn c' für die Zahl 3 steht, dann ist d' gleich 0. Wenn c` für die Zahl 2 steht, dann ist d' gleich 1. Wenn c` für die Zahl 1 steht, dann ist d' gleich 2.

Färbungen mit den besten Waschechtheiten konnten erhalten werden, wenn die Reste c und c` beide für die Zahl 3 stehen. In diesem Fall stehen d und d' beide für die Zahl 0.

In einer weiteren bevorzugten Ausführungsform ist ein erfindungsgemäßes Verfahren dadurch gekennzeichnet, dass die Zusammensetzung (A) ein oder mehrere organische C₁-C₆-Alkoxy-Silane der Formel (S-II) enthält,

(R₅O)_{c}(R₆)_{d}Si-(A)ₑ-[NR₇-(A')]_{f}-[O-(A")]_{g}-[NRa-(A‴)]ₕ-Si(R₆')_{d'}(OR₅')_{c'} (S-II),

wobei
- R5 und R5' unabhängig voneinander für eine Methylgruppe oder eine Ethylgruppe stehen,
- c und c' beide für die Zahl 3 stehen und
- d und d' beide für die Zahl 0 stehen.

Wenn c und c' beide für die Zahl 3 stehen und d und d' beide für die Zahl 0 stehen, entsprechen die erfindungsgemäßen organischen Siliciumverbindung der Formel (S-IIa)

(R₅O)₃Si-(A)e-[NR₇-(A')]_{f}-[O-(A")]_{g}-[NR₈-(A‴)]ₕ-Si(OR₅')₃ (S-IIa).

Die Reste e, f, g und h können unabhängig voneinander für die Zahl 0 oder 1 stehen, wobei mindestens ein Rest aus e, f, g und h von null verschieden ist. Durch die Kürzel e, f, g und h wird demnach definiert, welche der Gruppierungen -(A)ₑ- und -[NR₇-(A')]_{f}- und -[O-(A")]_{g}- und -[NR₈-(A‴)]ₕ-sich im Mittelteil der organischen Siliciumverbindung der Formel (II) befinden.

In diesem Zusammenhang hat sich die Anwesenheit bestimmter Gruppierungen als besonders vorteilhaft im Hinblick auf die Erzielung von waschechten Färbeergebnissen erwiesen. Besonders gute Ergebnisse konnten erhalten werden, wenn mindestens zwei der Reste e, f, g und h für die Zahl 1 stehen. Ganz besonders bevorzugt stehen e und f beide für die Zahl 1. Weiterhin ganz besonders bevorzugt stehen g und h beide für die Zahl 0.

Wenn e und f beide für die Zahl 1 stehen und g und h beide für die Zahl 0 stehen, entsprechen die erfindungsgemäßen organischen Siliciumverbindung der Formel (S-Ilb)

(R₅O)_{c}(R₆)_{d}Si-(A)-[NR₇-(A')]-Si(R₆')_{d'}(OR₅')_{c'} (S-IIb).

Die Reste A, A', A", A‴ und A"" stehen unabhängig voneinander für eine lineare oder verzweigte, zweiwertige C₁-C₂₀-Alkylengruppe. Bevorzugt stehen die Reste A, A', A", A‴ und A"" unabhängig voneinander für eine lineare, zweiwertige C₁-C₂₀-Alkylengruppe. Weiter bevorzugt stehen die Reste A, A', A", A‴ und A"" unabhängig voneinander für eine lineare zweiwertige C₁-C₆-Alkylengruppe.

Die zweiwertige C₁-C₂₀-Alkylengruppe kann alternativ auch als eine divalente oder zweibindige C₁-C₂₀-Alkylengruppe bezeichnet werden, womit gemeint ist, dass jede Gruppierung A, A', A", A‴ und A"" zwei Bindungen eingehen kann.

Besonders bevorzugt stehen die Reste A, A', A", A‴ und A"" unabhängig voneinander für eine Methylengruppe (-CH₂-), eine Ethylengruppe (-CH₂-CH₂-), eine Propylengruppe (-CH₂-CH₂-CH₂-) oder eine Butylengruppe (-CH₂-CH₂-CH₂-CH₂-). Ganz besonders bevorzugt stehen die Reste A, A', A", A‴ und A"" für eine Propylengruppe (-CH₂-CH₂-CH₂-).

Wenn der Rest f für die Zahl 1 steht, dann enthält die erfindungsgemäße organische Siliciumverbindung der Formel (II) eine strukturelle Gruppierung -[NR₇-(A')]-.

Wenn der Rest h für die Zahl 1 steht, dann enthält die erfindungsgemäße organische Siliciumverbindung der Formel (II) eine strukturelle Gruppierung -[NR₈-(A‴)]-.

Hierbei stehen die Reste R₇ und R₈ unabhängig voneinander für ein Wasserstoffatom, eine C₁-C₆-Alkylgruppe, eine Hydroxy-C₁-C₆-alkylgruppe, eine C₂-C₆-Alkenylgruppe, eine Amino-C₁-C₆-alkylgruppe oder eine Gruppierung der Formel (S-III)

- (Aʺʺ)-Si(R₆")_{d}"(OR₅")_{c}" (S-III).

Ganz besonders bevorzugt stehen die Reste R7 und R8 unabhängig voneinander für ein Wasserstoffatom, eine Methylgruppe, eine 2-Hydroxyethylgruppe, eine 2-Alkenylgruppe, eine 2-Aminoethylgruppe oder für eine Gruppierung der Formel (S-III).

Wenn der Rest f für die Zahl 1 steht und der Rest h für die Zahl 0 steht, enthält die erfindungsgemäße organische Silicumverbindung die Gruppierung [NR₇-(A')], aber nicht die Gruppierung -[NR₈-(A‴)]. Steht nun der Rest R7 für eine Gruppierung der Formel (III), so umfasst die organische Silicumverbindung 3 reaktive Silan-Gruppen.

In einer weiteren bevorzugten Ausführungsform ist ein erfindungsgemäßes Verfahren dadurch gekennzeichnet, dass die Zusammensetzung(A) ein oder mehrere organische C₁-C₆-Alkoxy-Silane (A2) der Formel (S-II) enthält

(R₅O)_{c}(R₆)_{d}Si-(A)e-[NR₇-(A')]_{f}-[O-(A")]_{g}-[NR₈-(A‴)]ₕ-Si(R₆')_{d'}(OR₅')c_{'} (II),

wobei
- e und f beide für die Zahl 1 stehen,
- g und h beide für die Zahl 0 stehen,
- A und A` unabhängig voneinander für eine lineare, zweiwertige C₁-C₆-Alkylengruppe stehen
   und
- R7 für ein Wasserstoffatom, eine Methylgruppe, eine 2-Hydroxyethylgruppe, eine 2-Alkenylgruppe, eine 2-Aminoethylgruppe oder für eine Gruppierung der Formel (S-III) steht.

In einer weiteren bevorzugten Ausführungsform ist ein erfindungsgemäßes Verfahren dadurch gekennzeichnet, dass die Zusammensetzung (A) ein oder mehrere organische C₁-C₆-Alkoxy-Silane (A2) der Formel (S-II) enthält, wobei
- e und f beide für die Zahl 1 stehen,
- g und h beide für die Zahl 0 stehen,
- A und A` unabhängig voneinander für eine Methylengruppe (-CH₂-), eine Ethylengruppe (-CH₂-CH₂-) oder eine Propylengruppe (-CH₂-CH₂-CH₂) stehen,
   und
- R7 für ein Wasserstoffatom, eine Methylgruppe, eine 2-Hydroxyethylgruppe, eine 2-Alkenylgruppe, eine 2-Aminoethylgruppe oder für eine Gruppierung der Formel (S-III) steht.

Zur Lösung der erfindungsgemäßen Aufgabenstellung gut geeignete organische Silicumverbindungen der Formel (S-II) sind
- 3-(Trimethoxysilyl)-N-[3-(trimethoxysilyl)propyl]-1-propanamin 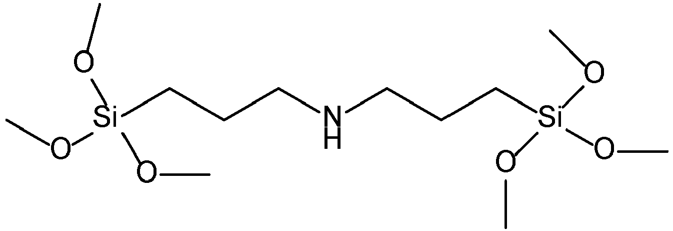
- 3-(Triethoxysilyl)-N-[3-(triethoxysilyl)propyl]-1-propanamin 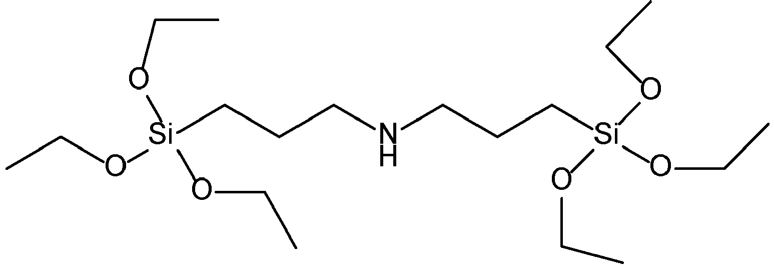
- N-Methyl-3-(trimethoxysilyl)-N-[3-(trimethoxysilyl)propyl]-1-propanamin 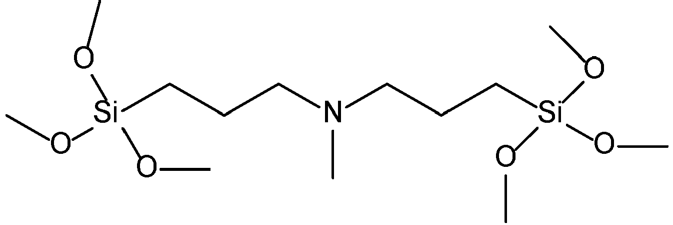
- N-Methyl-3-(triethoxysilyl)-N-[3-(triethoxysilyl)propyl]-1-propanamin 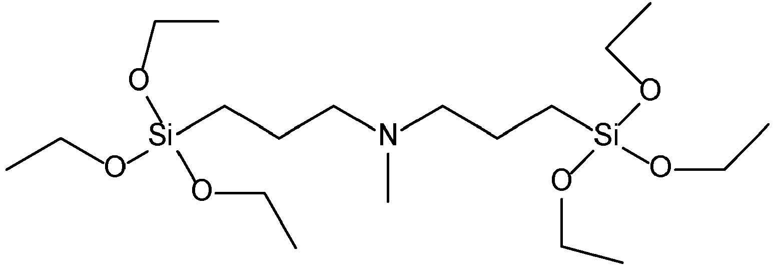
- 2-[Bis[3-(trimethoxysilyl)propyl]amino]-ethanol 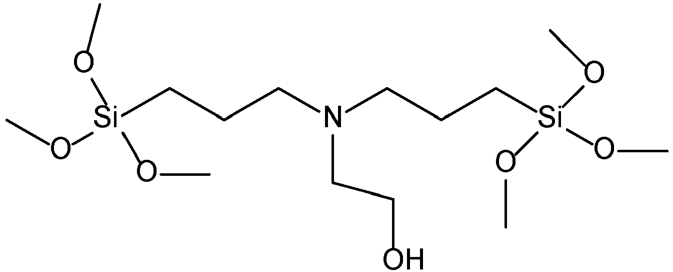
- 2-[Bis[3-(triethoxysilyl)propyl]amino]-ethanol 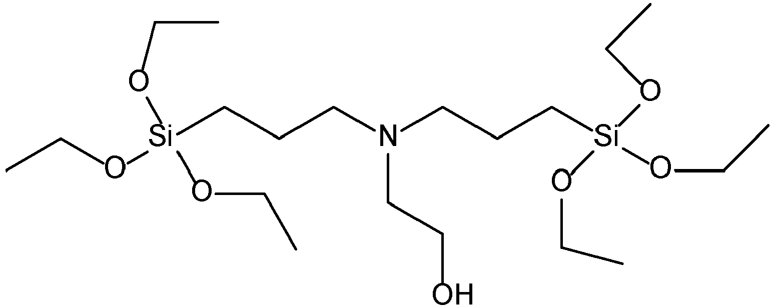
- 3-(Trimethoxysilyl)-N,N-bis[3-(trimethoxysilyl)propyl]-1-propanamin 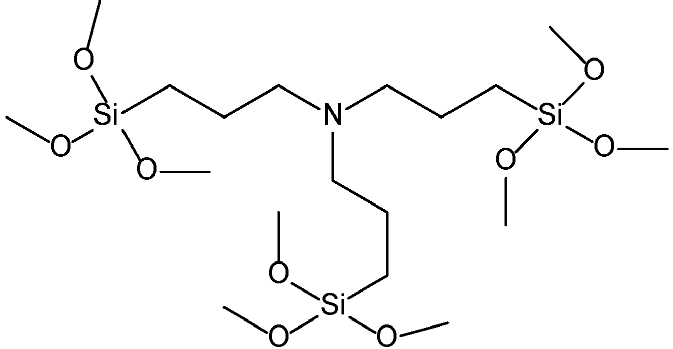
- 3-(Triethoxysilyl)-N,N-bis[3-(triethoxysilyl)propyl]-1-propanamin 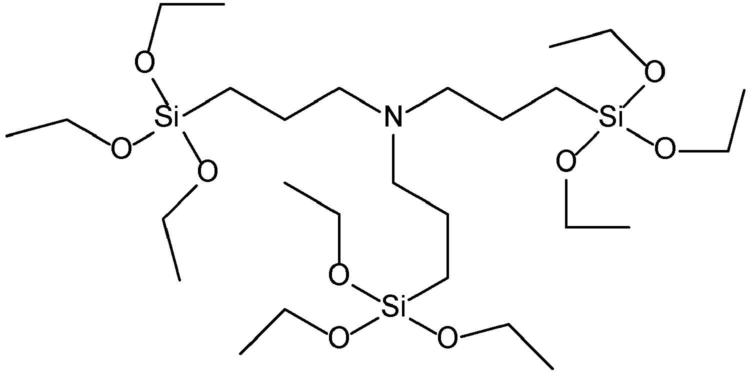
- N1 ,N1-Bis[3-(trimethoxysilyl)propyl]-1 ,2-ethanediamin, 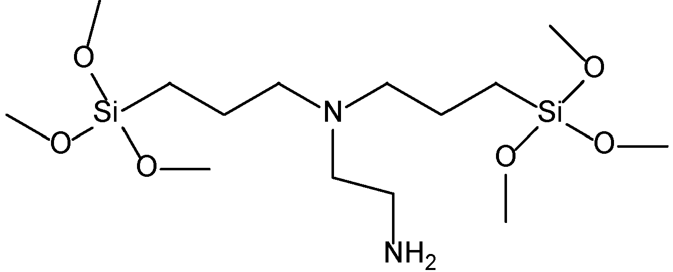
- N1 ,N1-Bis[3-(triethoxysilyl)propyl]-1 ,2-ethanediamin, 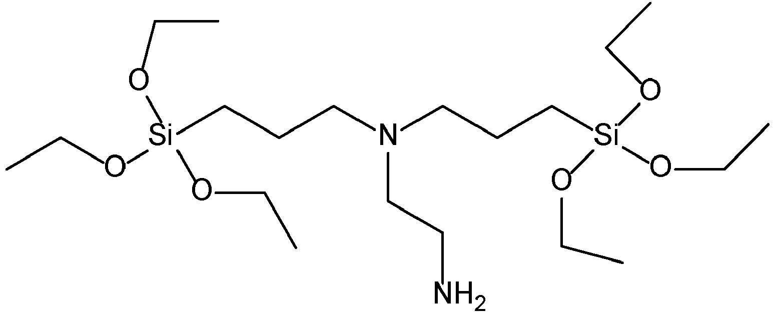
- N,N-Bis[3-(trimethoxysilyl)propyl]-2-propen-1-amin 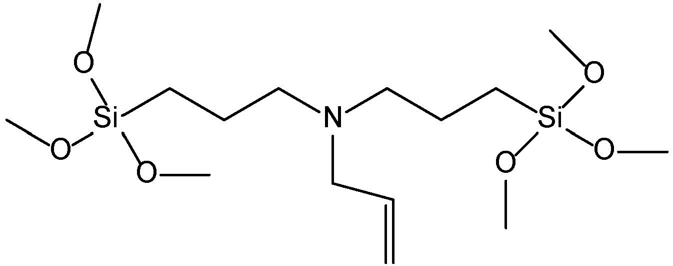
- N,N-Bis[3-(triethoxysilyl)propyl]-2-propen-1-amin

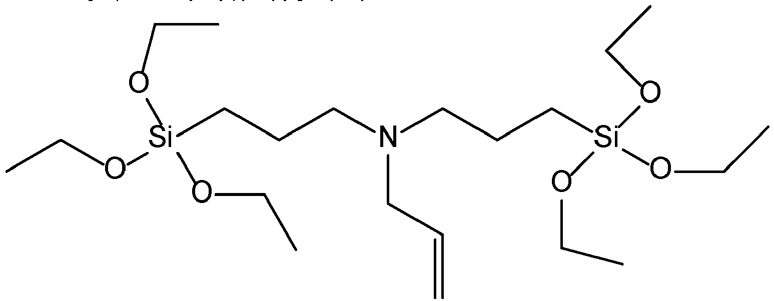

Die vorgenannten organische Siliciumverbindung der Formel (S-II) sind kommerziell erhältlich. Bis(trimethoxysilylpropyl)amine mit der CAS-Nummer 82985-35-1 kann beispielsweise von Sigma-Aldrich käuflich erworben werden.

Bis[3-(triethoxysilyl)propyl]amine mit der CAS-Nummer 13497-18-2 kann zum Beispiel von Sigma-Aldrich käuflich erworben werden.

N-Methyl-3-(trimethoxysilyl)-N-[3-(trimethoxysilyl)propyl]- 1-propanamin wird alternativ auch als Bis(3-trimethoxysilylpropyl)-N-methylamin bezeichnet und kann bei Sigma-Aldrich oder Fluorochem kommerziell erworben werden.

3-(Triethoxysilyl)-N,N-bis[3-(triethoxysilyl)propyl]-1-propanamin mit der CAS-Nummer 18784-74-2 kann beispielsweise von Fluorochem oder Sigma-Aldrich käuflich erworben werden.

In einer weiteren bevorzugten Ausführungsform ist ein erfindungsgemäßes Verfahren dadurch gekennzeichnet, dass die Zusammensetzung (A) ein oder mehrere organische C₁-C₆-Alkoxy-Silane der Formel (S-II) enthält, die ausgewählt sind aus der Gruppe aus
- 3-(Trimethoxysilyl)-N-[3-(trimethoxysilyl)propyl]-1-propanamin
- 3-(Triethoxysilyl)-N-[3-(triethoxysilyl)propyl]-1-propanamin
- N-Methyl-3-(trimethoxysilyl)-N-[3-(trimethoxysilyl)propyl]-1-propanamin
- N-Methyl-3-(triethoxysilyl)-N-[3-(triethoxysilyl)propyl]-1-propanamin
- 2-[Bis[3-(trimethoxysilyl)propyl]amino]-ethanol
- 2-[Bis[3-(triethoxysilyl)propyl]amino]-ethanol
- 3-(Trimethoxysilyl)-N,N-bis[3-(trimethoxysilyl)propyl]-1-Propanamin
- 3-(Triethoxysilyl)-N,N-bis[3-(triethoxysilyl)propyl]-1-Propanamin
- N1 ,N1-Bis[3-(trimethoxysilyl)propyl]-1 ,2-Ethanediamin,
- N1 ,N1-Bis[3-(triethoxysilyl)propyl]-1 ,2-Ethanediamin,
- N,N-Bis[3-(trimethoxysilyl)propyl]-2-Propen-1-amin und/oder
- N,N-Bis[3-(triethoxysilyl)propyl]-2-Propen-1-amin,
und/oder deren Kondensationsprodukten.

In weiteren Färbeversuchen hat es sich ebenfalls als ganz besonders vorteilhaft herausgestellt, wenn im erfindungsgemäßen Verfahren mindestens ein organisches C₁-C₆-Alkoxy-Silan (A2) der Formel (S-IV) eingesetzt wurde

R₉Si(OR₁₀)ₖ(R₁₁)m (S-IV).

Die Verbindungen der Formel (S-IV) sind organische Siliciumverbindungen, die aus Silanen mit einem, zwei oder drei Siliciumatomen ausgewählt sind, wobei die organische Siliciumverbindung eine oder mehrere hydrolysierbare Gruppen pro Molekül umfasst.

Das bzw. die organischen Siliciumverbindungen der Formel (S-IV) können auch als Silane vom Typ der Alkyl-C₁-C₆-alkoxy-silane bezeichnet werden,

R₉Si(OR₁₀)ₖ(R₁₁)m (S-IV),

wobei
- R₉ für eine C₁-C₁₂-Alkylgruppe steht,
- R₁₀ für eine C₁-C₆-Alkylgruppe steht,
- R₁₁ für eine C₁-C₆-Alkylgruppe steht
- k für eine ganze Zahl von 1 bis 3 steht, und
- m für die ganze Zahl 3 - k steht.

In einer weiteren Ausführungsform ist ein besonders bevorzugtes erfindungsgemäßes Verfahren dadurch gekennzeichnet, die erste Zusammensetzung (A) ein oder mehrere organische C₁-C₆-Alkoxy-Silane (A2) der Formel (S-IV) enthält,

R₉Si(OR₁₀)ₖ(R₁₁)m (S-IV),

wobei
- R₉ für eine C₁-C₁₂-Alkylgruppe steht,
- R₁₀ für eine C₁-C₆-Alkylgruppe steht,
- R₁₁ für eine C₁-C₆-Alkylgruppe steht
- k für eine ganze Zahl von 1 bis 3 steht, und
- m für die ganze Zahl 3 - k steht,
und/oder deren Kondensationsprodukte.

In den organischen C₁-C₆-Alkoxy-Silanen der Formel (S-IV) steht der Rest R₉ für eine C₁-C₁₂-Alkylgruppe. Diese C₁-C₁₂-Alkylgruppe ist gesättigt und kann linear oder verzweigt sein. Bevorzugt steht R₉ für eine lineare C₁-C₈-Alkylgruppe. Bevorzugt steht R₉ für eine Methylgruppe, eine Ethylgruppe, eine n-Propylgruppe, eine n-Butylgruppe, eine n-Pentylgruppe, eine n-Hexylgruppe, eine n-Octylgruppe oder eine n-Dodecylgruppe. Besonders bevorzugt steht R₉ für eine Methylgruppe, eine Ethylgruppe oder eine n-Octylgruppe.

In den organischen Siliciumverbindungen der Formel (S-IV) steht der Rest R₁₀ für eine C₁-C₆-Alkylgruppe. Besonders bevorzugt steht R₁₀ für eine Methylgruppe oder für eine Ethylgruppe.

In den organischen Siliciumverbindungen der Formel (S-IV) steht der Rest R₁₁ für eine C₁-C₆-Alkylgruppe. Besonders bevorzugt steht R₁₁ für eine Methylgruppe oder für eine Ethylgruppe.

Weiterhin steht k für eine ganze Zahl von 1 bis 3, und m steht für die ganze Zahl 3 - k. Wenn k für die Zahl 3 steht, dann ist m gleich 0. Wenn k für die Zahl 2 steht, dann ist m gleich 1. Wenn k für die Zahl 1 steht, dann ist m gleich 2.

Färbungen mit den besten Waschechtheiten konnten erhalten werden, wenn die Zusammensetzung (A) mindestens ein organisches C₁-C₆-Alkoxy-Silan (A2) der Formel (S-IV) enthält, bei welchem der Rest k für die Zahl 3 steht. In diesem Fall steht der Rest m für die Zahl 0.

Zur Lösung der erfindungsgemäßen Aufgabenstellung besonders gut geeignete organische Silicumverbindungen der Formel (S-IV) sind
- Methyltrimethoxysilan 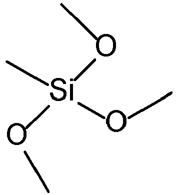
- Methyltriethoxysilan 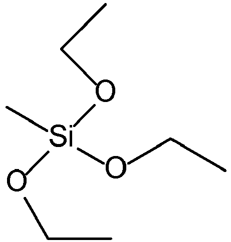
- Ethyltrimethoxysilan 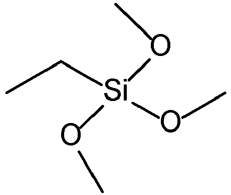
- Ethyltriethoxysilan 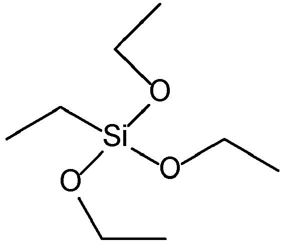
- n-Propyltrimethoxysilan (auch bezeichnet ans Propyltrimethoxysilan) 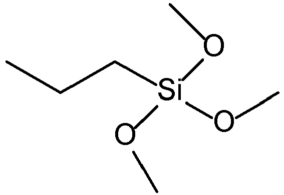
- n-Propyltriethoxysilan (auch bezeichnet als Propyltriethoxysilan) 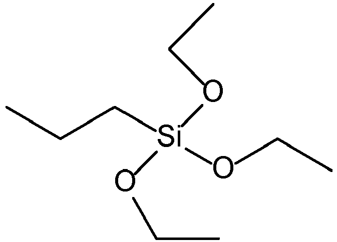
- n-Hexyltrimethoxysilan (auch bezeichnet als Hexyltrimethoxysilan) 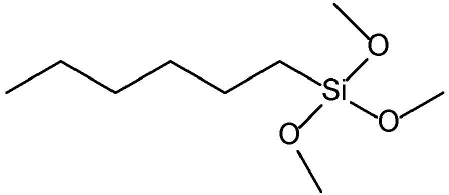
- n-Hexyltriethoxysilan (auch bezeichnet als Hexyltriethoxysilan) 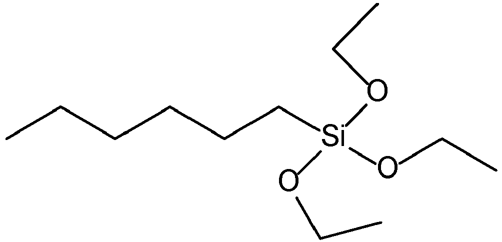
- n-Octyltrimethoxysilan (auch bezeichnet als Octyltrimethoxysilan) 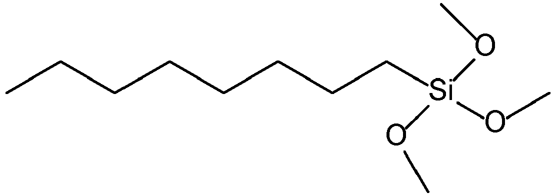
- n-Octyltriethoxysilan (auch bezeichnet als Octyltriethoxysilan) 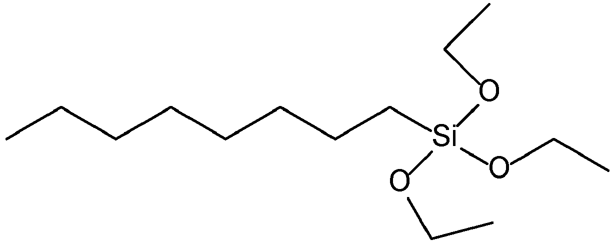
- n-Dodecyltrimethoxysilan (auch bezeichnet als Dodecyltrimethoxysilan) und/oder 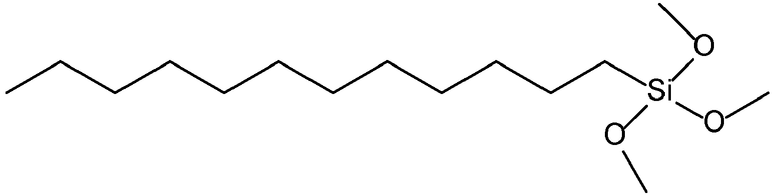
- n-Dodecyltriethoxysilan (auch bezeichnet als Dodecyltriethoxysilan).

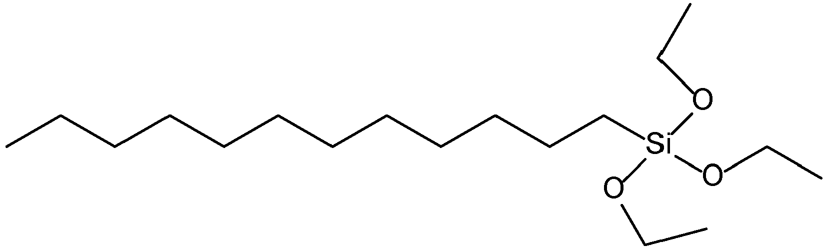

In einer weiteren bevorzugten Ausführungsform ist ein erfindungsgemäßes Verfahren dadurch gekennzeichnet, dass die erste Zusammensetzung (A) mindestens ein organisches C₁-C₆-Alkoxysilan (A2) der Formel (S-IV) enthält, das ausgewählt ist aus der Gruppe aus
- Methyltrimethoxysilan
- Methyltriethoxysilan
- Ethyltrimethoxysilan
- Ethyltriethoxysilan
- Hexyltrimethoxysilan
- Hexyltriethoxysilan
- Octyltrimethoxysilan
- Octyltriethoxysilan
- Dodecyltrimethoxysilan,
- Dodecyltriethoxysilan,
und/oder deren Kondensationsprodukten.

Bei den entsprechenden Hydrolyse bzw. Kondensationsprodukten handelt es sich beispielsweise um die folgenden Verbindungen:
Hydrolyse von C₁-C₆-Alkoxy-Silan der Formel (S-I) mit Wasser (Reaktionsschema am Beispiel von 3-Aminopropyltriethoxysilan):

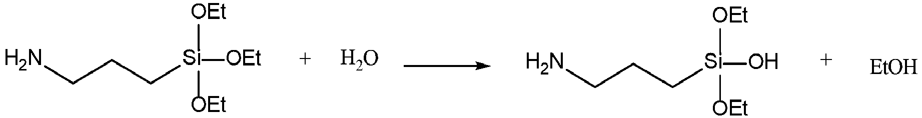

In Abhängigkeit von der eingesetzten Menge an Wasser kann die Hydrolyse-Reaktion auch mehrfach pro eingesetztem C₁-C₆-Alkoxy-Silan stattfinden: 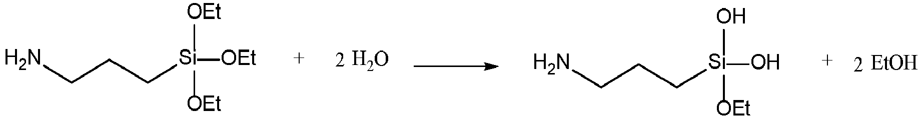 bzw.

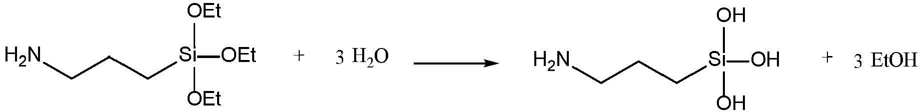

Hydrolyse von C₁-C₆-Alkoxy-Silan der Formel (S-IV) mit Wasser (Reaktionsschema am Beispiel von Methyltrimethoxysilan):

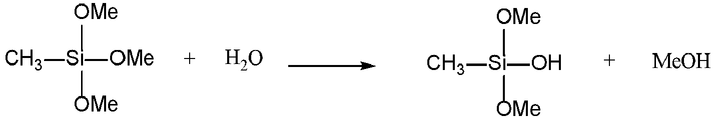

In Abhängigkeit von der eingesetzten Menge an Wasser kann die Hydrolyse-Reaktion auch mehrfach pro eingesetztem C₁-C₆-Alkoxy-Silan stattfinden: 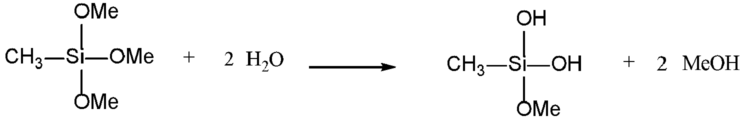 bzw.

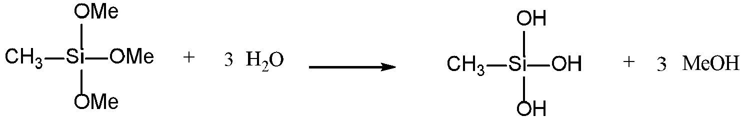

Mögliche Kondensationsreaktionen sind beispielsweise (gezeigt anhand des Gemisches (3-Aminopropyl)triethoxysilan und Methyltrimethoxysilan): 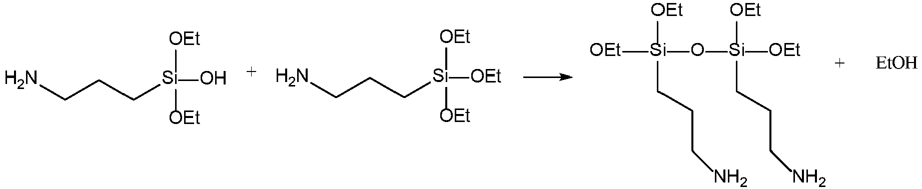 und/oder 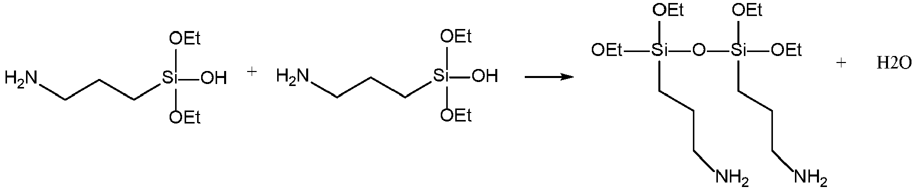 und/oder 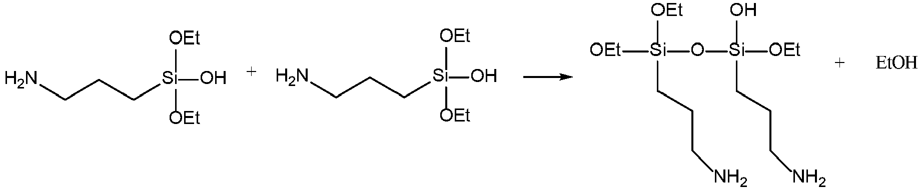 und/oder 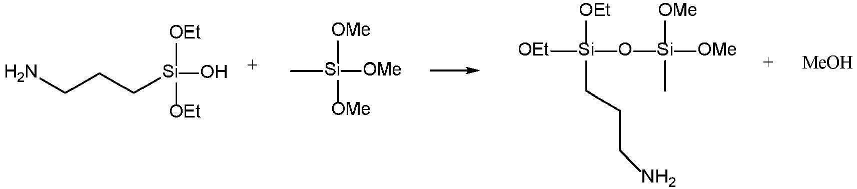 und/oder 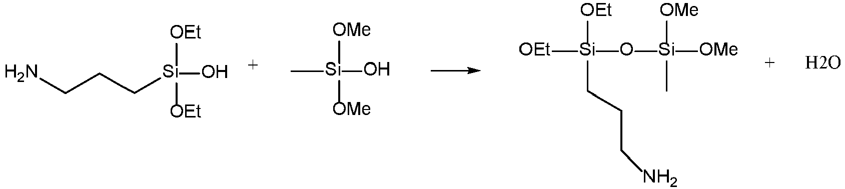 und/oder 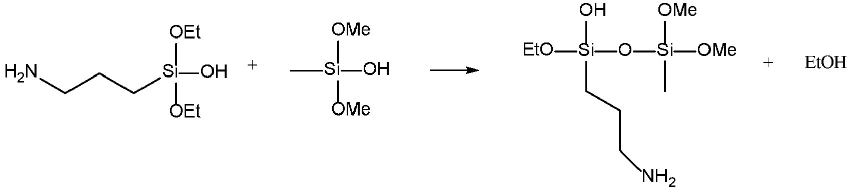 und/oder

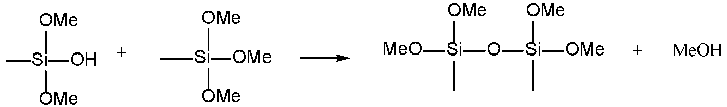

In den obigen beispielhaften Reaktionsschemata ist jeweils die Kondensation zu einem Dimer gezeigt, jedoch sind auch weitergehende Kondensationen zu Oligomeren mit mehreren Silan-Atomen möglich und auch bevorzugt.

An diesen Kondensationsreaktionen können sowohl partiell hydrolysierte als auch vollständig hydrolysierte C₁-C₆-Alkoxysilane der Formel (S-I) teilnehmen, die eine Kondensation mit noch nicht abreagierten, partiell oder auch vollständig hydrolysierten C₁-C₆-Alkoxysilanen der Formel (S-I) durchlaufen. In diesem Fall reagieren die C₁-C₆-Alkoxysilane der Formel (S-I) mit sich selbst.

Weiterhin können an den Kondensationsreaktionen auch sowohl partiell hydrolysierte als auch vollständig hydrolysierte C₁-C₆-Alkoxysilane der Formel (S-I) teilnehmen, die eine Kondensation mit noch nicht abreagierten, partiell oder auch vollständig hydrolysierten C₁-C₆-Alkoxysilanen der Formel (S-IV) durchlaufen. In diesem Fall reagieren die C₁-C₆-Alkoxysilane der Formel (S-I) mit den C₁-C₆-Alkoxysilanen der Formel (S-IV).

Weiterhin können an den Kondensationsreaktionen auch sowohl partiell hydrolysierte als auch vollständig hydrolysierte C₁-C₆-Alkoxysilane der Formel (S-IV) teilnehmen, die eine Kondensation mit noch nicht abreagierten, partiell oder auch vollständig hydrolysierten C₁-C₆-Alkoxysilanen der Formel (S-IV) durchlaufen. In diesem Fall reagieren die C₁-C₆-Alkoxysilane der Formel (S-IV) mit sich selbst.

Die erfindungsgemäße Zusammensetzung (A) kann ein oder mehrere organische C₁-C₆-Alkoxy-silane (A2) in verschiedenen Mengenanteilen enthalten. Diese bestimmt der Fachmann in Abhängigkeit von der gewünschten Dicke des Silan-Coatings auf dem Keratinmaterial und von der Menge des zu behandelnden Keratinmaterials.

Besonders lagerstabile Zubereitungen mit sehr gutem Färberesultat bei der Anwendung konnten erhalten werden, wenn die Zusammensetzung (A) - bezogen auf ihr Gesamtgewicht - ein oder mehrere organische C₁-C₆-Alkoxysilane (A2) und/oder die Kondensationsprodukte hiervon in einer Gesamtmenge von 30,0 bis 85,0 Gew.-%, bevorzugt von 35,0 bis 80,0 Gew.-%, weiter bevorzugt von 40,0 bis 75,0 Gew.-%, noch weiter bevorzugt von 45,0 bis 70,0 Gew.-% und ganz besonders bevorzugt von 50,0 bis 65,0 Gew.-% enthält.

In einer weiteren Ausführungsform ist ein ganz besonders bevorzugtes Verfahren dadurch gekennzeichnet, dass die erste Zusammensetzung (A) - bezogen auf das Gesamtgewicht der Zusammensetzung (A) - ein oder mehrere organische C₁-C₆-Alkoxysilane (A2) und/oder die Kondensationsprodukte hiervon in einer Gesamtmenge von 30,0 bis 85,0 Gew.-%, bevorzugt von 35,0 bis 80,0 Gew.-%, weiter bevorzugt von 40,0 bis 75,0 Gew.-%, noch weiter bevorzugt von 45,0 bis 70,0 Gew.-% und ganz besonders bevorzugt von 50,0 bis 65,0 Gew.-% enthält.

### Weitere kosmetische Inhaltsstoffe in der Zusammensetzung (A)

Prinzipiell kann die Zusammensetzung (A) auch noch einen oder mehrere weitere kosmetische Inhaltsstoffe enthalten.

Bei den fakultativ in der Zusammensetzung (A) einsetzbaren kosmetischen Inhaltsstoffen kann es sich um alle geeigneten Bestandteile handeln, um dem Mittel weitere positive Eigenschaften zu verleihen. Beispielsweise können in der Zusammensetzung (A) ein Lösungsmittel, ein verdickendes oder filmbildendes Polymer, eine oberflächenaktive Verbindung aus der Gruppe der nichtionischen, kationischen, anionischen oder zwitterionischen/amphoteren Tenside, der farbgebenden Verbindungen aus der Gruppe der Pigmente, der direktziehenden Farbstoffe, der Oxidationsfarbstoffvorprodukte, der Fettkomponenten aus der Gruppe der C₈-C₃₀-Fettalkohole, der Kohlenwasserstoffverbindungen, Fettsäureester, der zur Gruppe der pH-Regulatoren zugehörigen Säuren und Basen, der der Parfüme, der Konservierungsmittel, der Pflanzenextrakte und der Proteinhydrolysate enthalten sein.

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher verwiesen.

Wie bereits zuvor beschrieben, können die organischen C₁-C₆-Alkoxysilane (A2) jedoch nicht nur mit Wasser, sondern auch mit anderen kosmetischen Inhaltsstoffen reagieren. Zur Vermeidung dieser unerwünschten Reaktionen enthalten die Zubereitungen (A) mit Alkoxy-Silanen deshalb bevorzugt keine weiteren Inhaltsstoffe oder nur die ausgewählten Inhaltsstoffe, die sich gegenüber den C₁-C₆-Alkoxy-Silanen als chemisch inert herausgestellt haben. Als ganz besonders bevorzugt hat es sich in diesem Zusammenhang erwiesen, in der Zusammensetzung (A) einen kosmetischen Inhaltsstoff aus der Gruppe aus Hexamethyldisiloxan, Octamethyltrisiloxan, Decamethyltetrasiloxan, Hexamethylcyclotrisiloxan, Octamethylcyclotetrasiloxan und/oder Decamethylcyclopentasiloxan einzusetzen.

In einer weiteren ganz besonders bevorzugten Ausführungsform ist ein erfindungsgemäßes Verfahren dadurch gekennzeichnet, das die erste Zusammsensetzung (A) mindestens einen kosmetischen Inhaltsstoff aus der Gruppe aus Hexamethyldisiloxan. Octamethyltrisiloxan, Decamethyltetrasiloxan, Hexamethylcyclotrisiloxan, Octamethylcyclotetrasiloxan und Decamethylcyclopentasiloxan enthält.

Hexamethyldisiloxan besitzt die CAS-Nummer 107-46-0 und kann beispielsweise bei Sigma-Aldrich kommerziell erworben werden.

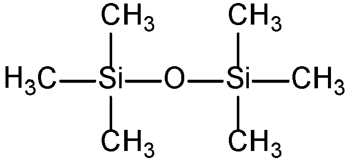

Octamethyltrisiloxan besitzt die CAS-Nummer 107-51-7 und ist ebenfalls bei Sigma-Aldrich kommerziell erhältlich.

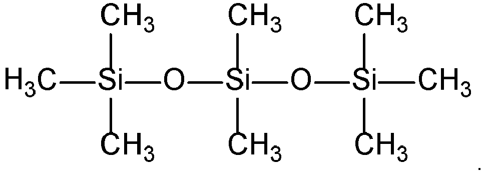

Decamethyltetrasiloxan trägt die CAS-Nummer 141-62-8 und ist ebenfalls bei Sigma-Aldrich kommerziell erhältlich.

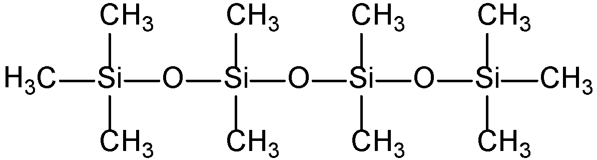

Hexamethylcyclotrisiloxan besitzt die CAS-Nr. 541-05-9.

Octamethylcyclotetrasiloxan besitzt die CAS-Nr. 556-67-2.

Decamethylcyclopentasiloxan besitzt die CAS-Nr. 541-02-6.

Als ganz besonders bevorzugt hat sich der Einsatz von Hexamethyldisiloxan in der Zusammensetzung (A) herausgestellt. Besonders bevorzugt ist Hexamethyldisiloxan - bezogen auf das Gesamtgewicht der Zusammensetzung (A) - in Mengen von 10,0 bis 50,0 Gew.-%, bevorzugt 15,0 bis 45,0 Gew.-%, weiter bevorzugt 20,0 bis 40,0 Gew.-%, noch weiter bevorzugt 25,0 bis 35,0 Gew.-% und ganz besonders bevorzugt 31,0 bis 34,0 Gew.-% in der Zusammensetzung (A) enthalten.

In einer weiteren ganz besonders bevorzugten Ausführungsform ist ein erfindungsgemäßes Verfahren dadurch gekennzeichnet, dass die erste Zusammensetzung (A) - bezogen auf das Gesamtgewicht der Zusammensetzung (A) - 10,0 bis 50,0 Gew.-%, bevorzugt 15,0 bis 45,0 Gew.-%, weiter bevorzugt 20,0 bis 40,0 Gew.-%, noch weiter bevorzugt 25,0 bis 35,0 Gew.-% und ganz besonders bevorzugt 31,0 bis 34,0 Gew.-% Hexamethyldisiloxan enthält.

### Herstellung der Zusammensetzung (A),

In Schritt (1) des erfindungsgemäßen Verfahrens erfolgt die Herstellung der Zusammensetzung (A). Die Herstellung kann beispielsweise durch Reaktion oder auch Umsetzung von einem oder mehreren organischen C₁-C₆-Alkoxy-Silanen mit Wasser erfolgen. Zur Initiierung dieser Reaktion werden das bzw. die C₁-C₆-Alkoxy-Silane bevorzugt mit Wasser gemischt.

Die Herstellung kann zum Beispiel in einem hierfür geeigneten Reaktionsgefäß oder auch Reaktor durchgeführt werden. Abhängig von der gewünschten Ansatzgröße sind hierfür verschiedene Modelle aus dem Stand der Technik bekannt und kommerziell erwerbbar.

So kann die Reaktion der organischen C₁-C₆-Alkoxy-Silane mit Wasser beispielsweise in einem Reaktionsgefäß oder einem Reaktor, bevorzugt in einem Doppelwandreaktor, einem Reaktor mit externem Wärmeaustauscher, einem Rohrreaktor, einem Reaktor mit Dünnfilm-Verdampfer, einem Reaktor mit Fallfilmverdampfer und/oder einem Reaktor mit angeschlossenem Kondensator durchgeführt werden.

In einer weiteren besonders bevorzugten Ausführungsform ist ein erfindungsgemäßes Verfahren gekennzeichnet durch das
(1) Herstellung der ersten Zusammensetzung (A) durch Mischen eines oder mehrerer organischer C₁-C₆-Alkoxy-Silane mit Wasser in einem Reaktionsgefäß oder einem Reaktor, bevorzugt in einem Doppelwandreaktor, einem Reaktor mit externem Wärmeaustauscher, einem Rohrreaktor, einem Reaktor mit Dünnfilm-Verdampfer, einem Reaktor mit Fallfilmverdampfer und/oder einem Reaktor mit angeschlossenem Kondensator.

Ein für kleinere Ansätze sehr gut geeignetes Reaktionsgefäß ist beispielsweise ein üblicherweise für chemische Reaktionen eingesetzter Glaskolben mit einem Fassungsvermögen von 1 Liter, 3 Litern oder 5 Litern handeln, beispielsweise ein 3-Liter Ein- oder Mehrhalskolben mit Schliffen.

Als Reaktor wird ein abgegrenzter Raum (Behältnis, Behälter) bezeichnet, der speziell dafür konstruiert und hergestellt wurde, um darin unter definierten Bedingungen bestimmte Reaktionen ablaufen lassen und steuern zu können.

Für größere Ansätze hat es sich als vorteilhaft herausgestellt, die Reaktion in Reaktoren aus Metall durchzuführen. Typische Reaktoren können beispielsweise eine Füllmenge von 10-Litern, 20-Litern oder 50-Litern umfassen. Größere Reaktoren für den Produktionsbereich können auch Füllmengen von 100-Litern, 500-Litern oder 1000-Litern umfassen.

Doppelwandreaktoren besitzen zwei Reaktor-Hüllen bzw. Reaktor-Wandungen, wobei in dem zwischen beiden Wandungen befindlichen Bereich eine Temperier-Flüssigkeit zirkulieren kann. Diese ermöglicht eine besonders gute Einstellung der Temperatur auf die benötigten Werte.

Als besonders gut geeignet hat sich auch der Einsatz von Reaktoren, insbesondere DoppelwandReaktoren mit vergrößerter Wärme-Austauschfläche erwiesen, wobei der Wärmeaustauch hierbei entweder durch interne Einbauten oder auch durch Einsatz eines externen Wärmeaustauschers erfolgen kann.

Entsprechende Reaktoren sind beispielsweise Laborreaktoren der Firma IKA. In diesem Zusammenhang können die Modelle "LR-2.ST" oder das Modell "magic plant" genannt werden.

Die in Schritt (1) stattfindende Reaktion der organischen C₁-C₆-Alkoxy-Silane mit Wasser kann auf verschiedenen Wegen stattfinden. Die Reaktion startet, sobald die C₁-C₆-Alkoxy-Silane mit Wasser durch Vermischen in Kontakt kommen. Eine Möglichkeit ist es, die gewünschte Wassermenge im Reaktionsgefäß oder Reaktor vorzulegen und im Anschluss daran dass oder die C₁-C₆-Alkoxy-Silane hinzuzufügen.

In einer weiteren Ausführungsform ist es auch möglich, zunächst das oder die organischen C₁-C₆-Alkoxy-Silane im Reaktionsgefäß bzw. Reaktor vorzulegen und dann die gewünschte Menge Wasser hinzuzugeben.

Für die Herstellung besonders leistungsstarker Keratinbehandlungsmittel hat sich bei der Herstellung in Schritt (1) die Einhaltung spezieller Temperaturbereiche als ganz besonders vorteilhaft herausgestellt.

In diesem Zusammenhang konnte gefunden werden, dass eine Mindest-Temperatur von 20 °C in Schritt (1) besonders gut geeignet ist, um die Hydrolyse mit ausreichend hoher Geschwindigkeit ablaufen zu lassen und eine effiziente Reaktionsführung zu gewährleisten.

Auf der anderen Seite sollte jedoch eine Erwärmung des Reaktionsgemisches auf Temperaturen über 70 °C vermieden werden. Erfolgt die Herstellung bei zu hohen Temperaturen, so findet vermutlich eine an dieser Stelle unerwünschte oder zu starke Polymerisierung bzw. Kondensationsreaktion statt, die dazu führt, dass sich bei der späteren Anwendung des Mittels auf dem Keratinmaterial kein an dem Keratin anhaftender Film mehr ausbilden kann. Bei Anwendung eines bei zu hohen Temperaturen hergestelltes Mittels in einem Färbeverfahren konnten somit keine ausreichend hohen Farbintensitäten mehr erzielt werden.

Aus diesen Gründen sollte die Reaktion des oder der organischer C₁-C₆-Alkoxy-Silane mit Wasser in Schritt (1) des Verfahrens bei einer Temperatur von 20 bis 70 °C durchgeführt werden.

Alle weiteren optional in der Zusammensetzung (A) enthaltenen Inhaltsstoffe können zum Beispiel im Anschluss an diese Reaktion zu der Zusammensetzung (A) hinzugefügt werden.

Im Anschluss an die Herstellung wird die Zusammensetzung (A) bevorzugt in eine Verpackungseinheit abgefüllt.

Bei der Verpackungseinheit kann es sich entweder um eine Endverpackung handeln, aus welcher der Anwender das Mittel zur Behandlung der Keratinmaterialien entnimmt. Geeignete Endverpackungen sind beispielsweise eine Flasche, eine Tube, einen Tiegel, eine Dose, ein Sachet, einen Aerosol-Druckbehälter, einen Non-Aerosol-Druckbehälter. Hierbei können diese Endverpackungen die Keratinbehandlungsmittel in Mengen enthalten, die für eine, gegebenenfalls auch führ mehrere Anwendungen ausreichen. Bevorzugt ist die Abfüllung in einer Menge, die für eine einmalige Anwendung ausreichend ist.

Weiterhin kann die Zusammensetzung (A) jedoch auch in eine Zwischenverpackung abgefüllt werden, bei der es sich beispielsweise um einen Kanister oder einen Hobbock handeln kann. Die Abfüllung in eine Zwischenverpackung ist insbesondere dann geeignet, wenn das Reaktionsgefäß bzw. der Reaktor, in dem das erfindungsgemäße Verfahren durchgeführt wurde, und die Abfüllanlage, in der eine Abfüllung in die Endverpackung erfolgt, räumlich getrennt sind.

In einer weiteren ganz besonders bevorzugten Ausführungsform ist ein erfindungsgemäßes Verfahren gekennzeichnet durch die
(1) Herstellung einer ersten Zusammensetzung (A), enthaltend
   (A1) weniger als 10 Gew.-% Wasser und
   (A2) ein oder mehrere organische C₁-C₆-Alkoxy-Silane und/oder deren Kondensationsprodukte,
   und Abfüllung der Zusammensetzung (A) in eine Flasche, eine Tube, einen Tiegel, eine Dose, ein Sachet, einen Aerosol-Druckbehälter, einen Non-Aerosol-Druckbehälter, einen Kanister oder einen Hobbock.

Bei den vorgenannten Verpackungseinheiten kann es sich um übliche, standardmäßig in der Kosmetik eingesetzte und kommerziell erhältliche Behältnisse handeln.

### Zusammensetzung (B)

Schritt (2) des erfindungsgemäßen Verfahrens ist gekennzeichnet durch die Herstellung der zweiten Zusammensetzung (B). Die Zusammensetzung (B) ist dadurch gekennzeichnet, dass sie
(B1) Wasser und
(B2) ein oder mehrere Fettbestandteile und
(B3) ein oder mehrere farbgebende Verbindungen aus der Gruppe der Pigmente und/oder der direktziehenden Farbstoffe
enthält.

### Wassergehalt (B1) in der Zusammensetzung (B)

Kurz vor der Anwendung auf dem keratinischen Material werden die Zusammensetzungen (A) und (B) miteinander vermischt. Durch das Vermischen von (A) und (B) wird das anwendungsbereite Keratinbehandlungsmittel hergestellt, d.h. der lagerstabile bzw. lagerfähige Silan-Blend (A) wird durch Kontakt mit der wasserhaltigen Zusammensetzung (B) in seine reaktive Form überführt. Mit Vermischen der Zusammensetzungen (A) und (B) startet eine von den Alkoxy-Silan-Monomeren oder Alkoxy-Silan-Oligomeren ausgehende Polymerisationsreaktion, die schließlich zur Ausbildung des Films bzw. des Coatings auf dem Keratinmaterial führt.

Je mehr Wasser mit dem oder den organischen C₁-C₆-Alkoxy-Silanen in Kontakt kommt, in desto stärkerem Ausmaß läuft die Polymerisationsreaktion ab. Enthält die Zusammensetzung (B) beispielsweise sehr viel Wasser (B1), so polymerisieren die zuvor in der wasserarmen Zusammensetzung (A) vorhandenen monomeren bzw. oligomeren Silan-Kondensate nun sehr schnell zu Polymeren höheren oder hohen Molekulargewichts. Die hochmolekularen Silan-Polymere bilden dann den Film auf dem keratinischen Material. Aus diesem Grund ist Wasser (B1) ein erfindungswesentlicher Inhaltsstoff der Zusammensetzung (B).

Besonders gleichmäßige Färbungen auf dem gesamten Kopf konnten erhalten werden, wenn die Zusammensetzung (B) - bezogen auf das Gesamtgewicht der Zusammensetzung (B) - 10,0 bis 90,0 Gew.-%, bevorzugt 30,0 bis 90,0 Gew.-%, weiter bevorzugt 50,0 bis 90,0 Gew.-% , nocht weiter bevorzugt 70,0 bis 90,0 Gew.-% und ganz besonders bevorzugt 75,0 bis 90,0 Gew.-% Wasser (B1) enthält.

Im Rahmen einer weiteren ganz besonders bevorzugten Ausführungsform ist ein erfindungsgemäßes Verfahren dadurch gekennzeichnet dass die zweite Zuammensetzung (B) - bezogen auf das Gesamtgewicht der Zusammensetzung (B) - 10,0 bis 90,0 Gew.-%, bevorzugt 30,0 bis 90,0 Gew.-%, weiter bevorzugt 50,0 bis 90,0 Gew.-% , nocht weiter bevorzugt 70,0 bis 90,0 Gew.-% und ganz besonders bevorzugt 75,0 bis 90,0 Gew.-% Wasser (B1) enthält.

### Fettbestandteile in der Zusammensetzung (B)

Kennzeichnend für die Zusammensetzung (B) ist weiterhin ihr Gehalt an mindestens einem Fettbestandteil. Überraschenderweise hat sich herausgestellt, dass der Einsatz mindestens eines Fettbestandteils die Reaktionsgeschwindigkeit der organischen C₁-C₆-Alkoxy-silane so optimiert, dass eine gleichmäßige Färbung auf dem gesamten Kopf ermöglicht wird.

Bei den Fettbestandteilen handelt es sich um hydrophobe Substanzen, die in Gegenwart von Wasser unter Ausbildung von Mizell-Systemen Emulsionen formen können. Ohne auf diese Theorie festgelegt zu sein, wird vermutet, dass die C₁-C₆-Alkoxysilane - entweder in Form ihrer Monomere oder gegebenenfalls in Form ihrer kondensierten Oligomere - in diese hydrophobe Umgebung bzw. in die Mizell-Systeme eingebettet werden, so dass sich die Polarität ihrer Umgebung verändert. Bedingt durch den hydrophoben Charakter der Fettbestandteile wird auch die Umgebung der C₁-C₆-Alkoxysilane hydrophobiert. Es wird angenommen, dass die zum Film bzw. Coating führende Polymerisationsreaktion der C₁-C₆-Alkoxy-silane in einem Milieu verringerter Polarität mit reduzierter Geschwindigkeit abläuft.

Unter "Fettbestandteilen" werden im Sinne der Erfindung organische Verbindungen mit einer Löslichkeit in Wasser bei Raumtemperatur (22 °C) und atmosphärischem Druck (760 mmHg) von weniger als 1 Gew.-%, bevorzugt von weniger als 0,1 Gew.-% verstanden. Unter die Definition der Fettbestandteile fallen explizit nur ungeladene (d.h. nichtionische) Verbindungen. Fettbestandteile besitzen mindestens eine gesättigte oder ungesättigte Alkylgruppe mit mindestens 12 C-Atomen. Das Molgewicht der Fettbestandteile liegt bei maximal 5000 g/mol, bevorzugt bei maximal 2500 g/mol und besonders bevorzugt bei maximal 1000 g/mol. Bei den Fettbestandteilen handelt es sich weder um polyoxyalkylierte noch um polyglycerylierte Verbindungen.

Ganz besonders bevorzugt werden die in der Zusammensetzung (B) enthaltenen Fettbestandteile (B2) ausgewählt aus der Gruppe der C₁₂-C₃₀-Fettalkohole, der C₁₂-C₃₀-Fettsäuretriglyceride, der C₁₂-C₃₀-Fettsäuremonoglyceride, der C₁₂-C₃₀-Fettsäurediglyceride und/oder der Kohlenwasserstoffe.

Als ganz besonders bevorzugte Fettbestandteile werden in diesem Zusammenhang die Bestandteile aus der Gruppe der C₁₂-C₃₀-Fettalkohole, der C₁₂-C₃₀-Fettsäuretriglyceride, der C₁₂-C₃₀-Fettsäuremonoglyceride, der C₁₂-C₃₀-Fettsäurediglyceride und/oder der Kohlenwasserstoffe verstanden. Im Sinne der vorliegenden Erfindung werden explizit nur nichtionische Substanzen als Fettbestandteile betrachtet. Geladene Verbindungen wie beispielsweise Fettsäuren und ihre Salze werden nicht als Fettbestandteil verstanden.

Im Rahmen einer Ausführungsform wurden ganz besonders gute Ergebnisse erhalten, wenn die zweite Zusammensetzung (B) einen oder mehrere Fettbestandteile (B2) aus der Gruppe der C₁₂-C₃₀-Fettalkohole, der C₁₂-C₃₀-Fettsäuretriglyceride, der C₁₂-C₃₀-Fettsäuremonoglyceride, der C₁₂-C₃₀-Fettsäurediglyceride und/oder der Kohlenwasserstoffe enthält.

Bei den C₁₂-C₃₀-Fettalkoholen kann es sich um gesättigte, ein- oder mehrfach ungesättigte, lineare oder verzweigte Fettalkohole mit 12 bis 30 C-Atomen handeln.

Beispiele für bevorzugte lineare, gesättigte C₁₂-C₃₀-Fettalkohole sind Dodecan-1-ol (Dodecylalkohol, Laurylalkohol), Tetradecan-1-ol (Tetradecylalkohol, Myristylalkohol), Hexadecan-1-ol (Hexadecylalkohol, Cetylalkohol, Palmitylalkohol), Octadecan-1-ol (Octadecylalkohol, Stearylalkohol), Arachylalkohol (Eicosan-1-ol), Heneicosylalkohol (Heneicosan-1-ol) und/oder Behenylalkohol (Docosan-1-ol).

Bevorzugte lineare, ungesättigte Fettalkohole sind (9Z)-Octadec-9-en-1-ol (Oleylalkohol), (9E)-Octadec-9-en-1-ol (Elaidylalkohol), (9Z,12Z)-Octadeca-9,12-dien-1-ol (Linoleylalkohol), (9Z,12Z,15Z)-Octadeca-9,12,15-trien-1-ol (Linolenoylalkohol), Gadoleylalkohol ((9Z)-Eicos-9-en-1-ol), Arachidonalkohol ((5Z,8Z,11Z,14Z)-Eicosa-5,8,11,14-tetraen-1-ol), Erucylalkohol ((13Z)-Docos-13-en-1-ol) und/oder Brassidylalkohol ((13E)-Docosen-1-ol).

Die bevorzugten Vertreter für verzweigte Fettalkohole sind 2-Octyl-dodecanol, 2-Hexyl-Dodecanol und/oder 2-Butyl-dodecanol.

Durch Auswahl besonders gut geeigneter Fettbestanteile kann die Polarität der Zusammensetzung (B) optimal eingestellt werden und die Polymerisationsgeschwindigkeit der C₁-C₆-Alkoxysilane besonders gut an die jeweils gewählten Anwendungsbedingungen angepasst werden.

In diesem Zusammenhang hat sich herausgestellt, dass insbesondere der Einsatz mindestens eines C₁₂-C₃₀-Fettalkohols (B2) in der Zusammensetzung (B) ein Emulsions-System schafft, in das die Alkoxysilane (A2) besonders gut eingebettet werden können.

Im Rahmen einer Ausführungsform wurden ganz besonders gute Ergebnisse erhalten, wenn die zweite Zusammensetzung (B) einen oder mehrere C₁₂-C₃₀-Fettalkohole aus der Gruppe aus Dodecan-1-ol (Dodecylalkohol, Laurylalkohol), Tetradecan-1-ol (Tetradecylalkohol, Myristylalkohol), Hexadecan-1-ol (Hexadecylalkohol, Cetylalkohol, Palmitylalkohol), Octadecan-1-ol (Octadecylalkohol, Stearylalkohol), Arachylalkohol (Eicosan-1-ol), Heneicosylalkohol (Heneicosan-1-ol), Behenylalkohol (Docosan-1-ol), (9Z)-Octadec-9-en-1-ol (Oleylalkohol), (9E)-Octadec-9-en-1-ol (Elaidylalkohol), (9Z,12Z)-Octadeca-9,12-dien-1-ol (Linoleylalkohol), (9Z, 12Z, 15Z)-Octadeca-9,12,15-trien-1-ol (Linolenoylalkohol), Gadoleylalkohol ((9Z)-Eicos-9-en-1-ol), Arachidonalkohol ((5Z,8Z,11Z,14Z)-Eicosa-5,8,11,14-tetraen-1-ol), Erucylalkohol ((13Z)-Docos-13-en-1-ol), Brassidylalkohol ((13E)-Docosen-1-ol) 2-Octyl-dodecanol, 2-Hexyl-Dodecanol und/oder 2-Butyl-dodecanol enthält.

Im Rahmen einer ganz besonders bevorzugten Ausführungsform ist ein erfindungsgemäßes Verfahren dadurch gekennzeichnet, dass die zweite Zusammensetzung (B) ein oder mehrere C₁₂-C₃₀-Fettalkohole (B2) aus der Gruppe aus
Dodecan-1-ol (Dodecylalkohol, Laurylalkohol),
Tetradecan-1-ol (Tetradecylalkohol, Myristylalkohol),
Hexadecan-1-ol (Hexadecylalkohol, Cetylalkohol, Palmitylalkohol),
Octadecan-1-ol (Octadecylalkohol, Stearylalkohol),
Arachylalkohol (Eicosan-1-ol),
Heneicosylalkohol (Heneicosan-1-ol),
Behenylalkohol (Docosan-1-ol),
(9Z)-Octadec-9-en-1-ol (Oleylalkohol),
(9E)-Octadec-9-en-1-ol (Elaidylalkohol),
(9Z,12Z)-Octadeca-9,12-dien-1-ol (Linoleylalkohol),
(9Z,12Z,15Z)-Octadeca-9,12,15-trien-1-ol (Linolenoylalkohol),
Gadoleylalkohol ((9Z)-Eicos-9-en-1-ol),
Arachidonalkohol ((5Z,8Z,11Z,14Z)-Eicosa-5,8,11,14-tetraen-1-ol),
Erucylalkohol ((13Z)-Docos-13-en-1-ol),
Brassidylalkohol ((13E)-Docosen-1-ol),
2-Octyl-dodecanol,
2-Hexyl-Dodecanol und/oder
2-Butyl-dodecanol enthält.

Durch Wahl der geeigneten Einsatzmengen an C₁₂-C₃₀-Fettalkoholen (B2) kann die Geschwindigkeit der von den C₁-C₆-Alkoxy-Silanen ausgehenden Filmbildung besonders stark mitbestimmt werden. Aus diesem Grund hat es sich als ganz besonders bevorzugt erwiesen, ein oder mehrere C₁₂-C₃₀-Fettalkohole (B2) in ganz bestimmten Mengenbereichen einzusetzen.

Es ist ganz besonders bevorzugt, wenn die zweite Zusammensetzung (B) - bezogen auf das Gesamtgewicht der Zusammensetzung (B) - ein oder mehrere C₁₂-C₃₀-Fettalkohole (B2) in einer Gesamtmenge von 2,0 bis 50,0 Gew.-%, bevorzugt von 3,0 bis 40,0 Gew.-%, weiter bevorzugt von 4,0 bis 30,0 Gew.-%, noch weiter bevorzugt von 5,0 bis 20,0 Gew.-% und ganz besonders bevorzugt von 6,0 bis 15,0 Gew.-% enthält.

Im Rahmen einer weiteren ganz besonders bevorzugten Ausführungsform ist ein erfindungsgemäßes Verfahren dadurch gekennzeichnet, dass die zweite Zusammensetzung (B) - bezogen auf das Gesamtgewicht der Zusammensetzung (B) - einen oder mehrere C₁₂-C₃₀-Fettalkohole (B2) in einer Gesamtmenge von 2,0 bis 50,0 Gew.-%, bevorzugt von 3,0 bis 40,0 Gew.-%, weiter bevorzugt von 4,0 bis 30,0 Gew.-%, noch weiter bevorzugt von 5,0 bis 20,0 Gew.-% und ganz besonders bevorzugt von 6,0 bis 15,0 Gew.-% enthält.

Weiterhin als ganz besonders bevorzugten Fettbestandteil (B2) kann die Zusammensetzung (B) auch mindestens ein C₁₂-C₃₀-Fettsäuretriglycerid, der C₁₂-C₃₀-Fettsäuremonoglycerid und/oder C₁₂-C₃₀-Fettsäurediglycerid enthalten. Unter einem C₁₂-C₃₀-Fettsäuretriglycerid wird im Sinne der vorliegenden Erfindung der Triester des dreiwertigen Alkohols Glycerin mit drei Äquivalenten Fettsäure verstanden. Dabei können sowohl strukturgleiche als auch unterschiedliche Fettsäuren innerhalb eines Triglyceridmoleküls an den Esterbildungen beteiligt sein.

Unter Fettsäuren sind erfindungsgemäß gesättigte oder ungesättigte, unverzweigte oder verzweigte, unsubstituierte oder substituierte C₁₂-C₃₀-Carbonsäuren zu verstehen. Ungesättigte Fettsäuren können einfach oder mehrfach ungesättigt sein. Bei einer ungesättigten Fettsäure kann bzw. können deren C-C-Doppelbindung(en) die Cis- oder Trans-Konfiguration aufweisen.

Es zeichnen sich die Fettsäuretriglyceride durch besondere Eignung aus, bei welchen mindestens eine der Estergruppen ausgehend von Glycerin mit einer Fettsäure ausgebildet wird, die ausgewählt wird aus Dodecansäure (Laurinsäure), Tetradecansäure (Myristinsäure), Hexadecansäure (Palmitinsäure), Tetracosansäure (Lignocerinsäure), Octadecansäure (Stearinsäure), Eicosansäure (Arachinsäure), Docosansäure (Behensäure), Petroselinsäure [(Z)-6-Octadecensäure], Palmitoleinsäure [(9Z)-Hexadec-9-ensäure], Ölsäure [(9Z)-Octadec-9-ensäure], Elaidinäsure [(9E)-Octadec-9-ensäure], Erucasäure [(13Z)-Docos-13-ensäure], Linolsäure [(9Z, 12Z)-Octadeca-9,12-diensäure, Linolensäure [(9Z,12Z,15Z)-Octadeca-9,12,15-triensäure, Elaeostearinäure [(9Z,11E,13E)-Octadeca-9,11,3-triensäure], Arachidonsäure [(5Z,8Z,11Z,14Z)-Icosa-5,8,11,14-tetraensäure] und/oder Nervonsäure [(15Z)-Tetracos-15-ensäure].

Die Fettsäuretriglyceride können auch natürlichen Ursprungs sein. Die in Sojaöl, Erdnußöl, Olivenöl, Sonnenblumenöl, Macadamianussöl, Moringaöl, Aprikosenkernöl, Marulaöl und/oder gegebenenfalls gehärtetem Rizinusöl vorkommenden Fettsäure-Triglyceride bzw. deren Gemische sind zum Einsatz im erfindungsgemäßen Produkt besonders geeignet.

Unter einem C₁₂-C₃₀-Fettsäuremonoglycerid wird der Monoester des dreiwertigen Alkohols Glycerin mit einem Äquivalent Fettsäure verstanden. Hierbei kann entweder die mittlere Hydroxygruppe des Glycerins oder die endständige Hydroxygruppe des Glycerins mit der Fettsäure verestert sein.

Es zeichnen sich die C₁₂-C₃₀-Fettsäuremonoglycerid durch besondere Eignung aus, bei welchen eine Hydroxygruppe des Glycerins mit einer Fettsäure verestert wird, wobei die Fettsäuren ausgewählt sind aus Dodecansäure (Laurinsäure), Tetradecansäure (Myristinsäure), Hexadecansäure (Palmitinsäure), Tetracosansäure (Lignocerinsäure), Octadecansäure (Stearinsäure), Eicosansäure (Arachinsäure), Docosansäure (Behensäure), Petroselinsäure [(Z)-6-Octadecensäure], Palmitoleinsäure [(9Z)-Hexadec-9-ensäure], Ölsäure [(9Z)-Octadec-9-ensäure], Elaidinäsure [(9E)-Octadec-9-ensäure], Erucasäure [(13Z)-Docos-13-ensäure], Linolsäure [(9Z, 12Z)-Octadeca-9,12-diensäure, Linolensäure [(9Z,12Z,15Z)-Octadeca-9,12,15-triensäure, Elaeostearinäure [(9Z,11E,13E)-Octadeca-9,11,3-triensäure], Arachidonsäure [(5Z,8Z,11Z,14Z)-lcosa-5,8,11,14-tetraensäure] oder Nervonsäure [(15Z)-Tetracos-15-ensäure].

Unter einem C₁₂-C₃₀-Fettsäurediglycerid wird der Diester des dreiwertigen Alkohols Glycerin mit zwei Äquivalenten Fettsäure verstanden. Hierbei können entweder die mittlere und eine endständige Hydroxygruppe des Glycerins mit zwei Äquivalenten Fettsäure verestert sein, oder aber beide endständigen Hydroxygruppen des Glycerins sind mit jeweils einer Fettsäure verestert. Das Glycerin kann hierbei sowohl mit zwei strukturgleichen als auch mit zwei unterschiedlichen Fettsäuren verestert sein.

Es zeichnen sich die Fettsäurediglyceride durch besondere Eignung aus, bei welchen mindestens eine der Estergruppen ausgehend von Glycerin mit einer Fettsäure ausgebildet wird, die ausgewählt wird aus Dodecansäure (Laurinsäure), Tetradecansäure (Myristinsäure), Hexadecansäure (Palmitinsäure), Tetracosansäure (Lignocerinsäure), Octadecansäure (Stearinsäure), Eicosansäure (Arachinsäure), Docosansäure (Behensäure), Petroselinsäure [(Z)-6-Octadecensäure], Palmitoleinsäure [(9Z)-Hexadec-9-ensäure], Ölsäure [(9Z)-Octadec-9-ensäure], Elaidinäsure [(9E)-Octadec-9-ensäure], Erucasäure [(13Z)-Docos-13-ensäure], Linolsäure [(9Z, 12Z)-Octadeca-9,12-diensäure, Linolensäure [(9Z,12Z,15Z)-Octadeca-9,12,15-triensäure, Elaeostearinäure [(9Z,11E,13E)-Octadeca-9,11,3-triensäure], Arachidonsäure [(5Z,8Z,11Z,14Z)-Icosa-5,8,11,14-tetraensäure] und/oder Nervonsäure [(15Z)-Tetracos-15-ensäure].

Ganz besonders gute Ergebnisse wurden erhalten, wenn die Zusammensetzung (B) mindestens ein C₁₂-C₃₀-Fettsäuremonoglycerid enthielt, welches ausgewählt ist aus den Monoestern von Glycerin mit einem Äquivalent Fettsäure aus der Gruppe aus Dodecansäure (Laurinsäure), Tetradecansäure (Myristinsäure), Hexadecansäure (Palmitinsäure), Tetracosansäure (Lignocerinsäure), Octadecansäure (Stearinsäure), Eicosansäure (Arachinsäure), Docosansäure (Behensäure), Petroselinsäure [(Z)-6-Octadecensäure], Palmitoleinsäure [(9Z)-Hexadec-9-ensäure], Ölsäure [(9Z)-Octadec-9-ensäure], Elaidinäsure [(9E)-Octadec-9-ensäure], Erucasäure [(13Z)-Docos-13-ensäure], Linolsäure [(9Z, 12Z)-Octadeca-9,12-diensäure, Linolensäure [(9Z,12Z,15Z)-Octadeca-9,12,15-triensäure, Elaeostearinäure [(9Z,11E,13E)-Octadeca-9,11,3-triensäure], Arachidonsäure [(5Z,8Z,11Z,14Z)-Icosa-5,8,11,14-tetraensäure] und/oder Nervonsäure [(15Z)-Tetracos-15-ensäure],

Im Rahmen einer ganz besonders bevorzugten Ausführungsform ist ein erfindungsgemäßes Verfahren dadurch gekennzeichnet, dass die zweite Zusammensetzung (B) mindestens ein C₁₂-C₃₀-Fettsäuremonoglycerid (B2) enthält, welches ausgewählt ist aus den Monoestern von Glycerin mit einem Äquivalent Fettsäure aus der Gruppe aus Dodecansäure, Tetradecansäure, Hexadecansäure, Tetracosansäure, Octadecansäure, Eicosansäure und/oder Docosansäure.

Auch durch Wahl der geeigneten Einsatzmengen an C₁₂-C₃₀-Fettsäuremono-, C₁₂-C₃₀-Fettsäuredi- und/oder C₁₂-C₃₀-Fettsäuretriglyceriden kann die Geschwindigkeit der von den C₁-C₆-Alkoxy-Silanen ausgehenden Filmbildung besonders stark mitbestimmt werden. Aus diesem Grund hat es sich als ganz besonders bevorzugt erwiesen, ein oder mehrere C₁₂-C₃₀-Fettsäuremono-, C₁₂-C₃₀-Fettsäuredi- und/oder C₁₂-C₃₀-Fettsäuretriglyceride (B2) in ganz bestimmten Mengenbereichen in der Zusammensetzung (B) einzusetzen.

Im Hinblick auf die Lösung der erfindungsgemäßen Aufgabenstellung hat es sich als ganz besonders bevozugt erwiesen, wenn die zweite Zusammensetzung (B) - bezogen auf das Gesamtgewicht der Zusammensetzung (B) - ein oder mehrere C₁₂-C₃₀-Fettsäuremono-, C₁₂-C₃₀-Fettsäuredi- und/oder C₁₂-C₃₀-Fettsäuretriglyceride (B2) in einer Gesamtmenge von 0,1 bis 20,0 Gew.-%, bevorzugt 0,3 bis 15,0 Gew.-%, weiter bevorzugt 0,5 bis 10,0 Gew.-% und ganz besonders bevorzugt von 0,8 bis 5,0 Gew.-% enthielt.

Im Rahmen einer ganz besonders bevorzugten Ausführungsform ist ein erfindungsgemäßes Verfahren dadurch gekennzeichnet, dass die zweite Zusammensetzung (B) - bezogen auf das Gesamtgewicht der Zusammensetzung (B) - ein oder mehrere C₁₂-C₃₀-Fettsäuremono-, C₁₂-C₃₀-Fettsäuredi- und/oder C₁₂-C₃₀-Fettsäuretriglyceride (B2) in einer Gesamtmenge von 0,1 bis 20,0 Gew.-%, bevorzugt 0,3 bis 15,0 Gew.-%, weiter bevorzugt 0,5 bis 10,0 Gew.-% und ganz besonders bevorzugt von 0,8 bis 5,0 Gew.-% enthält.

Die C₁₂-C₃₀-Fettsäuremono-, C₁₂-C₃₀-Fettsäuredi- und/oder C₁₂-C₃₀-Fettsäuretriglyceride können als alleinige Fettbestandteile (B2) in den Zusammensetzungen (B) eingesetzt werden. Es ist jedoch ganz besonders beovrzugt, mindestens ein C₁₂-C₃₀-Fettsäuremono-, C₁₂-C₃₀-Fettsäuredi- und/oder C₁₂-C₃₀-Fettsäuretriglycerid in Kombination mit mindestens einem C₁₂-C₃₀-Fettalkohol in die Zusammensetzung (B) einzuarbeiten.

Weiterhin als ganz besonders bevorzugten Fettbestandteil (B2) kann die Zusammensetzung (B) auch mindestens einen Kohlenwasserstoff enthalten.

Kohlenwasserstoffe sind ausschließlich aus den Atomen Kohlenstoff und Wasserstoff bestehende Verbindungen mit 8 bis 80 C-Atomen. Bevorzugt sind in diesem Zusammenhang insbesondere aliphatische Kohlenwasserstoffe wie beispielsweise Mineralöle, flüssige Paraffinöle (z.B. Paraffinum Liquidum oder Paraffinum Perliquidum), Isoparaffinöle, halbfeste Paraffinöle, Paraffinwachse, Hartparaffin (Paraffinum Solidum), Vaseline und Polydecene.

Als besonders geeignet haben sich in diesem Zusammenhang flüssige Paraffinöle (Paraffinum Liquidum und Paraffinium Perliquidum) erwiesen. Ganz besonders bevorzugt handelt es sich bei dem Kohlenwasserstoff um Paraffinum Liquidum, auch Weißöl genannt. Bei Paraffinum Liquidum handelt es sich um ein Gemisch gereinigter, gesättigter, aliphatischer Kohlenwasserstoffe, das größtenteils aus Kohlenwasserstoffketten mit einer C-Kettenverteilung von 25 bis 35 C-Atomen besteht.

Ganz besonders gute Ergebnisse wurden erhalten, wenn die Zusammensetzung (B) mindestens einen Kohlenwasserstoff (B2) enthielt, der ausgewählt ist aus der Gruppe der Mineralöle, der flüssige Paraffinöle, Isoparaffinöle, halbfeste Paraffinöle, Paraffinwachse, Hartparaffin (Paraffinum Solidum), Vaseline und Polydecene.

Im Rahmen einer ganz besonders bevorzugten Ausführungsform ist ein erfindungsgemäßes Verfahren dadurch gekennzeichnet, dass die zweite Zusammensetzung (B) mindestens einen Fettbestandteile (B2) aus der Gruppe der Kohlenwasserstoffe enthält.

Auch durch Wahl der geeigneten Einsatzmengen an Kohlenwasserstoffen kann die Geschwindigkeit der von den C₁-C₆-Alkoxy-Silanen ausgehenden Filmbildung besonders stark mitbestimmt werden. Aus diesem Grund hat es sich als ganz besonders bevorzugt erwiesen, ein oder mehrere Kohlenwasserstoffe in ganz bestimmten Mengenbereichen in der Zusammensetzung (B) einzusetzen.

Im Hinblick auf die Lösung der erfindungsgemäßen Aufgabenstellung hat es sich als ganz besonders bevozugt erwiesen, wenn die zweite Zusammensetzung (B) - bezogen auf das Gesamtgewicht der Zusammensetzung (B) - ein oder mehrere Kohlenwasserstoffe (B2) in einer Gesamtmenge von 0,5 bis 20,0 Gew.-%, bevorzugt 1,0 bis 15,0 Gew.-%, weiter bevorzugt von 1,5 bis 10,0 Gew.-% und ganz besonders bevorzugt von 2,0 bis 8,0 Gew.-% enthielt.

Im Rahmen einer ganz besonders bevorzugten Ausführungsform ist ein erfindungsgemäßes Verfahren dadurch gekennzeichnet, dass die zweite Zusammensetzung (B) - bezogen auf das Gesamtgewicht der Zusammensetzung (B) - einen oder mehrere Kohlenwasserstoffe (B2) in einer Gesamtmenge von 0,5 bis 20,0 Gew.-%, bevorzugt 1,0 bis 15,0 Gew.-%, weiter bevorzugt von 1,5 bis 10,0 Gew.-% und ganz besonders bevorzugt von 2,0 bis 8,0 Gew.-% enthält.

Der oder die Kohlenwasserstoffe können als alleinige Fettbestandteile (B2) in den Zusammensetzungen (B) eingesetzt werden. Es ist jedoch ganz besonders beovrzugt, mindestens einen Kohlenwasserstoff in Kombination mit mindestens einem weiteren Bestandteil in die Zusammensetzungen (B) einzuarbeiten.

Ganz besonders bevorzugt enthält die Zusammensetzung (B) mindestens einen Fettbestandteil (B2) aus der Gruppe der C₁₂-C₃₀-Fettalkohole und mindestens einen weiteren Fettbestandteil aus der Gruppe der Kohlenwasserstoffe.

### Tenside in der Zusammensetzung (B)

Bedingt durch ihren Gehalt an Wasser (B1) und Fettbestandteil (B2) liegt die Zusammensetzung (B) in Form einer Emulsion vor. Um die Ausbildung der Emulsion weiter zu optimieren, hat es sich als ganz besonders bevorzugt erwiesen, in der Zusammensetzung (B) weiterhin mindestens ein Tensid einzusetzen. Ganz besonders bevorzugt enthält die Zusammensetzung (B) deshalb zusätzlich optional mindestens ein Tensid.

Im Rahmen einer weiteren besonders bevorzugten Ausführungsform ist ein erfindungsgemäßes Verfahren dadurch gekennzeichnet, dass die zweite Zusammensetzung (B) mindestens ein Tensid, enthält.

Unter dem Begriff Tenside (T) werden grenzflächenaktive Substanzen, die an Ober- und Grenzflächen Adsorptionsschichten bilden oder in Volumenphasen zu Mizell-Kolloiden oder lyotropen Mesophasen aggregieren können, verstanden. Man unterscheidet anionische Tenside bestehend aus einem hydrophoben Rest und einer negativ geladenen hydrophilen Kopfgruppe, amphotere Tenside, welche sowohl eine negative als auch eine kompensierende positive Ladung tragen, kationische Tenside, welche neben einem hydrophoben Rest eine positiv geladene hydrophile Gruppe aufweisen, und nichtionische Tenside, welche keine Ladungen sondern starke Dipolmomente aufweisen und in wässriger Lösung stark hydratisiert sind.

Im Rahmen einer ganz besonders bevorzugten Ausführungsform ist ein erfindungsgemäßes Verfahren dadurch gekennzeichnet, dass die zweite Zusammensetzung (B) mindestens ein Tensid, besonders bevorzugt mindestens ein nichtionisches Tensid, enthält.

Nichtionische Tenside enthalten beipsielsweise als hydrophile Gruppe eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 50 Mol Ethylenoxid und/oder 0 bis 5 Mol Propylenoxid an lineare und verzweigte Fettalkohole mit 6 bis 30 C-Atomen, die Fettalkoholpolyglykolether bzw. die Fettalkoholpolypropylenglykolether bzw. gemischte Fettalkoholpolyether,
- Anlagerungsprodukte von 2 bis 50 Mol Ethylenoxid und/oder 0 bis 5 Mol Propylenoxid an lineare und verzweigte Fettsäuren mit 6 bis 30 C-Atomen, die Fettsäurepolyglykolether bzw. die Fettsäurepolypropylenglykolether bzw. gemischte Fettsäurepolyether,
- Anlagerungsprodukte von 2 bis 50 Mol Ethylenoxid und/oder 0 bis 5 Mol Propylenoxid an lineare und verzweigte Alkylphenole mit 8 bis 15 C-Atomen in der Alkylgruppe, die Alkylphenolpolyglykolether bzw. die Alkylpolypropylenglykolether, bzw. gemischte Alyklphenolpolyether,
- mit einem Methyl- oder C₂ - C₆ - Alkylrest endgruppenverschlossene Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, wie beispielsweise die unter den Verkaufsbezeichnungen Dehydol^{®} LS, Dehydol^{®} LT (Cognis) erhältlichen Typen,
- C₁₂-C₃₀-Fettsäuremono- und -diester von Anlagerungsprodukten von 1 bis 30 Mol Ethylenoxid an Glycerin,
- Anlagerungsprodukte von 5 bis 60 Mol Ethylenoxid an Rizinusöl und gehärtetes Rizinusöl,
- Polyolfettsäureester, wie beispielsweise das Handelsprodukt Hydagen^{®} HSP (Cognis) oder Sovermol^{®}-Typen (Cognis),
- alkoxilierte Triglyceride,
- alkoxilierte Fettsäurealkylester der Formel (Tnio-1)

   R¹CO-(OCH₂CHR²)_{w}OR³ (Tnio-1)

   in der R¹CO für einen linearen oder verzweigten, gesättigten und/oder ungesättigten Acylrest mit 6 bis 22 Kohlenstoffatomen, R² für Wasserstoff oder Methyl, R³ für lineare oder verzweigte Alkylreste mit 1 bis 4 Kohlenstoffatomen und w für Zahlen von 1 bis 20 steht,
- Aminoxide,
- Hydroxymischether, wie sie beispielsweise in der DE-OS 19738866 beschrieben sind,
- Sorbitanfettsäureester und Anlagerungeprodukte von Ethylenoxid an Sorbitanfettsäureester wie beispielsweise die Polysorbate,
- Zuckerfettsäureester und Anlagerungsprodukte von Ethylenoxid an Zuckerfettsäureester,
- Anlagerungsprodukte von Ethylenoxid an Fettsäurealkanolamide und Fettamine,
- Zuckertenside vom Typ der Alkyl- und Alkenyloligoglykoside gemäß Formel (E4-II),

   R⁴O-[G]ₚ (Tnio-2)

   in der R⁴ für einen Alkyl- oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden. Die Alkyl- und Alkenyloligoglykoside können sich von Aldosen bzw. Ketosen mit 5 oder 6 Kohlenstoffatomen, vorzugsweise von Glucose, ableiten. Die bevorzugten Alkyl- und/oder Alkenyloligoglykoside sind somit Alkyl- und/oder Alkenyloligoglucoside. Die Indexzahl p in der allgemeinen Formel (Tnio-2) gibt den Oligomerisierungsgrad (DP), d. h. die Verteilung von Mono- und Oligoglykosiden an und steht für eine Zahl zwischen 1 und 10. Während p im einzelnen Molekül stets ganzzahlig sein muß und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt. Der Alkyl- bzw. Alkenylrest R⁴kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestern oder im Verlauf der Hydrierung von Aldehyden aus der Roelen'schen Oxosynthese erhalten werden. Bevorzugt sind Alkyloligoglucoside der Kettenlänge C₈-C₁₀ (DP = 1 bis 3), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkyloligoglucoside auf Basis technischer C_{9/11}-Oxoalkohole (DP = 1 bis 3). Der Alkyl- bzw. Alkenylrest R¹⁵ kann sich ferner auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, Brassidylalkohol sowie deren technische Gemische, die wie oben beschrieben erhalten werden können. Bevorzugt sind Alkyloligoglucoside auf Basis von gehärtetem C_{12/14}-Kokosalkohol mit einem DP von 1 bis 3.
- Zuckertenside vom Typ der Fettsäure-N-alkylpolyhydroxyalkylamide, ein nichtionisches Tensid der Formel (Tnio-3),

   R⁵CO-NR⁶-[Z] (Tnio-3)

   in der R⁵CO für einen aliphatischen Acylrest mit 6 bis 22 Kohlenstoffatomen, R⁶ für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 12 Kohlenstoffatomen und 3 bis 10 Hydroxylgruppen steht. Bei den Fettsäure-N-alkylpolyhydroxyalkylamiden handelt es sich um bekannte Stoffe, die üblicherweise durch reduktive Aminierung eines reduzierenden Zuckers mit Ammoniak, einem Alkylamin oder einem Alkanolamin und nachfolgende Acylierung mit einer Fettsäure, einem Fettsäurealkylester oder einem Fettsäurechlorid erhalten werden können. Vorzugsweise leiten sich die Fettsäure-N-alkylpolyhydroxyalkylamide von reduzierenden Zuckern mit 5 oder 6 Kohlenstoffatomen, insbesondere von der Glucose ab. Die bevorzugten Fettsäure-N-alkylpolyhydroxyalkylamide stellen daher Fettsäure-N-alkylglucamide dar, wie sie durch die Formel (Tnio-4) wiedergegeben werden:

   R⁷CO-(NR⁸)-CH₂-[CH(OH)]₄-CH₂OH (Tnio-4)

   Vorzugsweise werden als Fettsäure-N-alkylpolyhydroxyalkylamide Glucamide der Formel (Tnio-4) eingesetzt, in der R⁸ für Wasserstoff oder eine Alkylgruppe steht und R⁷CO für den Acylrest der Capronsäure, Caprylsäure, Caprinsäure, Laurinsäure, Myristinsäure, Palmitinsäure, Palmoleinsäure, Stearinsäure, Isostearinsäure, Ölsäure, Elaidinsäure, Petroselinsäure, Linolsäure, Linolensäure, Arachinsäure, Gadoleinsäure, Behensäure oder Erucasäure bzw. derer technischer Mischungen steht. Besonders bevorzugt sind Fettsäure-N-alkylglucamide der Formel (Tnio-4), die durch reduktive Aminierung von Glucose mit Methylamin und anschließende Acylierung mit Laurinsäure oder C12/14-Kokosfettsäure beziehungsweise einem entsprechenden Derivat erhalten werden. Weiterhin können sich die Polyhydroxyalkylamide auch von Maltose und Palatinose ableiten.

Die Zuckertenside können in den erfindungsgemäß verwendeten Mitteln bevorzugt in Mengen von 0,1 - 20 Gew.-%, bezogen auf das gesamte Mittel, enthalten sein. Mengen von 0,5 - 15 Gew.-% sind bevorzugt, und ganz besonders bevorzugt sind Mengen von 0,5 - 7,5 Gew.-%.

Weitere typische Beispiele für nichtionische Tenside sind Fettsäureamidpolyglycolether, Fettaminpolyglycolether, Mischether bzw. Mischformale, Proteinhydrolysate (insbesondere pflanzliche Produkte auf Weizenbasis) und Polysorbate.

Als bevorzugte nichtionische Tenside haben sich die Alkylenoxid-Anlagerungsprodukte an gesättigte lineare Fettalkohole und Fettsäuren mit jeweils 2 bis 30 Mol Ethylenoxid pro Mol Fettalkohol bzw. Fettsäure sowie die Zuckertenside erwiesen. Zubereitungen mit hervorragenden Eigenschaften werden ebenfalls erhalten, wenn sie als nichtionische Tenside Fettsäureester von ethoxyliertem Glycerin enthalten.

Diese Verbindungen sind durch die folgenden Parameter gekennzeichnet. Der Alkylrest R enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so dass man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Ganz besonders gute Ergebnisse wurden erhalten, wenn im erfindungsgemäßen Verfahren eine zweite Zusammensetzung (B) eingesetzt wurde, welche mindestens einen ethoxylierten Fettalkohol mit einem Ethoxylierungsgrad von 10 bis 40 enthielt.

In einer weiteren ganz besonders bevorzugten Ausführungsform ist ein erfindungsgemäßes Verfahren dadurch gekennzeichnet, dass die zweite Zusammensetzung (B) mindestens ein nichtionisches Tensid der Formel (T-I) enthält, 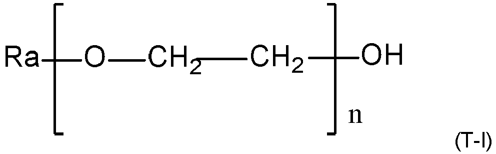 worin
- Ra: für eine gesättigte oder ungesättigte, unverzweigte oder verzweigte C₈-C₂₄-Alkylgruppe, bevorzugt für eine gesättigte, unverzweigte C₁₆- bis C₁₈- Alkylgruppe, steht und
- n: für eine ganze Zahl von 10 bis 40, bevorzugt für eine ganze Zahl von 20 bis 35 und besonders bevorzugt für die Zahl 30, steht.

Bei einem besonders gut geeigneten nichtionischen Tensid dieses Typs handelt es sich um Ceteareth-30. Bei Ceteareth-30 handelt es sich um ein Gemisch aus Cetylalkohol und Stearylalkohol, die jeweils mit 30 Einheiten Ethylenoxid ethoxyliert sind. Das Gemisch aus Cetylalkohol und Stearylalkohol wird als Cetearylalkohol bezeichnet. Ceteareth-30 besitzt die CAS-Nummer 68439-49-6 und ist beispielsweise unter dem Handelsnamen Eumulgin B3 von der Firma BASF käuflich zu erwerben.

Die nichtionischen Tenside der Formel (T-I) können in der erfindungsgemäß verwendeten Zubereitung (B) bevorzugt in Mengen von 0,1 - 20 Gew.-%, bezogen auf das Gesamtgewicht der Zubereitung (B), enthalten sein. Mengen von 0,5 - 15 Gew.-% sind bevorzugt, und ganz besonders bevorzugt sind Mengen von 0,5 - 7,5 Gew.-%.

### Farbgebende Verbindungen

Als dritten erfindungswesentlichen Bestandteil (B3) enthält die erfindungsgemäße Zusammensetzung (B) mindestens eine farbgebende Verbindung aus der Gruppe der Pigmente und/oder der direktziehenden Farbstoffe.

Das oder die farbgebenden Verbindungen können vorzugsweise ausgewählt werden aus der Pigmente und/oder der direktziehenden Farbstoffe. In die Gruppe der direktziehenden Farbstoffe fallan auch die photochromen Farbstoffe die thermochromen Farbstoffe.

Unter Pigmenten im Sinne der vorliegenden Erfindung werden farbgebende Verbindungen verstanden, welche bei 25 °C in Wasser eine Löslichkeit von weniger als 0,5 g/L, bevorzugt von weniger als 0,1 g/L, noch weiter bevorzugt von weniger als 0,05 g/L besitzen. Die Wasserlöslichkeit kann beispielsweise mittels der nachfolgend beschriebenen Methode erfolgen: 0,5 g des Pigments werden in einem Becherglas abgewogen. Ein Rührfisch wird hinzugefügt. Dann wird ein Liter destilliertes Wasser hinzugegeben. Dieses Gemisch wird unter Rühren auf einem Magnetrührer für eine Stunde auf 25 °C erhitzt. Sind in der Mischung nach diesem Zeitraum noch ungelöste Bestandteile des Pigments sichtbar, so liegt die Löslichkeit des Pigments unterhalb von 0,5 g/L. Sofern sich die Pigment-Wasser-Mischung aufgrund der hohen Intensität des gegebenenfalls feindispergiert vorliegenden Pigments nicht visuell beurteilten lässt, wird die Mischung filtriert. Bleibt auf dem Filterpapier ein Anteil an ungelösten Pigmenten zurück, so liegt die Löslichkeit des Pigments unterhalb von 0,5 g/L.

Geeignete Farbpigmente können anorganischen und/oder organischen Ursprungs sein.

In einer bevorzugten Ausführungsform ist en erfindungsgemäßes Mittel dadurch gekennzeichnet, dass es mindestens eine farbgebende Verbindung aus der Gruppe der anorganischen und/oder organischen Pigmente enthält.

Bevorzugte Farbpigmente sind ausgewählt aus synthetischen oder natürlichen anorganischen Pigmenten. Anorganische Farbpigmente natürlichen Ursprungs können beispielsweise aus Kreide, Ocker, Umbra, Grünerde, gebranntem Terra di Siena oder Graphit hergestellt werden. Weiterhin können als anorganische Farbpigmente Schwarzpigmente wie z. B. Eisenoxidschwarz, Buntpigmente wie z. B. Ultramarin oder Eisenoxidrot sowie Fluoreszenz- oder Phosphoreszenzpigmente eingesetzt werden.

Besonders geeignet sind farbige Metalloxide, -hydroxide und -oxidhydrate, Mischphasenpigmente, schwefelhaltige Silicate, Silicate, Metallsulfide, komplexe Metallcyanide, Metallsulfate, -chromate und/oder -molybdate. Auch der Einsatz von Pigmenten aus Metallen oder Metalllegierungen sind besonders gut geeignet. Insbesondere bevorzugte Farbpigmente sind schwarzes Eisenoxid (CI 77499), gelbes Eisenoxid (CI 77492), rotes und braunes Eisenoxid (CI 77491), Manganviolett (CI 77742), Ultramarine (Natrium-Aluminiumsulfosilikate, CI 77007, Pigment Blue 29), Chromoxidhydrat (CI77289), Eisenblau (Ferric Ferrocyanide, CI77510) und/oder Carmine (Cochineal).

Erfindungsgemäß ebenfalls besonders bevorzugte farbgebende Verbindungen aus der Gruppe der Pigmente sind farbige Perlglanzpigmente. Diese basieren üblicherweise auf Mica- und/oder Glimmerbasis und können mit einem oder mehreren Metalloxiden beschichtet sein. Glimmer gehört zu den Schicht-Silicaten. Die wichtigsten Vertreter dieser Silicate sind Muscovit, Phlogopit, Paragonit, Biotit, Lepidolith und Margarit. Zur Herstellung der Perlglanzpigmente in Verbindung mit Metalloxiden wird der Glimmer, überwiegend Muscovit oder Phlogopit, mit einem Metalloxid beschichtet.

Im Rahmen einer ganz besonders bevorzugten Ausführungsform ist ein erfindungsgemäßes Verfahren dadurch gekennzeichnet, dass die zweite Zusammensetzung (B) mindestens eine farbgebende Verbindung aus der Gruppe aus der Gruppe der anorganischen Pigmente enthält, die ausgewählt ist aus der Gruppe der farbigen Metalloxide, Metallhydroxide, Metalloxidhydrate, Silicate, Metallsulfide, komplexen Metallcyanide, Metallsulfate, Bronzepigmente, Metalle, Metalllegierungen und/oder aus farbigen Pigmenten auf Mica- oder Glimmerbasis, die mit mindestens einem Metalloxid und/oder einem Metalloxychlorid beschichtet sind.

Alternativ zu natürlichem Glimmer kann auch ggfs. mit einem oder mehrere Metalloxide(en) beschichtetes, synthetisches Mica als Perlglanzpigment verwendet werden. Besonders bevorzugte Perlglanzpigmente basieren auf natürlichem oder synthetischem Mica (Glimmer) und sind mit einem oder mehreren der zuvor genannten Metalloxide beschichtet. Die Farbe der jeweiligen Pigmente kann durch Variation der Schichtdicke des oder der Metalloxids(e) variiert werden.

In einer weiteren bevorzugten Ausführungsform ist die erfindungsgemäße Zusammensetzung (B) dadurch gekennzeichnet, dass sie mindestens eine farbgebende Verbindung aus der Gruppe der Pigmente enthält, die ausgewählt ist aus der Gruppe der farbigen Metalloxide, Metallhydroxide, Metalloxidhydrate, Silicate, Metallsulfide, komplexen Metallcyanide, Metallsulfate, Bronzepigmente und/oder aus farbgebenden Verbindungen auf Mica- oder Glimmerbasis, die mit mindestens einem Metalloxid und/oder einem Metalloxychlorid beschichtet sind.

In einer weiteren bevorzugten Ausführungsform ist eine erfindungsgemäße Zusammensetzung (B) dadurch gekennzeichnet, dass sie mindestens eine farbgebende Verbindung enthält, die ausgewählt ist aus Pigmenten auf Mica- oder Glimmerbasis, die mit einem oder mehreren Metalloxiden aus der Gruppe aus Titandioxid (CI 77891), schwarzem Eisenoxid (CI 77499), gelbem Eisenoxid (CI 77492), rotem und/oder braunem Eisenoxid (CI 77491, CI 77499), Manganviolett (CI 77742), Ultramarine (Natrium-Aluminiumsulfosilikate, CI 77007, Pigment Blue 29), Chromoxidhydrat (CI 77289), Chromoxid (CI 77288) und/oder Eisenblau (Ferric Ferrocyanide, CI 77510) beschichtet sind.

Beispiele für besonders geeignete Farbpigmente sind im Handel beispielsweise unter den Handelsbezeichnungen Rona^{®}, Colorona^{®}, Xirona^{®}, Dichrona^{®} und Timiron^{®} von der Firma Merck, Ariabel^{®} und Unipure^{®} von der Firma Sensient, Prestige^{®} von der Firma Eckart Cosmetic Colors und Sunshine^{®} von der Firma Sunstar erhältlich.

Ganz besonders bevorzugte Farbpigmente mit der Handelsbezeichnung Colorona^{®} sind beispielsweise:
Colorona Copper, Merck, MICA, CI 77491 (IRON OXIDES)
Colorona Passion Orange, Merck, Mica, CI 77491 (Iron Oxides), Alumina
Colorona Patina Silver, Merck, MICA, CI 77499 (IRON OXIDES), CI 77891 (TITANIUM DIOXIDE)
Colorona RY, Merck, CI 77891 (TITANIUM DIOXIDE), MICA, CI 75470 (CARMINE)
Colorona Oriental Beige, Merck, MICA, CI 77891 (TITANIUM DIOXIDE), CI 77491 (IRON OXIDES)
Colorona Dark Blue, Merck, MICA, TITANIUM DIOXIDE, FERRIC FERROCYANIDE
Colorona Chameleon, Merck, CI 77491 (IRON OXIDES), MICA
Colorona Aborigine Amber, Merck, MICA, CI 77499 (IRON OXIDES), CI 77891 (TITANIUM DIOXIDE)
Colorona Blackstar Blue, Merck, CI 77499 (IRON OXIDES), MICA
Colorona Patagonian Purple, Merck, MICA, CI 77491 (IRON OXIDES), CI 77891 (TITANIUM DIOXIDE), CI 77510 (FERRIC FERROCYANIDE)
Colorona Red Brown, Merck, MICA, CI 77491 (IRON OXIDES), CI 77891 (TITANIUM DIOXIDE)
Colorona Russet, Merck, CI 77491 (TITANIUM DIOXIDE), MICA, CI 77891 (IRON OXIDES)
Colorona Imperial Red, Merck, MICA, TITANIUM DIOXIDE (CI 77891), D&C RED NO. 30 (CI 73360)
Colorona Majestic Green, Merck, CI 77891 (TITANIUM DIOXIDE), MICA, CI 77288 (CHROMIUM OXIDE GREENS)
Colorona Light Blue, Merck, MICA, TITANIUM DIOXIDE (CI 77891), FERRIC FERROCYANIDE (CI 77510)
Colorona Red Gold, Merck, MICA, CI 77891 (TITANIUM DIOXIDE), CI 77491 (IRON OXIDES)
Colorona Gold Plus MP 25, Merck, MICA, TITANIUM DIOXIDE (CI 77891), IRON OXIDES (CI 77491)
Colorona Carmine Red, Merck, MICA, TITANIUM DIOXIDE, CARMINE
Colorona Blackstar Green, Merck, MICA, CI 77499 (IRON OXIDES)
Colorona Bordeaux, Merck, MICA, CI 77491 (IRON OXIDES)
Colorona Bronze, Merck, MICA, CI 77491 (IRON OXIDES)
Colorona Bronze Fine, Merck, MICA, CI 77491 (IRON OXIDES)
Colorona Fine Gold MP 20, Merck, MICA, CI 77891 (TITANIUM DIOXIDE), CI 77491 (IRON OXIDES)
Colorona Sienna Fine, Merck, CI 77491 (IRON OXIDES), MICA
Colorona Sienna, Merck, MICA, CI 77491 (IRON OXIDES)
Colorona Precious Gold, Merck, Mica, CI 77891 (Titanium dioxide), Silica, CI 77491(Iron oxides), Tin oxide
Colorona Sun Gold Sparkle MP 29, Merck, MICA, TITANIUM DIOXIDE, IRON OXIDES, MICA, CI 77891, CI 77491 (EU)
Colorona Mica Black, Merck, CI 77499 (Iron oxides), Mica, C! 77891 (Titanium dioxide)
Colorona Bright Gold, Merck, Mica, CI 77891 (Titanium dioxide), CI 77491(Iron oxides)
Colorona Blackstar Gold, Merck, MICA, CI 77499 (IRON OXIDES)
Weiterhin besonders bevorzugte Farbpigmente mit der Handelsbezeichnung Xirona^{®} sind beispielsweise:
   Xirona Golden Sky, Merck, Silica, CI 77891 (Titanium Dioxide), Tin Oxide
   Xirona Caribbean Blue, Merck, Mica, CI 77891 (Titanium Dioxide), Silica, Tin Oxide
   Xirona Kiwi Rose, Merck, Silica, CI 77891 (Titanium Dioxide), Tin Oxide
   Xirona Magic Mauve, Merck, Silica, CI 77891 (Titanium Dioxide), Tin Oxide.
Zudem sind besonders bevorzugte Farbpigmente mit der Handelsbezeichnung Unipure^{®} beispielsweise:
   Unipure Red LC 381 EM, Sensient CI 77491 (Iron Oxides), Silica
   Unipure Black LC 989 EM, Sensient, CI 77499 (Iron Oxides), Silica
   Unipure Yellow LC 182 EM, Sensient, CI 77492 (Iron Oxides), Silica

In einer weiteren bevorzugten Ausführungsform ist die erfindungsgemäße Zusammensetzung (B) dadurch gekennzeichnet, dass sie mindestens eine farbgebende Verbindung aus der Gruppe der Pigmente enthält, die ausgewählt ist aus der Gruppe bestehend aus Metallen und Metalllegierungen, insbesondere Aluminium und Aluminium-Legierungen.

Ganz besonders bevorzugte Farbpigmente dieses Typs können mit der Handelsbezeichnung Alegrace^{®} von der Firma Schlenk kommerziell erworben werden:
Alegrace^{®} Gorgeous B 56/77-1 Vibrant Silver
Alegrace^{®} Spectacular A 35/00-1 , Elegant Iris
Alegrace^{®} Spectacular A 20/00-1 , Tender Iris
Alegrace^{®} Spectacular A 150/00-1, Fancy Iris
Alegrace^{®} Marvelous A 12/77-1 Bright Silver
Alegrace^{®} Marvelous A 12/77-2 Platin Silver
Alegrace^{®} Marvelous A 12/77-3 White Silver
Alegrace^{®} Marvelous D 12/77-1 Shiny Silver
Alegrace^{®} Lustrous A 100/77-1 Smooth Silver
Alegrace^{®} Lustrous A 150/771-1 Vivid Silver
Alegrace^{®} Lustrous A 450/77-1 Intense Silver
Alegrace^{®} Aurous A 21/11-1 Yellow Gold
Alegrace^{®} Aurous A 21/71-1 White Gold

Im Rahmen einer weiteren Ausführungsform kann das erfindungsgemäße Mittel bzw. die erfindungsgemäße Zubereitung auch ein oder mehrere farbgebende Verbindungen aus der Gruppe der organischen Pigmente enthalten

Bei den erfindungsgemäßen organischen Pigmenten handelt es sich um entsprechend unlösliche, organische Farbstoffe oder Farblacke, die beispielsweise aus der Gruppe der Nitroso-, Nitro- Azo-, Xanthen-, Anthrachinon-, Isoindolinon-, Isoindolin-, Chinacridon-, Perinon-, Perylen- , Diketopyrrolopyorrol-, Indigo-, Thioindido-, Dioxazin-, und/oder Triarylmethan-Verbindungen ausgewählt sein können.

Als besonders gut geeignete organische Pigmente können beispielsweise Carmin, Chinacridon, Phthalocyanin, Sorgho, blaue Pigmente mit den Color Index Nummern CI 42090, CI 69800, CI 69825, CI 73000, CI 74100, CI 74160, gelbe Pigmente mit den Color Index Nummern CI 11680, CI 11710, CI 15985, CI 19140, CI 20040, CI 21100, CI 21108, CI 47000, CI 47005, grüne Pigmente mit den Color Index Nummern CI 61565, CI 61570, CI 74260, orange Pigmente mit den Color Index Nummern CI 11725, CI 15510, CI 45370, CI 71105, rote Pigmente mit den Color Index Nummern CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915 und/oder CI 75470 genannt werden.

In einer weiteren besonders bevorzugten Ausführungsform ist ein erfindungsgemäßes Verfahren dadurch gekennzeichnet, dass die Zusammensetzung (B) mindestens eine farbgebende Verbindung aus der Gruppe der organischen Pigmente enthält, die ausgewählt ist aus der Gruppe aus Carmin, Chinacridon, Phthalocyanin, Sorgho, blaue Pigmente mit den Color Index Nummern CI 42090, CI 69800, CI 69825, CI 73000, CI 74100, CI 74160, gelbe Pigmente mit den Color Index Nummern CI 11680, CI 11710, CI 15985, CI 19140, CI 20040, CI 21100, CI 21108, CI 47000, CI 47005, grüne Pigmente mit den Color Index Nummern CI 61565, CI 61570, CI 74260, orange Pigmente mit den Color Index Nummern CI 11725, CI 15510, CI 45370, CI 71105, rote Pigmente mit den Color Index Nummern CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915 und/oder CI 75470.

Zur Erzeugung von besonders gleichmäßigen und natürlichen Farbnuancen hat sich der Einsatz von ganz bestimmten Pigmenten in der Zubereitung (B) als besonders gut geeignet erwiesen. Besonders intensive und gleichmäßige Färbungen konnten erhalten werden, wenn die im erfindungsgemäßen Verfahren eingesetzte Zusammensetzung (B) mindestens eine farbgebende Verbindung (B3) enthielt, die ausgewählt ist aus der Gruppe aus dem blauen Pigment mit den Color Index Nummer CI 74160, dem gelben Pigment mit der Color Index Nummer CI 11680, und dem roten Pigmenten mit der Color Index Nummer CI 12490.

In einer weiteren besonders bevorzugten Ausführungsform ist ein erfindungsgemäßes Verfahren dadurch gekennzeichnet, dass die Zusammensetzung (B) mindestens eine farbgebende Verbindung (B3) aus der Gruppe der organischen Pigmente enthält, die ausgewählt ist aus der Gruppe aus
- dem blauen Pigment mit den Color Index Nummer CI 74160,
- dem gelben Pigment mit der Color Index Nummer CI 11680, und
- dem roten Pigmenten mit der Color Index Nummer CI 12490.

Bei dem organischen Pigment kann es sich weiterhin auch um einen Farblack handeln. Unter der Bezeichnung Farblack wird im Sinn der Erfindung Partikel verstanden, welche eine Schicht aus absorbierten Farbstoffen umfassen, wobei die Einheit aus Partikel und Farbstoff unter den o.g. Bedingungen unlöslich ist. Bei den Partikeln kann es sich beispielsweise um anorganische Substrate handeln, die Aluminium, Silica, Calciumborosilkat, Calciumaluminiumborosilikat oder auch Aluminium sein können.

Als Farblack kann beispielsweise der Alizarin-Farblack eingesetzt werden.

Aufgrund ihrer ausgezeichneten Licht- und Temperaturbeständigkeit ist die Verwendung der zuvor genannten Pigmente in dem erfindungsgemäßen Mitteln besonders bevorzugt. Weiterhin ist es bevorzugt, wenn die eingesetzten Pigmente eine bestimmte Teilchengröße aufweisen. Diese Teilchengröße führt einerseits zu einer gleichmäßigen Verteilung der Pigmente in dem gebildeten Polymerfilm und vermeidet andererseits ein raues Haar- oder Hautgefühl nach dem Auftragen des kosmetischen Mittels. Es ist daher erfindungsgemäß vorteilhaft, wenn das mindestens eine Pigment eine mittlere Teilchengröße D₅₀ von 1,0 bis 50 µm, vorzugsweise von 5,0 bis 45 µm, bevorzugt von 10 bis 40 µm, insbesondere von 14 bis 30 µm, aufweist. Die mittlere Teilchengröße D₅₀ kann beispielsweise unter Verwendung von dynamischer Lichtstreuung (DLS) bestimmt werden.

Das oder die Pigmente können in einer Menge von 0,001 bis 20 Gew.-%, insbesondere von 0,05 bis 5 Gew.-%, jeweils bezogen auf das Gesamtgewicht des erfindungsgemäßen Mittels bzw. der Zubereitung, eingesetzt werden.

Als farbgebende Verbindungen können die erfindungsgemäßen Mittel auch einen oder mehrere direktziehende Farbstoffe enthalten. Bei direktziehende Farbstoffen handelt es sich um Farbstoffe, die direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone, Triarylmethanfarbstoffe oder Indophenole.

Die direktziehenden Farbstoffe im Sinne der vorliegenden Erfindung besitzen eine Löslichkeit in Wasser (760 mmHg) bei 25 °C von mehr als 0,5 g/L und sind daher nicht als Pigmente anzusehen. Bevorzugt besitzen die direktziehenden Farbstoffe im Sinne der vorliegenden Erfindung eine Löslichkeit in Wasser (760 mmHg) bei 25 °C von mehr als 1,0 g/L. Besonders bevorzugt besitzen die direktziehenden Farbstoffe im Sinne der vorliegenden Erfindung eine Löslichkeit in Wasser (760 mmHg) bei 25 °C von mehr als 1,5 g/L.

Direktziehende Farbstoffe können in anionische, kationische und nichtionische direktziehende Farbstoffe unterteilt werden.

In einer weiteren bevorzugten Ausführungsform ist ein erfindungsgemäßes Mittel dadurch gekennzeichnet, dass es als farbgebende Verbindung mindestens einen anionischen, kationischen und/oder nichtionischen direktziehenden Farbstoff enthält.

In einer weiteren bevorzugten Ausführungsform ist ein erfindungsgemäßes Verfahren dadurch gekennzeichnet, dass die Zusammensetzung (B) und/oder die Zusammensetzung (C) mindestens eine farbgebende Verbindung aus der Gruppe der anionischen, nichtionischen, und/oder kationischen direktziehenden Farbstoffe enthält.

Geeignete kationische direktziehende Farbstoffe sind beispielsweise Basic Blue 7, Basic Blue 26, Basic Violet 2 und Basic Violet 14, Basic Yellow 57, Basic Red 76, Basic Blue 16, Basic Blue 347 (Cationic Blue 347 / Dystar), HC Blue No. 16, Basic Blue 99, Basic Brown 16, Basic Brown 17, Basic Yellow 57, Basic Yellow 87, Basic Orange 31, Basic Red 51 Basic Red 76

Als nichtionische direktziehende Farbstoffe können beispielsweise nichtionische Nitro- und Chinonfarbstoffe und neutrale Azofarbstoffe eingesetzt werden. Geeignete nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)-aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)-amino-5-chlor-2-nitrobenzol, 4-Amino-3-nitrophenol, 1-(2'-Ureidoethyl)amino-4-nitrobenzol, 2-[(4-Amino-2-nitrophenyl)amino]-benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

Anionische direktziehende Farbstoffe werden auch als Säurefarbstoffe bezeichnet. Unter Säurefarbstoffen werden direktziehende Farbstoffe verstanden, die mindestens eine Carbonsäuregruppierung (-COOH) und/oder eine Sulfonsäuregruppierung (-SO₃H) besitzen. In Abhängigkeit vom pH-Wert liegen die protonierten Formen (-COOH, -SO₃H) der Carbonsäure- bzw. Sulfonsäuregruppierungen mit ihren deprotonierten Formen (-COO-, -SO₃⁻ vor) im Gleichgewicht vor. Mit abnehmendem pH-Wert steigt der Anteil der protonierten Formen. Werden direktziehende Farbstoffe in Form ihrer Salze eingesetzt, so liegen die Carbonsäuregruppen bzw. Sulfonsäuregruppen in deprotonierter Form vor und sind zur Einhaltung der Elektroneutralität mit entsprechenden stöchiometrischen Äquivalente an Kationen neutralisiert. Erfindungsgemäße Säurefarbstoffe können auch in Form ihrer Natriumsalze und/oder ihrer Kaliumsalze eingesetzt werden.

Die Säurefarbstoffe im Sinne der vorliegenden Erfindung besitzen eine Löslichkeit in Wasser (760 mmHg) bei 25 °C von mehr als 0,5 g/L und sind daher nicht als Pigmente anzusehen. Bevorzugt besitzen die Säurefarbstoffe im Sinne der vorliegenden Erfindung besitzen eine Löslichkeit in Wasser (760 mmHg) bei 25 °C von mehr als 1,0 g/L.

Die Erdalkalisalze (wie beispielsweise Calciumsalze und Magnesiumsalze) bzw. Aluminiumsalze von Säurefarbstoffen besitzen oftmals eine schlechtere Löslichkeit als die entsprechenden Alkalisalze. Sofern die Löslichkeit dieser Salze unterhalb von 0,5 g/L (25 °C, 760 mmHg) liegt, fallen diese nicht unter die Definition eines direktziehenden Farbstoffes.

Ein wesentliches Merkmal der Säurefarbstoffe ist ihr Vermögen, anionische Ladungen auszubilden, wobei die hierfür verantwortlichen Carbonsäure- bzw. Sulfonsäuregruppen üblicherweise an verschiedene chromophore Systeme geknüpft sind. Geeignete chromophore Systeme finden sich beispielsweise in den Strukturen von Nitrophenylendiaminen, Nitroaminophenolen, Azofarbstoffen, Anthrachinonfarbstoffen, Triarylmethanfarbstoffen, Xanthen-Farbstoffen, Rhodamin-Farbstoffen, Oxazinfarbstoffen und/oder Indophenolfarbstoffen.

Als besonders gut geeignete Säurefarbstoffe können beispielsweise eine oder mehrere Verbindungen aus der folgenden Gruppe ausgewählt werden: Acid Yellow 1 (D&C Yellow 7, Citronin A, Ext. D&C Yellow No. 7, Japan Yellow 403,CI 10316, COLIPA n° B001), Acid Yellow 3 (COLIPA n° : C 54, D&C Yellow N° 10, Quinoline Yellow, E104, Food Yellow 13), Acid Yellow 9 (CI 13015), Acid Yellow 17 (CI 18965), Acid Yellow 23 (COLIPA n° C 29, Covacap Jaune W1100 (LCW), Sicovit Tartrazine 85 E 102 (BASF), Tartrazine, Food Yellow 4, Japan Yellow 4, FD&C Yellow No. 5), Acid Yellow 36 (CI 13065), Acid Yellow 121 (CI 18690), Acid Orange 6 (CI 14270), Acid Orange 7 (2-Naphthol orange, Orange II, CI 15510, D&C Orange 4, COLIPA n° C015), Acid Orange 10 (C.I. 16230; Orange G sodium salt), Acid Orange 11 (CI 45370), Acid Orange 15 (CI 50120), Acid Orange 20 (CI 14600), Acid Orange 24 (BROWN 1;CI 20170;KATSU201 ;nosodiumsalt;Brown No.201;RESORCIN BROWN;ACID ORANGE 24;Japan Brown 201;D & C Brown No.1), Acid Red 14 (C.I.14720), Acid Red 18 (E124, Red 18; CI 16255), Acid Red 27 (E 123, CI 16185, C-Rot46, Echtrot D, FD&C Red Nr.2, Food Red 9, Naphtholrot S), Acid Red 33 (Red 33, Fuchsia Red, D&C Red 33, CI 17200), Acid Red 35 (CI C.!.18065), Acid Red 51 (CI 45430, Pyrosin B, Tetraiodfluorescein, Eosin J, lodeosin), Acid Red 52 (CI 45100, Food Red 106, Solar Rhodamine B, Acid Rhodamine B, Red n° 106 Pontacyl Brilliant Pink), Acid Red 73 (CI 27290), Acid Red 87 (Eosin, CI 45380), Acid Red 92 (COLIPA n° C53, CI 45410), Acid Red 95 (CI 45425, Erythtosine,Simacid Erythrosine Y), Acid Red 184 (CI 15685), Acid Red 195, Acid Violet 43 (Jarocol Violet 43, Ext. D&C Violet n° 2, C.I. 60730, COLIPA n° C063), Acid Violet 49 (CI 42640), Acid Violet 50 (CI 50325), Acid Blue 1 (Patent Blue, CI 42045), Acid Blue 3 (Patent Blau V, CI 42051), Acid Blue 7 (CI 42080), Acid Blue 104 (CI 42735), Acid Blue 9 (E 133, Patentblau AE, Amidoblau AE, Erioglaucin A, CI 42090, C.I. Food Blue 2), Acid Blue 62 (CI 62045), Acid Blue 74 (E 132, CI 73015), Acid Blue 80 (CI 61585), Acid Green 3 (CI 42085, Foodgreen1), Acid Green 5 (CI 42095), Acid Green 9 (C.I.42100), Acid Green 22 (C.I.42170), Acid Green 25 (CI 61570, Japan Green 201, D&C Green No. 5), Acid Green 50 (Brillantsäuregrün BS, C.I. 44090, Acid Brilliant Green BS, E 142), Acid Black 1 (Black n° 401, Naphthalene Black 10B, Amido Black 10B, CI 20 470, COLIPA n° B15), Acid Black 52 (CI 15711), Food Yellow 8 (CI 14270), Food Blue 5, D&C Yellow 8, D&C Green 5, D&C Orange 10, D&C Orange 11, D&C Red 21, D&C Red 27, D&C Red 33, D&C Violet 2 und/oder D&C Brown 1.

Die Wasserlöslichkeit der anionischen direktziehenden Farbstoffe kann beispielsweise auf dem folgenden Weg bestimmt werden. 0,1 g des anionischen direktziehenden Farbstoffes werden in ein Becherglas gegeben. Es wird ein Rührfisch hinzugegeben. Dann werden 100 ml Wasser hinzugefügt. Diese Mischung wird auf einem Magnetrührer unter Rühren auf 25 °C erwärmt. Es wird für 60 Minuten gerührt. Danach wird die wässrige Mischung visuell beurteilt. Sind noch ungelöste Reste vorhanden, wird die Wassermenge - beispielsweise in Schritten von 10 ml - erhöht. Es wird solange Wasser zugegeben, bis sich die eingesetzte Farbstoffmenge komplett gelöst hat. Sofern sich die Farbstoff-Wasser-Mischung aufgrund der hohen Intenstität des Farbstoffes nicht visuell beurteilten lässt, wird die Mischung filtriert. Bleibt auf dem Filterpapier ein Anteil an ungelösten Farbstoffen zurück, wird der Löslichkeitsversuch mit einer höheren Wassermenge wiederholt. Lösen sich 0,1 g des anionischen direktziehenden Farbstoffes bei 25 °C in 100 ml Wasser, so liegt die Löslichkeit des Farbstoffes bei 1,0 g/L.

Acid Yellow 1 trägt den Namen 8-Hydroxy-5,7-dinitro-2-naphthalenesulfonsäure Dinatriumsalz und besitzt eine Löslichkeit in Wasser von mindestens 40 g/L (25°C).

Acid Yellow 3 ist ein Gemisch der Natriuimsalze von Mono- und Sisulfonsäuren von 2-(2-Chinolyl)-1H-indene-1 ,3(2H)-dion und besitzt eine Wasserlöslichkeit von 20 g/L (25 °C).

Acid Yellow 9 ist das Dinatriumsalz der 8-Hydroxy-5,7-dinitro-2-naphthalenesulfonsäure, seine Wasserlöslichkeit liegt oberhalb von 40 g/L (25 °C).

Acid Yellow 23 ist das Trinatriumsalz der4,5-Dihydro-5-oxo-1-(4-sulfophenyl)-4-((4-sulfophenyl)azo)-1H-pyrazole-3-carbonsäure und bei 25 °C gut in Wasser löslich.

Acid Orange 7 ist das Natriumsalz des 4-[(2-Hydroxy-1-naphthyl)azo]benzensulfonats. Seine Wasserlöslichkeit beträgt mehr als 7 g/L (25 °C).

Acid Red 18 ist das Trinatirumsalz von 7-Hydroxy-8-[(E)-(4-sulfonato-1-naphthyl)-diazenyl)]-1,3-naphthalenedisulfonat und besitzt eine sehr hohe Wasserlöslichkeit von mehr als 20 GEw.-%.

Acid Red 33 ist das Diantriumsalz des 5-Amino-4-hydroxy-3-(phenylazo)-naphthalene-2,7-disulphonats, seine Wasserlöslichkeit liegt bei 2,5 g/L (25 °C).

Bei Acid Red 92 handelt es sich um das Dinatriumsalz der 3,4,5,6-Tetrachloro-2-(1 ,4,5,8-tetrabromo-6-hydroxy-3-oxoxanthen-9-yl)benzoesäure, dessen Wasserlöslichkeit mit größer 10 g/L angegeben wird (25 °C).

Acid Blue 9 ist das Dinatriumsalz von 2-({4-[N-ethyl(3-sulfonatobenzyl]amino]phenyl}{4-[(N-ethyl(3-sulfonatobenzyl)imino]-2,5-cyclohexadien-1-ylidene}methyl)-benzenesulfonat und besitzt eine Wasserlöslichkeit von mehr als 20 Gew.-% (25 °C).

Weiterhin können auch thermochrome Farbstoffe eingesetzt werden. Thermochromie beinhaltet die Eigenschaft eines Materials, in Abhängigkeit der Temperatur reversibel oder irreversibel seine Farbe zu ändern. Dies kann sowohl durch Änderung der Intensität und/oder des Wellenlängenmaximums erfolgen.

Schließlich ist es auch möglich, photochrome Farbstoffe einzusetzen. Photochromie beinhaltet die Eigenschaft eines Materials, in Abhängigkeit der Bestrahlung mit Licht, insbesondere UV-Licht, reversibel oder irreversibel seine Farbe zu ändern. Dies kann sowohl durch Änderung der Intensität und/oder des Wellenlängenmaximums erfolgen.

Die Einsatzmengen der farbgebenden Verbindungen (B3) in der Zusammensetzung (B) kann der Fachmann in Abhängigkeit von der gewünschten Farbintensität und Farbnuance wählen, besonders gute Ergebnisse konnten erhalten werden, wenn die zweite Zusammensetzung (B) - bezogen auf das Gesamtgewicht der Zusammensetzung (B) - ein oder mehrere farbgebende Verbindungen (B3) in einer Gesamtmenge von 0,1 bis 6,0 Gew.-%, bevorzugt von 0,3 bis 5,0 Gew.-%, weiter bevorzugt von 0,5 bis 4,0 Gew.-% und ganz besonders bevorzugt von 0,7 bis 1,5 Gew.-% enthielt.

In einer weiteren besonders bevorzugten Ausführungsform ist ein erfindungsgemäßes Verfahren dadurch gekennzeichnet, dass die Zusammensetzung (B) - bezogen auf das Gesamtgewicht der Zusammensetzung (B) - ein oder mehrere farbgebende Verbindungen (B3) in einer Gesamtmenge von 0,1 bis 6,0 Gew.-%, bevorzugt von 0,3 bis 5,0 Gew.-%, weiter bevorzugt von 0,5 bis 4,0 Gew.-% und ganz besonders bevorzugt von 0,7 bis 1,5 Gew.-% enthält.

In einer weiteren besonders bevorzugten Ausführungsform ist ein erfindungsgemäßes Verfahren dadurch gekennzeichnet, dass die Zusammensetzung (B) - bezogen auf das Gesamtgewicht der Zusammensetzung (B) - ein oder mehrere Pigmente (B3) in einer Gesamtmenge von 0,1 bis 6,0 Gew.-%, bevorzugt von 0,3 bis 5,0 Gew.-%, weiter bevorzugt von 0,5 bis 4,0 Gew.-% und ganz besonders bevorzugt von 0,7 bis 1,5 Gew.-% enthält.

### Weitere kosmetische Inhaltsstoffe in der Zusammensetzung (B)

Zusätzlich zu den bereits zuvor beschriebenen erfindungswesentlichen und optionalen Inhaltsstoffen kann die Zusammensetzung (B) darüber hinaus auch noch einen oder mehrere weitere kosmetische Inhaltsstoffe enthalten.

Bei den fakultativ in der Zusammensetzung (B) einsatzbaren kosmetischen Inhaltsstoffen kann es sich um alle geeigneten Bestandteile handeln, um dem Mittel weitere positive Eigenschaften zu verleihen. Beispielsweise können in der Zusammensetzung (A) ein Lösungsmittel, ein verdickendes oder filmbildendes Polymer, eine oberflächenaktive Verbindung aus der Gruppe der nichtionischen, kationischen, anionischen oder zwitterionischen/amphoteren Tenside, der farbgebenden Verbindungen aus der Gruppe der Pigmente, der direktziehenden Farbstoffe, der Oxidationsfarbstoffvorprodukte, der Fettkomponenten aus der Gruppe der C₈-C₃₀-Fettalkohole, der Kohlenwasserstoffverbindungen, Fettsäureester, der zur Gruppe der pH-Regulatoren zugehörigen Säuren und Basen, der der Parfüme, der Konservierungsmittel, der Pflanzenextrakte und der Proteinhydrolysate enthalten sein.

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher verwiesen.

### Herstellung der Zusammensetzung (B)

Bei der in Schritt (2) des erfindungsgemäßen Verfahrens hergestellten Zusammensetzung (B) handelt es sich um eine Emulsion, die neben Wasser und dem bzw. den Fettbestandteilen auch die färbende(n) Verbindung(en) enthält. Die Herstellung kann zum Beispiel nach den üblichen, dem Fachmann bekannten Verfahren erfolgen.

Eine Möglichkeit zur Herstellung der Emulsion (B) ist es, zunächst die Fettbestandteile (B2) aufzuschmelzen, dann unter Homogenisierung, beispielsweise durch Rühren, mit Wasser (B1) zu versetzten und im Anschluss daran die farbgebenden Verbindungen (B3) hinzuzufügen.

Die Herstellung kann zum Beispiel in einem hierfür geeigneten Reaktionsgefäß oder auch Reaktor durchgeführt werden. Abhängig von der gewünschten Ansatzgröße sind hierfür verschiedene Modelle aus dem Stand der Technik bekannt und kommerziell erwerbbar. Besonders bevorzugt wird die Zusammensetzung (B) in einem Kessel mit Rührwerk bzw. Homogenisator hergestellt.

### pH-Werte der Zusammensetzungen im Verfahren

In weiteren Versuchen hat sich herausgestellt, dass die pH-Werte der Zusammensetzungen (A) und/oder (B) einen Einfluss auf die bei der Anwendung ablaufenden zuvor beschriebenen Hydrolyse- bzw. Kondensationsreaktionen nehmen können. Hierbei wurde gefunden, dass insbesondere alkalische pH-Werte die Kondensation auf der Stufe der Oligomere anhalten. Je saurer das Reaktionsgemisch ist, desto stärker scheint die Kondensation abzulaufen und desto höher ist das Molekulargewicht der bei der Kondensation entstehenden Silan-Kondensate. Aus diesem Grund ist es bevorzugt, dass die Zusammensetzungen (A) und/oder (B) einen pH-Wert von 7,0 bis 12,0, bevorzugt von 7,5 bis 11,5, weiter bevorzugt von 8,5 bis 11,0 und ganz besonders bevorzugt von 9,0 bis 11,0 besitzen.

Der Wassergehalt der Zusammensetzung (A) liegt bei maximal 10,0 Gew.-% und wird bevorzugt noch geringer eingestellt. Im Rahmen einiger Ausführungsformen kann auch der Wassergehalt der Zusammensetzung (B) gering gewählt sein. Insbesondere bei Zusammensetzungen mit sehr geringem Wassergehalt kann sich die Messung des pH-Wertes mit den üblichen aus dem Stand der Technik bekannten Methoden (pH-Wert Messung mittels Glaselektroden über Einstabmessketten oder aber über pH-Indikator-Papier) als schwierig erweisen. Aus diesem Grund handelt es sich bei den erfindungsgemäßen pH-Werten um die Werte, die nach Vermischen oder Verdünnung der Zubereitung im Gewichtsverhältnis 1:1 mit destilliertem Wasser erhalten wurden.

Der entsprechende pH-Wert wird dementsprechend gemessen, nachdem beispielsweise 50 g der erfindungsgemäßen Zusammensetzung mit 50 g destilliertem Wasser vermischt wurden.

In einer weiteren ganz besonders bevorzugten Ausführungsform ist ein erfindungsgemäßes Verfahren, dadurch gekennzeichnet, dass die Zusammensetzung (A) und/oder (B) nach der Verdünnung mit destilliertem Wasser im Gewichtsverhältnis 1:1 einen pH-Wert von 7,0 bis 11,5, weiter bevorzugt von 8,5 bis 11,0 und ganz besonders bevorzugt von 9,0 bis 11,0 besitzt.

Zur Einstellung dieses alkalischen pH-Wertes kann es erforderlich werden, der Reaktionsmischung ein Alkalisierungsmittel und/oder Acidifizierungsmittel zuzusetzen. Bei den pH-Werten im Sinne der vorliegenden Erfindung handelt es sich um pH-Werte, die bei einer Temperatur von 22°C gemessen wurden.

Als Alkalisierungsmittel können beispielsweise Ammoniak, Alkanolamine und/oder basische Aminosäuren eingesetzt werden.

Alkanolamine können ausgewählt werden aus primären Aminen mit einem C₂-C₆-Alkylgrundkörper, der mindestens eine Hydroxylgruppe trägt. Bevorzugte Alkanolamine werden aus der Gruppe ausgewählt, die gebildet wird aus 2-Aminoethan-1-ol (Monoethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methyl-propan-2-ol, 3-Aminopropan-1,2-diol, 2-Amino-2-methylpropan-1,3-diol.

Als Aminosäure im Sinne der Erfindung gilt eine organische Verbindung, die in ihrer Struktur mindestens eine protonierbare Aminogruppe und mindestens eine -COOH- oder eine -SOsH-Gruppe enthält. Bevorzugte Aminosäuren sind Aminocarbonsäuren, insbesondere α-(alpha)-Aminocarbonsäuren und ω-Aminocarbonsäuren, wobei α-Aminocarbonsäuren besonders bevorzugt sind.

Unter basischen Aminosäuren sind erfindungsgemäß solche Aminosäuren zu verstehen, welche einen isoelektrischen Punkt pl von größer 7,0 besitzen.

Basische α-Aminocarbonsäuren enthalten mindestens ein asymmetrisches Kohlenstoffatom. Im Rahmen der vorliegenden Erfindung können beide möglichen Enantiomere als spezifische Verbindung oder auch deren Gemische, insbesondere als Racemate, gleichermaßen eingesetzt werden. Es ist jedoch besonders vorteilhaft, die natürlich bevorzugt vorkommende Isomerenform, üblicherweise in L-Konfiguration, einzusetzen.

Die basischen Aminosäuren werden bevorzugt ausgewählt aus der Gruppe, die gebildet wird aus Arginin, Lysin, Ornithin und Histidin, besonders bevorzugt aus Arginin und Lysin. In einer weiteren besonders bevorzugten Ausführungsform ist ein erfindungsgemäßes Mittel daher dadurch gekennzeichnet, dass es sich bei dem Alkalisierungsmittel um eine basische Aminosäure aus der Gruppe Arginin, Lysin, Ornithin und/oder Histidin handelt.

Darüber hinaus können auch anorganische Alkalisierungsmittel eingesetzt werden. Erfindungsgemäß einsetzbare, anorganische Alkalisierungsmittel sind bevorzugt ausgewählt aus der Gruppe, die gebildet wird aus Natriumhydroxid, Kaliumhydroxid, Calciumhydroxid, Bariumhydroxid, Natriumphosphat, Kaliumphosphat, Natriumsilicat, Natriummetasilicat, Kaliumsilicat, Natriumcarbonat und Kaliumcarbonat.

Ganz besonders bevorzugte Alkalisierungsmittel sind Ammoniak, 2-Aminoethan-1-ol (Monoethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methylpropan-2-ol, 3-Aminopropan-1,2-diol, 2-Amino-2-methylpropan-1,3-diol, Arginin, Lysin, Ornithin, Histidin, Natriumhydroxid, Kaliumhydroxid, Calciumhydroxid, Bariumhydroxid, Natriumphosphat, Kaliumphosphat, Natriumsilicat, Natriummetasilicat, Kaliumsilicat, Natriumcarbonat und Kaliumcarbonat.

Neben den zuvor beschriebenen Alkalisierungsmitteln sind dem Fachmann zur Feineinstellung des pH-Wertes gängige Acidifizierungsmittel geläufig. Erfindungsgemäß bevorzugte Acidifizierungsmittel sind Genuss-Säuren, wie beispielsweise Zitronensäure, Essigsäure, Äpfelsäure oder Weinsäure, sowie verdünnte Mineralsäuren.

Im Anschluss an die Herstellung wird die Zusammensetzung (B) bevorzugt in eine Verpackungseinheit abgefüllt.

Bei der Verpackungseinheit kann es sich entweder um eine Endverpackung handeln, aus welcher der Anwender das Mittel zur Behandlung der Keratinmaterialien entnimmt. Geeignete Endverpackungen sind beispielsweise eine Flasche, eine Tube, einen Tiegel, eine Dose, ein Sachet, einen Aerosol-Druckbehälter, einen Non-Aerosol-Druckbehälter. Hierbei können diese Endverpackungen die Keratinbehandlungsmittel in Mengen enthalten, die für eine, gegebenenfalls auch führ mehrere Anwendungen ausreichen. Bevorzugt ist die Abfüllung in einer Menge, die für eine einmalige Anwendung ausreichend ist.

Weiterhin kann die Zusammensetzung (B) jedoch auch in eine Zwischenverpackung abgefüllt werden, bei der es sich beispielsweise um einen Kanister oder einen Hobbock handeln kann. Die Abfüllung in eine Zwischenverpackung ist insbesondere dann geeignet, wenn das Reaktionsgefäß bzw. der Reaktor, in dem das erfindungsgemäße Verfahren durchgeführt wurde, und die Abfüllanlage, in der eine Abfüllung in die Endverpackung erfolgt, räumlich getrennt sind.

In einer weiteren ganz besonders bevorzugten Ausführungsform ist ein erfindungsgemäßes Verfahren gekennzeichnet durch die
(2) Herstellung einer zweiten Zusammensetzung (B), enthaltend
(B1) Wasser und
(B2) ein oder mehrere Fettbestandteile und
(B3) ein oder mehrere farbgebende Verbindungen aus der Gruppe der Pigmente und/oder der direktziehenden Farbstoffe,
und Abfüllung der Zusammensetzung (A) in eine Flasche, eine Tube, einen Tiegel, eine Dose, ein Sachet, einen Aerosol-Druckbehälter, einen Non-Aerosol-Druckbehälter, einen Kanister oder einen Hobbock.

Bei den vorgenannten Verpackungseinheiten kann es sich um übliche, standardmäßig in der Kosmetik eingesetzte und kommerziell erhältliche Behältnisse handeln.

### Lagerung der Zusammensetzung (A) und/oder (B)

Der Schritt (3) des erfindungsgemäßen Verfahrens ist gekennzeichnet durch die Lagerung der Zusammensetzung (A) und/oder (B) für einen Zeitraum von mindestens 24 Stunden.

Dieser Schritt hat sich für die Erzielung von besonders Farbergebnissen mit besonders guten Waschechtheiten als erfindungswesentlich herausgestellt.

In Schritt (3) werden die Zusammensetzungen (A) und (B), die bevorzugt nach ihrer Herstellung jeweils in eine geeignete Verpackungseinheit abgefüllt wurden, in dieser Verpackungseinheit für einen Zeitraum von mindestens 24 Stunden gelagert.

Die Verpackungseinheit liegt während der Lagerung in verschlossenem Zustand vor. Dies kann beispielsweise dadurch erfolgen, dass die verschlossenen Verpackungseinheiten jeweils für mindestens 24 Stunden in einem Lagerraum oder einer Lagerhalle abgestellt werden.

Unter der Lagerung der Zusammensetzung (A) bzw. (B) in der Verpackungseinheit wird im Sinne der vorliegenden Erfindung verstanden, die verschlossene Verpackungseinheit für einen Zeitraum von mindestens 24 Stunden nicht zu öffnen. Da die Zubereitung sich während der Lagerung in einer verschlossenen Verpackungseinheit befindet, kommt sie weder mit der außerhalb der Verpackungseinheit befindlichen Luftfeuchtigkeit noch mit Sauerstoff in Kontakt.

Bei der verschlossenen Verpackungseinheit kann es sich beispielsweise um eine Flasche, eine Tube, einen Tiegel, eine Dose, ein Sachet, einen Aerosol-Druckbehälter, einen Non-Aerosol-Druckbehälter, einen Kanister oder einen Hobbock handeln, die jeweils mit einem geeigneten Deckel verschlossen sind.

Als Verpackungseinheiten können die üblicherweise im Bereich der Kosmetik verwendeten Verpackungen aus den üblichen Materialien eingesetzt werden. Diese Verpackungseinheiten sind dem Fachmann bekannt und kommerziell erhältlich.

Das Fassungsvermögen der Verpackungseinheit wird der Fachmann von den benötigten Anwendungsmengen abhängig machen. Als Flasche kann beispielsweise eine mit einem dichten Deckel, bevorzugt einem Schraubdeckel mit Dichtung, verschlossene Flasche mit einem Volumen von 20 ml, 50 ml, 100 ml, 250 ml, 500 ml, oder auch 1000 ml eingesetzt werden.

Als Tube kann beispielsweise eine Tube mit Schraubverschluss oder auch mit Klappschanier-Verschluss mit einem Fassungsvermögen von 20 ml, 50 ml, 100 ml, 250 ml, 500 ml, oder auch 1000 ml eingesetzt werden. Besonders bevorzugt ist es, die Tube zu versiegeln und die Versiegelung erst kurz vor der Anwendung durch Einsatz des Deckels zu öffnen.

Auch Dosen können mit einem Schraubdeckel mit Dichtung versehen sein und zum Beispiel ein Fassungsvermögen von 20 ml, 50 ml, 100 ml, 250 ml, 500 ml, oder auch 1000 ml besitzen.

Als preiswerte Verpackungsform mit geringem Materialverbrauch bietet sich in diesem Zusammenhang auch das Sachet an. Ein Sachet ist eine kleine Verpackung in Taschen- oder Beutelform, die oft bei der Verpackung von Kosmetika eingesetzt wird. Ein typisches Sachet kann beispielsweise durch Verklebung oder Heißverpressung von zwei übereinander liegenden Folien hergestellt werden, wobei die Verklebung an allen Rändern der Folien erfolgt. Der durch das Verkleben hergestellte Innenraum des Sachets (d.h. des Beutels) kann dann mit der gewünschten kosmetischen Zubereitung befüllt werden. Die Öffnung des Sachets kann durch Aufreißen oder Aufschneiden des Beutels erfolgen.

Soll die Lagerung in einem Zwischengefäß erfolgen, aus dem die Zubereitung nochmals in einem weiteren Schritt in die endgültig vom Anwender benutzte Endverpackung umgefüllt wird, so bieten sich als Verpackungseinheiten Kanister oder auch Hobbocks an. Diese besitzen üblicherweise ein größeres Fassungsvermögen von 1 Liter, 5 Litern, 10 Litern, 20 Litern oder auch 50 Litern.

Ohne auf diese Theorie festgelegt zu sein, wird in diesem Zusammenhang für die Zubereitung (A) vermutet, dass die Hydrolyse-Reaktionen, die durch das Mischen der C₁-C₆-Alkoxy-Silane (A2) mit Wasser (A1) initiiert werden, mit Abschluss der Hertellung der Zubereitung (A) noch nicht abgeschlossen sind, sondern auch in der Verpackungseinheit noch über einen Zeitraum von mehreren Stunden, bevorzugt mehreren Tagen, ablaufen.

Vermutlich führen die in der Zusammensetzung (A) stattfindenden Kondensationsreaktionen zur Ausbildung von oligomeren Molekülverbänden, die eine bestimmte Mindestgröße besitzen müssen, um auf dem Keratinmaterial mit ausreichender Schnelligkeit einen widerstandsfähigen Film ausbilden zu können. Im Zuge der zu dieser Erfindung führenden Arbeiten hat sich herausgestellt, dass bei Anwendung der Zubereitungen in einem Färbeverfahren besonders dann gute und intensive Färbungen erhalten werden konnten, wenn die Zubereitung (A) für einen Zeitraum von mindestens 5 Tagen gelagert wurde.

In einer weiteren ganz besonders bevorzugten Ausführungsform ist ein erfindungsgemäßes Verfahren gekennzeichnet durch die
(3) Lagerung der Zusammensetzung (A) für einen Zeitraum von mindestens 5 Tagen, bevorzugt von mindestens 10 Tagen, weiter bevorzugt von mindestens 14 Tagen und ganz besonders bevorzugt von mindestens 21 Tagen.

In einer weiteren ganz besonders bevorzugten Ausführungsform ist ein erfindungsgemäßes Verfahren gekennzeichnet durch die
(3) Lagerung der Zusammensetzung (A) in einer Verpackungseinheit für einen Zeitraum von mindestens 5 Tagen, bevorzugt von mindestens 10 Tagen, weiter bevorzugt von mindestens 14 Tagen und ganz besonders bevorzugt von mindestens 21 Tagen.

Die Lagerung der Zusammensetzung (A) erfolgt besonders bevorzugt innerhalb bestimmter Temperaturbereiche. Hierbei hat es sich insbesondere als besonders vorteilhaft herausgestellt, während des Lagerzeitraums, der direkt nach der Herstellung in Schritt (1) erfolgt, bestimmte Temperaturbereiche einzuhalten. Es konnten besonders dann sehr gute Färbeergebnisse erhalten werden, wenn die Zubereitung (A) in der Verpackungseinheit bei einer Temperatur von 15 °C bis 40 °C, bevorzugt von 15 °C bis 35 °C und besonders bevorzugt von 15 °C bis 25 °C gelagert wurde.

In einer weiteren ganz besonders bevorzugten Ausführungsform ist ein erfindungsgemäßes Verfahren gekennzeichnet durch die
(3) Lagerung der Zusammensetzung (A) für einen Zeitraum von mindestens 5 Tagen, bevorzugt von mindestens 10 Tagen, weiter bevorzugt von mindestens 14 Tagen und ganz besonders bevorzugt von mindestens 21 Tagen bei einer Temperatur im Bereich von 15 °C bis 40 °C, bevorzugt von 15 °C bis 35 °C und besonders bevorzugt von 15 °C bis 25 °C.

Unter den gegebenen Lagerbedingungen, insbesondere innerhalb der vorgenannten Temperaturbereiche, scheint die Kondensationsreaktion der Silane nach einiger Zeit zum Erliegen zu kommen, so dass eine längere Lagerung keinen negativen Einfluss auf ein späteres Färbeergebnis zeigt. So können die Zubereitungen (A) beipsielsweise in einer verschlossenen Verpackungseinheit für einen Zeitraum von bis zu 365 Tagen bei einer Temperatur von 15 bis 40 °C gelagert werden. Da die Verpackungseinheit bei der Lagerung verschlossen ist und auf diesem Wege der Kontakt zur gegebenenfalls feuchten Außenluft unterbunden wird, sind auch längere Lagerzeiträume als 365 Tage möglich.

In einer weiteren ganz besonders bevorzugten Ausführungsform ist ein erfindungsgemäßes Verfahren gekennzeichnet durch die
(3) Lagerung der Zubereitung (A), bevorzugt in einer verschlossenen Verpackungseinheit, für einen Zeitraum von 5 bis 365 Tagen, bevorzugt von 10 bis 365 Tagen, weiter bevorzugt für einen Zeitraum von 14 bis 365 Tagen und besonders bevorzugt für einen Zeitraum von 21 bis 365 Tagen.

In weiteren Versuchen hat sich gezeigt, dass bei Anwendung des erfindungsgemäßen Verfahrens insbesondere auch dann besonders waschechte Färbungen erhalten werden konnten, wenn die Zusammensetzung (B) für einen Zeitraum von mindestens 24 Stunden gelagert wurde.

Ohne auf diese Theorie festgelegt zu sein, könnte eine mögliche Ursache für die Verbesserung der Waschechtheit darin zu suchen sein, dass die in der Zusammensetzung (B) enthaltenen farbgebenden Verbindungen (B3), insbesondere die Pigmente, sich bei einer Lagerung verstärkt in die hydrophoben Bereiche der Emulsion einlagern, so dass eine Lagerung eine verstärkte Interaktion der Fettbestandteile (B2) und der farbgebenden Verbindungen (B3) ermöglicht, die sich in resistenteren Färbungen äußert. Es konnte gefunden werden, dass diese Interaktion einen Zeitraum von mindestens 24 Stunden benötigt. Bevorzugt ist eine noch längere Lagerung für einen Zeitraum von mindestens 48 Stunden (2 Tagen), besonders bevorzugt von mindestens 5 Tagen und ganz besonders bevorzugt von mindestens 10 Tagen.

In einer weiteren ganz besonders bevorzugten Ausführungsform ist ein erfindungsgemäßes Verfahren gekennzeichnet durch die
(3) Lagerung der Zusammensetzung (B) für einen Zeitraum von mindestens 24 Stunden, bevorzugt von mindestens 2 Tagen, noch weiter bevorzugt von mindestens 5 Tagen und ganz besonders bevorzugt von mindestens 10 Tagen.

In einer weiteren ganz besonders bevorzugten Ausführungsform ist ein erfindungsgemäßes Verfahren gekennzeichnet durch die
(3) Lagerung der Zusammensetzung (B) in einer Verpackungseinheit für einen Zeitraum von mindestens 24 Stunden, bevorzugt von mindestens 2 Tagen, noch weiter bevorzugt von mindestens 5 Tagen und ganz besonders bevorzugt von mindestens 10 Tagen.

Auch die Lagerung der Zusammensetzung (B) erfolgt besonders bevorzugt innerhalb bestimmter Temperaturbereiche. Hierbei hat es sich insbesondere als besonders vorteilhaft herausgestellt, während des Lagerzeitraums, der direkt nach der Herstellung in Schritt (2) erfolgt, bestimmte Temperaturbereiche einzuhalten. Es konnten besonders dann sehr gute Färbeergebnisse erhalten werden, wenn die Zubereitung (A) in der Verpackungseinheit bei einer Temperatur von 15 °C bis 40 °C, bevorzugt von 15 °C bis 35 °C und besonders bevorzugt von 15 °C bis 25 °C gelagert wurde.

In einer weiteren ganz besonders bevorzugten Ausführungsform ist ein erfindungsgemäßes Verfahren gekennzeichnet durch die
(3) Lagerung der Zusammensetzung (B) für einen Zeitraum von mindestens 24 Stunden, bevorzugt von mindestens 2 Tagen, noch weiter bevorzugt von mindestens 5 Tagen und ganz besonders bevorzugt von mindestens 10 Tagen bei einer Temperatur im Bereich von 15 °C bis 40 °C, bevorzugt von 15 °C bis 35 °C und besonders bevorzugt von 15 °C bis 25 °C.

Auch die Zusammensetzungen (B) haben sich als stabile Emulsions-Systeme herausgestellt, so das eine Lagerung über den vorgenannten Mindest-Zeitraum hinaus keinen negativen Einfluss auf die Zusammensetzungen (B) ausübt, insbesondere, wenn die Zusammensetzungen (B) in einer verschlossenen Verpackungseinheit gelagert werden. Aus diesem Grund sind auch für die Zusammensetzungen (B) längere Lagerzeiträume als 365 Tage möglich.

In einer weiteren ganz besonders bevorzugten Ausführungsform ist ein erfindungsgemäßes Verfahren gekennzeichnet durch die
(3) Lagerung der Zusammensetzung (B) für einen Zeitraum von 24 Stunden bis 365 Tagen, bevorzugt von mindestens 2 bis 365 Tagen, noch weiter bevorzugt von mindestens 5 bis 365 Tagen und ganz besonders bevorzugt von mindestens 10 bis 365 Tagen.

### Mischung der Zusammensetzungen (A) und (B)

In Schritt (4) des erfindungsgemäßen Verfahrens werden die beiden Zusammensetzungen (A) und (B) miteinander vermischt. Auf diese Weise wird das anwendungsbereite Mittel zur Färbung des keratinischen Materials hergestellt.

Die zu dieser Erfindung führenden Arbeiten haben gezeigt, dass die Wasser (B1), Fettbestandteile (B2) und farbgebende Verbindungen (B3) enthaltende Zusammensetzung (B) insbesondere dann optimalen Einfluss auf den wasserarmen Silan-Blend (d.h. auf die Zusammensetzung (A)) nehmen kann, wenn die Zusammensetzungen (A) und (B) vor der Anwendung miteinander vermischt wurden.

Dieses Vermischen kann beispielsweise durch Verrrühren oder Verschütteln erfolgen. Ganz gesonders vorteilhaft ist es, die beiden Zusammensetzungen (A) und (B) getrennt in zwei Containern zu konfektionieren, und vor der Anwendung dann die gesamte Menge der Zusammensetzung (A) aus ihrem Container in den Container zu überführen, in dem sich die zweite Zusammsensetzung (B) befindet. Es ist besonders bevorzugt, den Mischvorgang durch Schütteln zu unterstützen.

Im Rahmen einer ganz besonders bevorzugten Ausführungsform ist ein erfindungsgemäßes Verfahren dadurch gekennzeichnet, dass auf dem keratinischen Material eine Zusammensetzung angewendet wird, die unmittelbar vor der Anwendung durch Verschütteln der ersten Zusammensetzung (A) und der zweiten Zusammensetzung (B) hergestellt wurde.

Die beiden Zusammensetzungen (A) und (B) können in unterschiedlichen Mengenverhältnissen miteinander vermischt werden. So ist es beispielsweise möglich, 10 Gewichtsanteile an Zusammensetzung (A) mit 1 Gewichtsanteil an Zusammensetzung (B) zu vermischen.

Besonders bevorzugt wird die Zusammensetzung (A) in Form eines relativ hoch konzentrierten, wasserarmen Silan-Blends eingesetzt, der durch Vermischen mit der Zusammensetzung (B) quasi verdünnt wird. Aus diesem Grund ist es ganz besonders bevorzugt, die Zusammensetzung (A) mit einem Gewichtsüberschuss an Zusammensetzung (B) zu vermischen. So kann beispielsweise 1 Gewichtsanteil (A) mit 20 Gewichtsanteilen (B) vermischt werden, oder 1 Gewichtsanteil (A) wird mit 1 Gewichtsanteil (B) vermischt, oder aber 1 Gewichtsanteil (A) wird mit 5 Gewichtsanteilen (B) vermischt, oder aber 1 Gewichtsanteil (A) wird mit 10 Gewichtsanteilen (B) vermischt.

Im Rahmen einer weiteren besonders bevorzugten Ausführungsform ist ein erfindungsgemäße Verfahren gekennzeichnet durch das Vermischen der Zusammensetzungen (A) und (B) im Gewichtsmengenverhältnis (A)/(B) von 1:20 bis 20:1, bevorzugt von 1:5 bis 1:20, und ganz besonders bevorzugt von 1:6 bis 1:14.

### Anwendung der Mischung aus (A) und (B) auf dem keratinischen Material.

Im Anschluss an das Vermischen der beiden Zubereitungen (A) und (B) wird die auf diese Weise hergestellte anwendungsbereite Mischung auf das keratinische Material appliziert. In diesem Zusammenhang hat es sich als besonders bevorzugt erwiesen, die Mischung aus (A) und (B) direkt im Anschluss an das Vermischen, d.h. innerhalb eines Zeitraumaumes von höchstents 60 Minuten, bevorzugt höchstens 45 Minuten und ganz besonders bevorzugt höchstnes 30 Minuten auf dem keratinischen Material anzuwenden.

Im Rahmen einer weiteren besonders bevorzugten Ausführungsform ist ein erfindungsgemäßes Verfahren deshalb dadurch gekennzeichnet, dass die Mischung aus (A) und (B) innerhalb eines Zeitraums von 1 bis 60 Minuten, bevorzugt von 1 bis 45 Minuten und ganz besonders bevorzugt innerhalb eines Zeitraums von 1 bis 30 Minuten nach ihrem Vermischen auf dem keratinischen Material angewendet wird.

Die Anwendungsdauer, während welcher die Mischung aus (A) und (B) auf das keratinische Material einwirkt kann im Prinzip 1 bis 60 Minuten betragen. Ein wesentlicher Vorteil des auf diesem System beruhenden Färbeprinzips liegt jedoch darin, dass innerhalb sehr kurzer Zeiträume sehr intensive und widerstandsfähige Färbungen erzeugt werden können. Aus diesem Grund ist es ganz besonders bevorzugt, die aus (A) und (B) hergestellte Anwendungsmischung für einen recht kurzen Zeitraum von 1 bis 10 Minuten auf dem Keratinmaterial zu belassen, und im Anschluss daran mit Wasser auszuwaschen.

Im Rahmen einer weiteren besonders bevorzugten Ausführungsform ist ein erfindungsgemäßes Verfahren deshalb gekennzeichnet durch
(5) die Anwendung der Mischung aus (A) und (B) auf dem keratinischen Material, das Einwirken lassen der Mischung auf dem keratinischen Material für einen Zeitraum von 1 bis 10 Minuten, und das Auswaschen der Mischung mit Wasser.

### Anwendung von weiteren Zusammensetzungen im Verfahren

Im Rahmen einer Ausführungsform kann nur die Abmischung der beiden Zusammensetzungen (A) und (B) auf dem keratinischen Material angewendet werden. Bei Anwendung des erfindungsgemäßen Verfahrens zum Färben von keratinischem Material kann es jedoch auch ganz besonders bevorzugt sein, wenn auf dem Keratinmaterial nicht nur die beiden Zusammensetzungen (A) und (B), sondern weiterhin noch mindestens eine dritte Zusammensetzung (C) angewendet werden.

Diese dritte Zusammensetzung (C) kann beispielsweise nach der Anwendung der Abmischung auf (A) und (B) auf das keratinische Material appliziert werden.

Besonders bevorzgut wird die dritte Zusammensetzung (C) auf dem Keratinmaterial angewendet, nachdem das Gemisch aus (A) und (B), aufgetragen, einwirken gelassen und wieder mit Wasser ausgespült wurde. Innerhalb dieser Ausführungsform kann die dritte Zusammensetzung (C) beispielsweise die Funktion einer Farbversiegelung übernehmen. Die Farbversiegelung kann insbesondere dadurch erfolgen, dass die Zusammensetzung (C) mindestens ein filmbildendes Polymer enthält.

Im Rahmen einer weiteren besonders Ausführungsform ganz besonders bevorzugt ist ein erfindungsgemäßes Verfahren, umfassend die
(6) Anwendung einer Zusammensetzung (C) auf dem keratinischen Material, wobei die Zusammensetzung (C) enthält:
(C1) mindestens ein filmbildendes Polymer.

### Filmbildende Polymere

Die zuvor beschriebene Zusammensetzung (C) enthält mindestens ein filmbildendes Polymer (C1). Als optionalen, zusätzlichen Bestandteil können aber auch die Zusammensetzungen (A) und (B) mindestens ein filmbildenes Polymer enthalten.

Unter Polymeren werden Makromoleküle mit einem Molekulargewicht von mindestens 1000 g/mol, bevorzugt von mindestens 2500 g/mol, besonders bevorzugt von mindestens 5000 g/mol, verstanden, welche aus gleichen, sich wiederholenden organischen Einheiten bestehen. Bei den Polymeren der vorliegenden Erfindung kann es sich um synthetisch hergestellte Polymere handeln, die durch Polymerisation eines Monomertyps oder durch Polymerisation verschiedener, strukturell voneinander unterschiedlicher Monomertypen hergestellt werden. Wird das Polymer durch Polymerisation eines Monomertyps hergestellt, spricht man von Homo-Polymeren. Werden strukturell unterschiedliche Monomertypen in der Polymerisation eingesetzt, wird das resultierende Polymer als Copolymer bezeichnet.

Das maximale Molekulargewicht des Polymers hängt von dem Polymerisationsgrad (Anzahl der polymerisierten Monomere) und der Ansatzgröße ab und wird durch die Polymerisationsmethode mitbestimmt. Im Sinne der vorliegenden Erfindung ist es bevorzugt, wenn das maximale Molekulargewicht des filmbildenden, hydrophoben Polymers (c) nicht mehr als 10⁷ g/mol, bevorzugt nicht mehr als 10⁶ g/mol und besonders bevorzugt nicht mehr als 10⁵ g/mol beträgt.

Unter einem filmbildenden Polymer wird im Sinne der Erfindung ein Polymer verstanden, welches in der Lage ist, auf einem Substat, beispielsweise auf einem keratinischen Material oder einer keratinischen Faser, einen Film auszubilden. Die Ausbildung eines Films kann beispielsweise durch Betrachtung des mit dem Polymer behandelten Keratinmaterials unter einem Mikroskop nachgewiesen werden.

Die filmbildenden Polymere können hydrophil oder hydrophob sein.

Im Rahmen einer ersten Ausführungsform kann es bevorzugt sein, in der Zubereitung (B), (C) und/oder (D), ganz besonders in der Zubereitung (D), mindestens ein hydrophobes, filmbildendes Polymer einzusetzen.

Unter einem hydrophoben Polymer wird ein Polymer verstanden, dass eine Löslichkeit in Wasser bei 25 °C (760 mmHg) von weniger als 1 Gew.-% besitzt.

Die Wasserlöslichkeit des filmbildenden, hydrophoben Polymers kann beispielsweise auf dem folgenden Weg bestimmt werden. 1,0 g des Polymers werden in ein Becherglas gegeben. Mit Wasser wird auf 100 g aufgefüllt. Es wird ein Rührfisch hinzugegeben, und die Mischung wird auf einem Magnetrührer unter Rühren auf 25 °C erwärmt. Es wird für 60 Minuten gerührt. Danach wird die wässrige Mischung visuell beurteilt. Sofern sich die Polymer-Wasser-Mischung aufgrund einer hohen Trübung des Gemisches nicht visuell beurteilten lässt, wird die Mischung filtriert. Bleibt auf dem Filterpapier ein Anteil an ungelöstem Polymer zurück, dann liegt die Löslichkeit des Polymers bei weniger als 1 Gew.-%.

Hier können insbesondere die Polymere des Acrylsäure-Typs, die Polyurethane, die Polyester, die Polyamide, die Polyharnstoffe, die Cellulose-Polymere, die Nitro-Cellulose-Polymere, die Silikon-Polymere, die Polymere des Acrylamid-Typs und die Polyisoprene genannt werden.

Besonders gut geeignete filmbildende, hydrophobe Polymere sind beispielsweise Polymere aus der Gruppe der Copolymere von Acrylsäure, der Copolymere der Methacrylsäure, der Homopolymere oder Copolymere von Acrylsäure-Estern, der Homopolymere oder Copolymere von Methacrylsäure-Estern, der Homopolymere oder Copolymere von Acrylsäureamiden, der Homopolymere oder Copolymere von Methacrylsäure-Amiden, der Copolymere des Vinylpyrrolidons, der Copolymere des Vinylalkohols, der Copolymere des Vinylacetats, der Homopolymere oder Copolymere des Ethylens, der Homopolymere oder Copolymere des Propylens, der Homopolymere oder Copolymere des Styrens, der Polyurethane, der Polyester und/oder der Polyamide.

In einer weiteren bevorzugten Ausführungsform ist ein erfindungsgemäßes Mittel dadurch gekennzeichnet, dass es mindestens ein filmbildendes, hydrophobes Polymer (c) enthält, das ausgewählt ist aus der Gruppe der Copolymere von Acrylsäure, der Copolymere der Methacrylsäure, der Homopolymere oder Copolymere von Acrylsäure-Estern, der Homopolymere oder Copolymere von Methacrylsäure-Estern, der Homopolymere oder Copolymere von Acrylsäureamiden, der Homopolymere oder Copolymere von Methacrylsäure-Amiden, der Copolymere des Vinylpyrrolidons, der Copolymere des Vinylalkohols, der Copolymere des Vinylacetats, der Homopolymere oder Copolymere des Ethylens, der Homopolymere oder Copolymere des Propylens, der Homopolymere oder Copolymere des Styrens, der Polyurethane, der Polyester und/oder der Polyamide.

Zur Lösung der erfindungsgemäßen Aufgabenstellung haben sich insbesondere die filmbildenden hydrophoben Polymere als gut geeignet erwiesen, die aus der Gruppe der synthetischen Polymere, der durch radikalische Polymerisation erhältlichen Polymere oder der natürlichen Polymere ausgewählt werden.

Weitere besonders gut geeignete filmbildende hydrophobe Polymere können ausgewählt werden aus den Homopolymere oder Copolymeren von Olefinen, wie beispielsweise Cycloolefinen, Butadien, Isopren oder Styren, Vinylethern, Vinylamiden, den Estern oder Amiden von (Meth)Acrylsäure mit mindestens einer C₁-C₂₀-Alkylgruppe, einer Arylgruppe oder einer C₂-C₁₀-Hydroxyalkylgruppe.

Weitere filmbildende hydrophobe Polymere können ausgewählt sein aus den Homo- oder Copolymeren von Isooctyl-(meth)acrylat; Isononyl-(meth)acrylat; 2-Ethylhexyl-(meth)acrylat; Lauryl-(meth)acrylat); isopentyl-(meth)acrylat; n-Butyl-(meth)acrylat); Isobutyl-(meth)acrylat; Ethyl-(meth)acrylat; Methyl-(meth)acrylat; tert-Butyl-(meth)acrylat; Stearyl-(meth)acrylat; Hydroxyethyl-(meth)acrylat; 2-Hydroxypropyl-(methacrylat; 3-Hydroxypropyl-(meth)acrylat und/oder Gemischen hiervon.

Weitere filmbildende hydrophobe Polymere können ausgewählt sein aus den Homo- oder Copolymeren von (Meth)acrylamid; N-Alkyl-(meth)acrylamiden, insbesondere solchen mit C2-C18 Alkylgruppen, wie beispielsweise N-Ethyl-acrylamid, N-tert-butyl-acrylamid, le N-Octyl-crylamid; N-Di(C1-C4)alkyl-(meth)acrylamid.

Weitere bevorzugte anionische Copolymere sind beispielsweise Copolymere aus Acrylsäure, Methacrylsäure oder deren C₁-C₆-Alkylestern, wie sie unter der INCI-Deklaration Acrylates Copolymere vertrieben werden. Ein geeignetes Handelsprodukt ist beispielsweise Aculyn^{®} 33 der Firma Rohm & Haas. Weiterhin bevorzugt sind aber auch Copolymere aus Acrylsäure, Methacrylsäure oder deren C₁-C₆-Alkylestern und den Estern einer ethylenisch ungesättigten Säure und einem alkoxylierten Fettalkohol. Geeignete ethylenisch ungesättigte Säuren sind insbesondere Acrylsäure, Methacrylsäure und Itaconsäure; geeignete alkoxylierte Fettalkohole sind insbesondere Steareth-20 oder Ceteth-20.

Auf dem Markt befindliche ganz besonders bevorzugte Polymere sind beispielsweise Aculyn^{®} 22 (Acrylates/Steareth-20 Me-thacrylate Copolymer), Aculyn^{®}28 (Acrylates/Beheneth-25 Methacrylate Copolymer), Structure 2001^{®} (Acryla-tes/Steareth-20 Itaconate Copolymer), Structure 3001^{®} (Acrylates/Ceteth-20 Itaconate Copolymer), Structure Plus^{®} (Acrylates/Aminoacrylates C10-30 Alkyl PEG-20 Itaconate Copolymer), Carbopol^{®} 1342, 1382, Ultrez 20, Ultrez 21 (Acrylates/C10-30 Alkyl Acrylate Crosspolymer), Synthalen W 2000^{®} (Acrylates/Palmeth-25 Acrylate Copolymer) oder das Rohme und Haas vertriebene Soltex OPT (Acrylates/C12-22 Alkyl methacrylate Copolymer).

Als geeignete Polymere auf der Basis von Vinyl-Monomeren können beispielsweise genannt werden die Homo- und Copolymere des N-Vinylpyrrolidons, des Vinylcaprolactams, des Vinyl-(C1-C6-)alkyl-Pyrrols, des Vinyl-oxazols, des Vinyl-thiazols, des Vinylpyrimidins, des Vinylimidazols.

Weiterhin ganz besonders gut geeignet sind die Copolymere Octylacrylamid/acrylates/ butylaminoethyl-methacrylate copolymer, wie es beispielsweise unter den Handelsnamen AMPHOMER^{®} ou LOVOCRYL^{®} 47 von NATIONAL STARCH kommerziell vertrieben wird, oder auch die Copolymere von Acrylates/Octylacrylamide die unter den Handelsnamen DERMACRYL^{®} LT und DERMACRYL^{®} 79 von NATIONAL STARCH vertrieben werden.

Als geeignete Polymere auf der Basis von Olefinen können beispielsweise genannt werden die Homo- und Copolymere des Ethylens, des Propylens, des Butens, des Isoprens und des Butadiens.

Im Rahmen einer weiteren Ausführungsform können als filmbildenden hydrophoben Polymere die Block-Copolymere eingesetzt werden, die mindestens einen Block aus Styren oder den Derivaten des Styrens umfassen. Bei diesen Block-Copolymeren kann es sich um Copolymere handeln, die neben einem Styren-Block einen oder mehrere weitere Blöcke enthalten, wie beispielsweise Styren/Ethylen, Styren/Ethylen/Butylen, Styen/Butylen, Styren/Isopren, Styren/Butadien. Entsprechende Polymere werden von der BASF unter dem Handelsnamen "Luvitol HSB" kommerziell vertrieben.

Es konnten auch dann intensive und waschechte Färbungen erhalten werden konnten, wenn die Zusammensetzung (C) mindestens ein filmbildendes Polymer enthielt, das ausgewählt wurde aus der Gruppe der der Homopolymere und Copolymere von Acrylsäure, der Homopolymere und Copolymere von Methacrylsäure, der Homopolymere und Copolymere von Acrylsäure-Estern, der Homopolymere und Copolymere von Methacrylsäure-Estern, der Homopolymere und Copolymere von Acrylsäureamiden, der Homopolymere und Copolymere von Methacrylsäure-Amiden, der Homopolymere und Copolymere des Vinylpyrrolidons, der Homopolymere und Copolymere des Vinylalkohols, der Homopolymere und Copolymere des Vinylacetats, der Homopolymere und Copolymere des Ethylens, der Homopolymere und Copolymere des Propylens, der Homopolymere und Copolymere des Styrens, der Polyurethane, der Polyester und der Polyamide.

In einer weiteren bevorzugten Ausführungsform ist ein erfindungsgemäßes Verfahren dadurch gekennzeichnet, dass die Zusammensetzung (C) mindestens ein filmbildendes Polymer enthält, das ausgewählt ist aus der Gruppe der Homopolymere und Copolymere von Acrylsäure, der Homopolymere und Copolymere von Methacrylsäure, der Homopolymere und Copolymere von Acrylsäure-Estern, der Homopolymere und Copolymere von Methacrylsäure-Estern, der Homopolymere und Copolymere von Acrylsäureamiden, der Homopolymere und Copolymere von Methacrylsäure-Amiden, der Homopolymere und Copolymere des Vinylpyrrolidons, der Homopolymere und Copolymere des Vinylalkohols, der Homopolymere und Copolymere des Vinylacetats, der Homopolymere und Copolymere des Ethylens, der Homopolymere und Copolymere des Propylens, der Homopolymere und Copolymere des Styrens, der Polyurethane, der Polyester und der Polyamide.

Im Rahmen einerweiteren Ausführungsform kann es auch bevorzugt sein, in der Zusammensetzung (C) mindestens ein hydrophiles, filmbildendes Polymer einzusetzen.

Unter einem hydrophilen Polymer wird ein Polymer verstanden, dass eine Löslichkeit in Wasser bei 25 °C (760 mmHg) von mehr als 1 Gew.-%, bevorzugt von mehr als 2 Gew.-%, besitzt.

Die Wasserlöslichkeit des filmbildenden, hydrophilen Polymers kann beispielsweise auf dem folgenden Weg bestimmt werden. 1,0 g des Polymers werden in ein Becherglas gegeben. Mit Wasser wird auf 100 g aufgefüllt. Es wird ein Rührfisch hinzugegeben, und die Mischung wird auf einem Magnetrührer unter Rühren auf 25 °C erwärmt. Es wird für 60 Minuten gerührt. Danach wird die wässrige Mischung visuell beurteilt. Ein vollständig gelöstes Polymer erscheint markoskopisch homogen. Sofern sich die Polymer-Wasser-Mischung aufgrund einer hohen Trübung des Gemisches nicht visuell beurteilten lässt, wird die Mischung filtriert. Bleibt auf dem Filterpapier kein ungelöstes Polymer zurück, dann liegt die Löslichkeit des Polymers bei mehr als 1 Gew.-%.

Als filmbildende, hydrophile Polymere können nichtionische, anionische und kationische Polymere eingesetzt werden.

Geeignete filmbildende, hydrophile Polymere können beispielsweise aus der Gruppe der Polyvinylpyrrolidon-(Co)Polymere, der Polyvinylalkohol-(Co)Polymere, der Vinylacetat-(Co)Polymere, der Carboxyvinyl-(Co)Polymere, der Acrylsäure-(Co)Polymere, der Methacrylsäure-(Co)Polymere, der natürlichen Gummen, der Polysaccharide und/oder der Acrylamid-(Co)Polymere ausgewählt werden.

Weiterhin ist es ganz besonders bevorzugt, als filmbildendes hydrophiles Polymer Polyvinylpyrrolidon (PVP) und/oder ein Vinylpyrrolidon-haltiges Copolymer einzusetzen.

In einer weiteren ganz besonders bevorzugten Ausführungsform ist ein erfindungsgemäßes Mittel dadurch gekennzeichnet, dass es (c) mindestens ein filmbildendes, hydrophiles Polymer enthält, das ausgewählt ist aus der Gruppe aus Polyvinylpyrrolidon (PVP) und den Copolymeren des Polyvinylpyrrolidons.

Es ist weiterhin bevorzugt, wenn das erfindungsgemäße Mittel als filmbildendes, hydrophiles Polymer Polyvinylpyrrolidon (PVP) enthält. Überraschenderweise war auch die Waschechtheit der Färbungen, die mit mit PVP-haltigen Mitteln (b9 erhalten werden konnten, sehr gut.

Besonders gut geeignete Polyvinylpyrrolidone sind beispielsweise unter der Bezeichnung Luviskol^{®} K von der BASF SE erhältlich, insbesondere Luviskol^{®}K 90 oder Luviskol^{®}K 85 der Firma BASF SE.

Als weiteres explizit ganz besonders gut geeignetes Polyvinylpyrrolidon (PVP) kann auch das Polymer PVP K30 eingesetzt werden, das von der Firma Ashland (ISP, POI Chemical) vertrieben wird. PVP K 30 ist ein in kaltem Wasser sehr gut lösliches Polyvinylpyrrolidon, welches die CAS-Nummer 9003-39-8 besitzt. Das Molgewicht von PVP K 30 liegt bei ca. 40000 g/mol.

Weitere ganz besonders gut geeignete Polyvinylpyrrolidone sind die unter den Handelsnamen LUVITEC K 17, LUVITEC K 30, LUVITEC K 60, LUVITEC K 80, LUVITEC K 85, LUVITEC K 90 und LUVITEC K 115 bekannten und von der BASF erhältlichen Substanzen.

Der Einsatz von filmbildenden hydrophilen Polymeren aus der Gruppe der Copolymere des Polyvinylpyrrolidons hat ebenfalls zu besonders guten und waschechten Farbergebnissen geführt.

Als besonders gut geeignete filmbildende, hydrophile Polymere können in diesem Zusammenhang Vinylpyrrolidon-Vinylester-Copolymere genannt werden, wie sie beispielsweise unter dem Warenzeichen Luviskol^{®} (BASF) vertrieben werden. Luviskol^{®} VA 64 und Luviskol^{®} VA 73, jeweils Vinylpyrrolidon/Vinylacetat-Copolymere, sind besonders bevorzugte nichtionische Polymere.

Von den Vinylpyrrolidon-haltigen Copolymeren werden ganz besonders bevorzugt ein Styrene/VP Copolymer und/oder ein Vinylpyrrolidon-Vinylacetat-Copolymer und/oder ein VP/DMAPA Acrylates Copolymer und/oder ein VP/Vinyl Caprolactam/DMAPA Acrylates Copolymer in den kosmetischen Zusammensetzungen eingesetzt.

Vinylpyrrolidon-Vinylacetat-Copolymere werden unter der Bezeichnung Luviskol^{®} VA von der BASF SE vertrieben. Ein VP/Vinyl Caprolactam/DMAPA Acrylates Copolymer wird beispielsweise unter dem Handelsnamen Aquaflex^{®} SF-40 von Ashland Inc. vertrieben. Ein VP/DMAPA Acrylates Copolymer wird beispielsweise unter der Bezeichnung Styleze CC-10 von Ashland vertrieben und ist ein höchst bevorzugtes Vinylpyrrolidon-haltiges Copolymer.

Als weitere geeignete Copolymere des Polyvinylpyrrolidons können auch die Copolymere genannt werden, die durch Umsetzung von N-Vinylpyrrolidon mit mindestens einem weiteren Monomer aus der Gruppe aus V-Vinylformamid, Vinylacetat, Ethylen, Propylen, Acrylamid, Vinylcaprolactam, Vinylcaprolacton und/oder Vinylalkohol erhalten werden.

In einer weiteren ganz besonders bevorzugten Ausführungsform ist ein erfindungsgemäßes Mittel dadurch gekennzeichnet, dass es mindestens ein filmbildendes, hydrophiles Polymer enthält, das ausgewählt ist aus der Gruppe aus Polyvinylpyrrolidon (PVP), Vinylpyrrolidon/Vinylacetat-Copolymeren, Vinylpyrrolidon/Styren-Copolymeren, Vinylpyrrolidon/Ethylen-Copoylmeren, Vinylpyrrolidon/Propylen-Copolymeren, Vinylpyrrolidon/Vinylcaprolactam-Copolymeren, Vinylpyrrolidon/Vinylformamid-Copolymeren und/oder Vinylpyrrolidon/Vinylalkohol-Copolymeren.

Ein weiteres geeigetes Copolymer des Vinylpyrrolidons ist das unter der INCI Bezeichnung Maltodextrin/VP Copolymer bekannte Polymer.

Weiterhin konnte intensiv gefärbtes Keratinmaterial, insbesondere Haare, mit sehr guten Waschechtheiten erhalten werden, wenn als filmbildendes, hydrophiles Polymer ein nichtionisches, filmbildendes, hydrophiles Polymer eingesetzt wurde.

Rahmen einer ersten Ausführungsform kann es bevorzugt sein, wenn die Zusammensetzung (C) mindestens ein nichtionisches, filmbildendes, hydrophiles Polymer entalten.

Unter einem nichtionischen Polymer wird erfindungsgemäß ein Polymer verstanden, das in einem protischen Lösemittel - wie beispielsweise Wasser - bei Standardbedingungen keine Struktureinheiten mit permanent kationischen oder anionischen Gruppen trägt, welche durch Gegenionen unter Erhaltung der Elektroneutralität kompensiert werden müssen. Unter kationische Gruppen fallen beispielsweise quaternisierte Ammoniumgruppen jedoch keine protonierten Amine. Unter anionische Gruppen fallen beispielsweise Carboxyl- und Sulfonsäuregruppen.

Es sind die Mittel ganz besonders bevorzugt, die als nichtionisches, filmbildendes, hydrophiles Polymer mindestens ein Polymer enthalten, das ausgewählt ist aus der Gruppe aus
- Polyvinylpyrrolidon,
- Copolymeren aus N-Vinylpyrrolidon und Vinylestern von Carbonsäuren mit 2 bis 18 Kohlenstoffatomen, insbesondere aus N-Vinylpyrrolidon und Vinylacetat,
- Copolymeren aus N-Vinylpyrrolidon und N-Vinylimidazol und Methacrylamid,
- Copolymeren aus N-Vinylpyrrolidon und N-Vinylimidazol und Acrylamid,
- Copolymeren aus N-Vinylpyrrolidon mit N,N-Di(C₁ bis C₄)-Alkylamino-(C₂ bis C₄)-alkylacrylamid.

Kommen Copolymere aus N-Vinylpyrrolidon und Vinylacetat zum Einsatz, ist es wiederum bevorzugt, wenn das Molverhältnis der aus dem Monomer N-Vinylpyrrolidon enthaltenen Struktureinheiten zu den aus dem Monomer Vinylacetat enthaltenen Struktureinheiten des Polymers im Bereich von 20 zu 80 bis 80 zu 20, insbesondere von 30 zu 70 bis 60 zu 40, liegt. Geeignete Copolymerisate aus Vinylpyrrolidon und Vinylacetat sind beispielsweise unter dem Warenzeichen Luviskol^{®} VA 37, Luviskol^{®} VA 55, Luviskol^{®} VA 64 und Luviskol^{®} VA 73 von der Firme BASF SE erhältlich.

Ein weiteres besonders bevorzugtes Polymer wird dabei ausgewählt aus den Polymeren der INCI-Bezeichnung VP/Methacrylamide/Vinyl Imidazole Copolymer, die beispielsweise unter dem Handelsnamen Luviset Clear von der Fa. BASF SE erhältlich sind.

Ein weiteres ganz besonders bevorzugtes nichtionisches, filmbildendes, hydrophiles Polymer st ein Copolymer aus N-Vinylpyrrolidon und N,N-Dimethylaminiopropylmethacrylamid,welches beispielsweise mit der INCI-Bezeichnung VP/DMAPA Acrylates Copolymer z. B. unter der Handelsbezeichnung Styleze^{®}CC 10 von der Firma ISP verkauft wird.

Ein kationisches erfindungsgemäßes Polymer ist das Copolymer aus N-Vinylpyrrolidon, N-Vinylcaprolactam, N-(3-Dimethylaminopropyl)methacrylamid und 3-(Methacryloylamino)propyl-lauryl-dimethylammoniumchlorid (INCI-Bezeichnung: Polyquaternium-69), welches beispielsweise unter dem Handelsnamen AquaStyle^{®} 300 (28-32 Gew.-% Aktivsubstanz in Ethanol-WasserGemisch, Molekulargewicht 350000) von der Firma ISP vertrieben wird.

Weitere geeignete filmbildende, hydrophile Polymere sind beispielsweise
- Vinylpyrrolidon-Vinylimidazoliummethochlorid-Copolymere, wie sie unter den Bezeichnungen Luviquat^{®} FC 370, FC 550 und der INCI-Bezeichnung Polyquaternium-16 sowie FC 905 und HM 552 angeboten werden,
- Vinylpyrrolidon-Vinylcaprolactam-Acrylat-Terpolymere, wie sie mit Acrylsäureestern und Acrylsäureamiden als dritter Monomerbaustein im Handel beispielsweise unter der Bezeichnung Aquaflex^{®} SF 40 angeboten werden.

Polyquaternium-11 ist das Reaktionsprodukt von Diethylsulfat mit einem Copolymer von Vinylpyrrolidon und Dimethylaminoethylmethacrylat. Geeignete Handelsprodukte sind beispielsweise unter den Bezeichnungen Dehyquart^{®} CC 11 und Luviquat^{®} PQ 11 PN von der BASF SE oder Gafquat 440, Gafquat 734, Gafquat 755 oder Gafquat 755N von Ashland Inc. erhältlich.

Polyquaternium-46 ist das Reaktionsprodukt von Vinylcaprolactam und Vinylpyrrolidon mit Methylvinylimidazoliummethosulfat und ist beispielsweise unter der Bezeichnung Luviquat^{®} Hold von der BASF SE erhältlich. Polyquaternium-46 wird bevorzugt in einer Menge von 1 bis 5 Gew.-% - bezogen auf das Gesamtgewicht der kosmetischen Zusammensetzung - eingesetzt. Es ganz besonders bevorzugt, dass Polyquaternium-46 in Kombination mit einer kationischen Guar-Verbindung eingesetzt wird. Es ist sogar höchst bevorzugt, dass Polyquaternium-46 in Kombination mit einer kationischen Guar-Verbindung und Polyquaternium-11 eingesetzt wird.

Als geeignete anionische filmbildende, hydrophile Polymere können zum Beispiel Acrylsäure-Polymere eingesetzt werden, die in unvernetzter oder vernetzter Form vorliegen können. Entsprechende Produkte werden beispielsweise unter den Handelsnamen Carbopol 980, 981, 954, 2984 and 5984 von der Firma Lubrizol oder auch unter den Namen Synthalen M and Synthalen K von der Firma 3V Sigma (The Sun Chemicals, Inter Harz) kommerziell vertrieben.

Beispiele für geeignete filmbildende, hydrophile Polymere aus der Gruppe der natürlichen Gums sind Xanthan Gum, Gellan Gum, Carob Gum .

Beispiele für geeignete filmbildende, hydrophile Polymere aus der Gruppe der Polysaccharide sind Hydroxyethylcellulose, Hydroxypropylcellulose, Ethylcellulose und Carboxymethyl-Cellulose.

Geeignete filmbildende, hydrophile Polymere aus der Gruppe der Acrylamde sind beispielsweise Polymere, welche ausgehend von Monomeren der (Methy)Acrylamido-C1-C4-alkyl-sulfonsäure bzw. der Salze hiervon hergestellt werden. Entsprechende Polymere können ausgewählt sein aus den Polymeren von Polyacrylamidomethansulfonsäure, Polyacrylamidoethansulfonsäure, Polyacrylamidopropansulfonsäure, Poly2-acrylamido-2-methylpropansulfonsäure, Poly-2-Methylacrylamido-2-methylpropansulfonsäure und/oder Poly-2-methylacrylamido-n-butansulfonsäure.

Bevorzugte Polyemre der Poly(meth)arylamido-C1-C4-alkyl-sulfonsäuren sind vernetzt und zu mindestens 90 % neutralisiert. Diese Poylmere können vernetzt oder auch unvernetzt sein. Vernetzte und ganz oder teilweise neutraliserte Polymere des Typs der Poly-2-acrylamido-2-methylpropansulfonsäuren sind unter den INCI-Namen "Ammonium Polyacrylamido-2-methyl-propanesulphonate" oder "Ammonium Polyacryldimethyltauramide" bekannt.

Ein weiteres bevorzugtes Polymer dieses Typs ist das der Firma Clamant unter dem Handelsnamen Hostacerin AMPS vertriebene vernetzte Poly-2-acrylamido-2methyl-propanesulphonsäure-Polymer, das teilweise mit Ammoniak neutralisiert ist.

In einer weiteren explizit ganz besonders bevorzugten Ausführungsform ist ein erfindungsgemäßes Verfahren dadurch gekennzeichnet, dass die Zusammensetzung (C) mindestens ein anionisches, filmbildendes, Polymer enthält.

In diesem Zusammenhang konnten die besten Ergebnisse erhalten werden, wenn die Zusammensetzung (C) mindestens ein filmbildendes Polymer enthält, das mindestens eine Struktureinheit der Formel (P-I) und mindestens eine Struktureinheit der Formel (P-II) umfasst 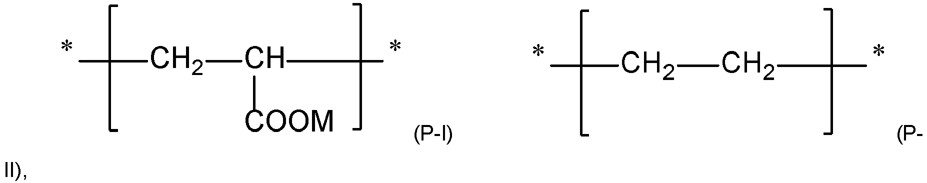 wobei
M für ein Wasserstoffatom oder für Ammonium (NH₄), Natrium, Kalium, ½ Magnesium oder ½ Calcium steht.

In einer weiteren bevorzugten Ausführungsform ist ein erfindungsgemäßes Verfahren dadurch gekennzeichnet, dass die Zusammensetzung (C) mindestens ein filmbildendes Polymer enthält, das mindestens eine Struktureinheit der Formel (P-I) und mindestens eine Struktureinheit der Formel (P-II) umfasst 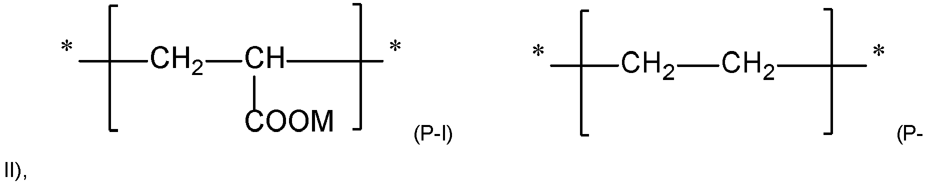 wobei
M für ein Wasserstoffatom oder für Ammonium (NH₄), Natrium, Kalium, ½ Magnesium oder ½ Calcium steht.

Wenn M für ein Wasserstoffatom steht, basiert die Struktureinheit der Formel (P-I) auf einer Acrylsäure-Einheit.

Wenn M für ein Ammonium-Gegenion steht, basiert die Struktureinheit der Formel (P-I) auf dem Ammoniumsalz der Acrylsäure.

Wenn M für ein Natrium-Gegenion steht, basiert die Struktureinheit der Formel (P-I) auf dem Natriumsalz der Acrylsäure.

Wenn M für ein Kalium-Gegenion steht, basiert die Struktureinheit der Formel (P-I) auf dem Kaliumsalz der Acrylsäure.

Wenn M für ein halbes Äquivalent eines Magnesium-Gegenions steht, basiert die Struktureinheit der Formel (P-I) auf dem Magnesiumsalz der Acrylsäure.

Wenn M für ein halbes Äquivalent eines Calcium-Gegenions steht, basiert die Struktureinheit der Formel (P-I) auf dem Calciumsalz der Acrylsäure.

Das oder die erfindungsgemäßen filmbildenden Polymere werden bevorzugt in bestimmten Mengenbereichen im erfindungsgemäßen Zubereitungen (C) eingesetzt. In diesem Zusammenhang hat es sich zur Lösung der erfindungsgemäßen Aufgabenstellung als besonders bevorzugt erwiesen, wenn die Zubereitung - jeweils bezogen auf ihr Gesamtgewicht- ein oder mehrere filmbildende Polymere in einer Gesamtmenge von 0,1 bis 18,0 Gew.-%, bevorzugt von 1,0 bis 16,0 Gew.-%, weiter bevorzugt von 5,0 bis 14,5 Gew.-% und ganz besonders bevorzugt von 8,0 bis 12,0 Gew.-% enthält.

In einer weiteren bevorzugten Ausführungsform ist ein erfindungsgemäßes Verfahren dadurch gekennzeichnet, dass die Zusammensetzung (C)- bezogen auf das Gesamtgewicht der Zusammensetzung (C) - ein oder mehrere filmbildende Polymere in einer Gesamtmenge von 0,1 bis 18,0 Gew.-%, bevorzugt von 1,0 bis 16,0 Gew.-%, weiter bevorzugt von 5,0 bis 14,5 Gew.-% und ganz besonders bevorzugt von 8,0 bis 12,0 Gew.-% enthält.

### Abfolge der Verfahrensschritte

Es ist kennzeichnend für das erfindungsgemäße Verfahren, dass es die Schritte (1), (2), (3), (4), (5) umfasst. Weiterhin kann das Verfahren noch den optionalen Schritt (6) umfassen.

Schritt (1) betrifft die Herstellung der Zusammensetzung (A), und Schritt (2) ist auf die Herstellung der Zusammensetzung (B) gerichtet. Diese beiden Schritte können entweder zeitgleich oder auch suksessive erfolgen, d.h. es ist sowohl erfindungsgemäß, wenn Schritt (1) zeitlich vor Schritt (2) erfolgt, Schritt (2) zeitlich vor Schritt (1) erfolgt oder beide Schritte (1) und (2) zeitgleich erfolgen.

In diesem Zusammenhang hat es sich als bevorzugt herausgestellt, die Herstellung der beiden Zusammensetzungen in Abhängigkeit von ihrer jeweils geplanten Lagerzeit in Schritt (3) durchzuführen. Soll beispielsweise die Zusammensetzung (A) für einen Zeitraum von 10 Tagen und die Zusammensetzung (B) für einen Zeitraum von 2 Tagen gelagert werden, so ist es bevorzugt, zunächst Zusammensetzung (B) und dann Zusammensetzung (A) herzustellen. Soll jedoch die Lagerung der Zusammensetzung (B) einen Zeitraum von 10 Tagen, die Lagerung der Zusammensetzung (A) hingegen nur einen Zeitraum von 5 Tagen andauern, so wird praktischerweise die Zusammensetzung (A) zeitlich vor der Zusammensetzung (B) hergestellt.

Im Anschluss an die Schritte (1) bzw. (2) erfolgt mit Schritt (3) die Lagerung der jeweiligen Zusammensetzung. Nach der Lagerung der Zusammensetzungen (A) und (B) werden diese miteinander vermischt, d.h. Schritt (4) des Verfahrens erfolgt zwingend nach Schritt (3).

Die Mischung aus (A) und (B) wird auf dem Keratinmaterial aufgetragen, so dass Schritt (5) des Verfahrens auch zwingend nach Schritt (4) erfolgt.

Bevorzugt ist daher ein Verfahren zur Herstellung und Anwendung eines Mittels zur Färbung von keratinischem Material, insbesondere menschlichen Haaren, umfassend die folgenden Schritte:
(1) Herstellung einer ersten Zusammensetzung (A), enthaltend
   (A1) weniger als 10 Gew.-% Wasser und
   (A2) ein oder mehrere organische C₁-C₆-Alkoxy-Silane und/oder deren Kondensationsprodukte, und
(2) Herstellung einer zweiten Zusammensetzung (B), enthaltend
   (B1) Wasser und
   (B2) ein oder mehrere Fettbestandteile und
   (B3) ein oder mehrere farbgebende Verbindungen aus der Gruppe der Pigmente und/oder der direktziehenden Farbstoffe,
(3) Lagerung der Zusammensetzung (A) und/oder (B) für einen Zeitraum von mindestens 24 Stunden,
(4) Vermischen der Zusammensetzungen (A) und (B),
(5) Anwendung der Mischung aus (A) und (B) auf dem keratinischen Material, wobei
   - Schritt (3) zeitlich nach Schritt (1) erfolgt, und
   - Schritt (3) zeitlich nach Schritt (2) erfolgt, und
   - Schritt (4) zeitlich nach Schritt (3) erfolgt, und
   - Schritt (5) zeitlich nach Schritt (4) erfolgt.

Der optional durchführbare Schritt (6) (d.h. die Anwendung der Zusammensetzung (C)) erfolgt besonders bevorzugt zeitlich nach Schritt (5).

Beispiele

### 1. Herstellung des Silan-Blends (Zusammensetzung (A))

Ein Reaktor mit beheizbarer/kühlbarer Außenhülle und mit einem Fassungsvermögen von 10 Litern wurde mit 4,67 kg Methyltrimethoxysilan (34,283 mol) befüllt. Unter Rühren wurden dann 1,33 kg (3-Aminopropyl)triethoxysilan (6,008 mol) hinzugegeben. Dieses Gemisch wurde bei 30 °C gerührt. Im Anschluss daran wurden 670 ml destilliertes Wasser (37,18 mol) tropfenweise unter kräftigem Rühren hinzugegeben, wobei die Temperatur des Reaktionsgemisches unter externer Kühlung bei 30 °C gehalten wurde. Nach Beendigung der Wasserzugabe wurde für weitere 10 Minuten nachgerührt. Danach wurde ein Vakuum von 280 mbar angelegt und das Reaktionsgemisch auf eine Temperatur von 44 °C erwärmt. Sobald das Reaktionsgemisch die Temperatur von 44 °C erreicht hatte, wurden das bei der Reaktion freigesetzte Ethanol und Methanol über einen Zeitraum von 190 Minuten abdestilliert. Im Verlauf der Destillation wurde das Vakuum auf 200 mbar abgesenkt. Die abdestillierten Alkohole wurden in einer gekühlten Vorlage aufgefangen. Danach ließ man das Reaktionsgemisch auf Raumtemperatur abkühlen. Zu dem auf diese Weise erhaltenen Gemisch wurden dann unter Rühren 3,33 kg Hexamethyldisiloxan getropft. Es wurde 10 Minuten nachgerührt. Jeweils 100 ml des Silan-Blends wurden in eine Flasche mit 100 ml Fassungsvermögen und Schraubdeckelverschluss mit Dichtung abgefüllt. Nach dem Abfüllen wurden die Flaschen fest verschlossen. Der Wassergehalt betrug kleiner 2,0 Gew.-%.

### 2. Herstellung der Zusammensetzung (B)

Es wurden die folgenden Zusammensetzungen (B) hergestellt (sofern nichts anderes angegeben ist, sind alle Angaben in Gew.-%).

### Zusammensetzung (B)

| | B Gel | B Emulsion |
|---|---|---|
| Hydroxyethylcellulose | 1,0 | --- |
| Cetylalkohol (C₁₆-Fettalkohol) | --- | 4,0 |
| Lorol techn. (C12-C18-Fettalkohole) | --- | 4,0 |
| Ceteareth-30 (Cetearylalkohol, ethoxylated 30 EO) | --- | 2,0 |
| Lavanya Zuni (Neelikon Red) CI = 12490 | 0,3 | 0,3 |
| Lavanya Belmont CI = 74160 | 0,1 | 0,1 |
| Lavanya Revolutum (Neelikon Yellow) CI = 11680 | 0,8 | 0,6 |
| Wasser (dest.) | ad 100 | ad 100 |

Emulsion: Alle Fettkomponenten wurden auf 80°C erhitzt. In einem separaten Behälter wurde auch das Wasser auf 80 °C erhitzt. Unter Rühren wurde dann das Wasser langsam zur Fettphase hinzu gegeben. Während des Abkühlens wurden unter Rühren das Tensid und die Pigmente hinzugefügt. Danach wurde die Zusamensetzung (B) auf Raumtemperatur abkühlen gelassen, und jeweils 200 g wurden in eine Flasche abgefüllt.

### 3. Herstellung der Zusammensetzungen (C)

Es wurden die folgenden Zusammensetzungen hergestellt (sofern nichts anderes angegeben ist, sind alle Angaben in Gew.-%).

| | Gew.-% |
|---|---|
| Ethylene/Sodium Acrylate Copolymer (25%ige Lösung) | 40,0 |
| Wasser | ad 100 |

### 3. Lagerung

Der in Schritt 1 hergestellte und in Flaschen abgefüllte Silan-Blend (Zusammensetzung (A)) wurde für einen Zeitraum von 21 Tagen bei 20 °C gelagert.

Die in Schritt 2 hergestellten Zusammensetzungen (B) wurden unter definierten Bedingungen gelagert:

### Lagerung (L)

| Vergleich B-V1 | B-V2 | B-E1 |
|---|---|---|
| Gel, gelagert | Emulsion, nicht gelagert | Emulsion, gelagert |
| Gel | Emulsion | Emulsion |
| 48 Stunden (2 Tage) | Ausärbung ohne Lagerung | 48 Stunden (2 Tage) |
| 20 °C | -- | 20 °C |

### 5. Anwendung

Die anwendungsbereite Zusammensetzung wurde jeweils durch Vermischen von 1,5 g der Zusammensetzung (A) und 20 g der Zusammensetzung (B) hergestellt. Die Zusammensetzungen (A) und (B) wurden jeweils für 1 Minute verschüttelt. Dann wurde dieses anwendungsbereite Mittel jeweils auf zwei Haarsträhnen (Kerling, Euronaturhaar weiß) ausgefärbt.

Eine Minuten nach Beendigung des Verschüttelns wurde die anwendungsbereite Zusammensetzung auf eine erste Strähne (Strähne 1) appliziert, 1 min einwirken gelassen, und danach ausgespült. 25 Minuten nach Beendigung des Verschüttelns wurde die anwendungsbereite Zusammensetzung auf eine zweite Strähne (Strähne 2) appliziert, 1 min einwirken gelassen und danach ausgespült.

Im Anschluss daran wurde die Zusammensetzung (C) auf jede Haarstähne appliziert, für 5 Minuten einwirken gelassen und danach ebenfalls mit Wasser ausgespült.

Die beiden gefärbten Strähnen wurden jeweils getrocknet und visuell unter einer Tageslichtlampe miteinander verglichen.

Danach wurden die Strähnen 5 mal mit der Hand unter Zuhilfenahme eines Shampoos (Schauma, 7-Kräuter) gewaschen (5 Haarwäschen = 5 HW), getrocknet und erneut visuell unter einer Tageslichtlampe miteinander verglichen.

| | Vergleich V1 | Vergleich V2 | Erfindung E1 |
|---|---|---|---|
| Zusammensetzung (A) | Silan-Blend (A) | Silan-Blend (A) | Silan-Blend (A) |
| | Lagerung 21 Tage, 20 °C | Lagerung 21 Tage, 20 °C | Lagerung 21 Tage, 20 °C |
| Zusammensetzung (B) | B-V1 | B-V2 | B-E1, |
| | Gel, | Emulsion, | Emulison |
| | Lagerung 2 Tage, 20 °C | keine Lagerung | Lagerung 2 Tage, 20 °C |
| Mischung (A) + (B) | 1,5 g (A) + 20,0 g (B-V1) | 1,5 g (A) + 20,0 g (B-V2) | 1,5 g (A) + 20,0 g (B-E1) |
| Zusammensetzung (C) | 10,0 g (C) | 10,0 g (C) | 10,0 g (C) |
| Farbunterschied zwischen Strähne 1 und 2 | hoch | gering | gering |
| Farbunterschied Strähne 1 vor und nach 5 HW | gering | hoch | gering |
| Farbunterschied Strähne 2 vor und nach 5 HW | gering | hoch | gering |
| Farbunterschied zwischen Strähne 1 und 2 nach 5 HW | hoch | hoch | gering |

## Patentansprüche

1. Verfahren zur Herstellung und Anwendung eines Mittels zur Färbung von keratinischem Material, insbesondere menschlichen Haaren, umfassend die folgenden Schritte:
(1) Herstellung einer ersten Zusammensetzung (A), enthaltend
(A1) weniger als 10 Gew.-% Wasser und
(A2) ein oder mehrere organische C₁-C₆-Alkoxy-Silane und/oder deren Kondensationsprodukte, und
(2) Herstellung einer zweiten Zusammensetzung (B), enthaltend
(B1) Wasser und
(B2) ein oder mehrere Fettbestandteile und
(B3) ein oder mehrere farbgebende Verbindungen aus der Gruppe der Pigmente und/oder der direktziehenden Farbstoffe,
(3) Lagerung der Zusammensetzung (A) und/oder (B) für einen Zeitraum von mindestens 24 Stunden,
(4) Vermischen der Zusammensetzungen (A) und (B),
(5) Anwendung der Mischung aus (A) und (B) auf dem keratinischen Material.

2. Verfahren nach Anspruch 1, **dadurch gekennzeichnet, dass** die erste Zusammensetzung (A) - bezogen auf das Gesamtgewicht der Zusammensetzung (A) - 0,01 bis 9,5 Gew.-%, bevorzugt 0,01 bis 8,0 Gew.-%, weiter bevorzugt 0,01 bis 6,0 und ganz besonders bevorzugt 0,01 bis 4,0 Gew.-% Wasser (A1) enthält.

3. Verfahren nach einem der Ansprüche 1 bis 2, **dadurch gekennzeichnet, dass** die erste Zusammensetzung (A) ein oder mehrerer organische C₁-C₆-Alkoxy-Silane (A2) der Formel (S-I) und/oder (S-II) enthält,
R₁R₂N-L-Si(OR₃)ₐ(R₄)_{b} (S-I)
wobei
- R₁, R₂ unabhängig voneinander für ein Wasserstoffatom oder eine C₁-C₆-Alkylgruppe stehen,
- L für eine lineare oder verzweigte, zweiwertige C₁-C₂₀-Alkylengruppe steht,
- R₃, R₄ unabhängig voneinander für eine C₁-C₆-Alkylgruppe stehen,
- a, für eine ganze Zahl von 1 bis 3 steht, und
- b für die ganze Zahl 3 - a steht, und
(R₅O)_{c}(R₆)_{d}Si-(A)ₑ-[NR₇-(A')]_{f}-[O-(A")]_{g}-[NR₈-(A‴)]ₕ-Si(R₆')_{d'}(OR₅')_{c'} (S-II),
wobei
- R5, R5`, R5", R6, R6' und R6" unabhängig voneinander für eine C₁-C₆-Alkylgruppe stehen,
- A, A', A", A‴ und Aʺʺ unabhängig voneinander für eine lineare oder verzweigte, zweiwertige C₁-C₂₀-Alkylengruppe stehen,
- R₇ und R₈ unabhängig voneinander für ein Wasserstoffatom, eine C₁-C₆-Alkylgruppe, eine Hydroxy-C₁-C₆-alkylgruppe, eine C₂-C₆-Alkenylgruppe, eine Amino-C₁-C₆-alkyl-gruppe oder eine Gruppierung der Formel (S-III) stehen,
- (Aʺʺ)-Si(R₆")_{d}"(OR₅")_{c}" (S-III),
- c, für eine ganze Zahl von 1 bis 3 steht,
- d für die ganze Zahl 3 - c steht,
- c' für eine ganze Zahl von 1 bis 3 steht,
- d' für die ganze Zahl 3 - c` steht,
- c" für eine ganze Zahl von 1 bis 3 steht,
- d" für die ganze Zahl 3 - c" steht,
- e für 0 oder 1 steht,
- f für 0 oder 1 steht,
- g für 0 oder 1 steht,
- h für 0 oder 1 steht,
- mit der Maßgabe, dass mindestens einer der Reste aus e, f, g und h von 0 verschieden ist,
und/oder deren Kondensationsprodukte.

4. Verfahren nach einem der Ansprüche 1 bis 3, **dadurch gekennzeichnet, dass** das die erste Zusammensetzung (A) mindestens ein organisches C₁-C₆-Alkoxysilan (A2) der Formel (S-I) enthält, das ausgewählt ist aus der Gruppe aus
- (3-Aminopropyl)triethoxysilan
- (3-Aminopropyl)trimethoxysilan
- (2-Aminoethyl)triethoxysilan
- (2-Aminoethyl)trimethoxysilan
- (3-Dimethylaminopropyl)triethoxysilan
- (3-Dimethylaminopropyl)trimethoxysilan
- (2-Dimethylaminoethyl)triethoxysilan,
- (2-Dimethylaminoethyl)trimethoxysilan
und/oder deren Kondensationsprodukten.

5. Verfahren nach einem der Ansprüche 1 bis 4, **dadurch gekennzeichnet**, die erste Zusammensetzung (A) ein oder mehrere organische C₁-C₆-Alkoxy-Silane (A2) der Formel (S-IV) enthält,
R₉Si(OR₁₀)ₖ(R₁₁)ₘ (S-IV),
wobei
- R₉ für eine C₁-C₁₂-Alkylgruppe steht,
- R₁₀ für eine C₁-C₆-Alkylgruppe steht,
- R₁₁ für eine C₁-C₆-Alkylgruppe steht
- k für eine ganze Zahl von 1 bis 3 steht, und
- m für die ganze Zahl 3 - k steht,
und/oder deren Kondensationsprodukte.

6. Verfahren nach einem der Ansprüche 1 bis 5, **dadurch gekennzeichnet, dass** die erste Zusammensetzung (A) mindestens ein organisches C₁-C₆-Alkoxysilan (A2) der Formel (S-IV) enthält, das ausgewählt ist aus der Gruppe aus
- Methyltrimethoxysilan
- Methyltriethoxysilan
- Ethyltrimethoxysilan
- Ethyltriethoxysilan
- Hexyltrimethoxysilan
- Hexyltriethoxysilan
- Octyltrimethoxysilan
- Octyltriethoxysilan
- Dodecyltrimethoxysilan,
- Dodecyltriethoxysilan,
und/oder deren Kondensationsprodukten.

7. Verfahren nach einem der Ansprüche 1 bis 6, **dadurch gekennzeichnet, dass** die erste Zusammensetzung (A) - bezogen auf das Gesamtgewicht der Zusammensetzung (A) - ein oder mehrere organische C₁-C₆-Alkoxysilane (A2) und/oder die Kondensationsprodukte hiervon in einer Gesamtmenge von 30,0 bis 85,0 Gew.-%, bevorzugt von 35,0 bis 80,0 Gew.-%, weiter bevorzugt von 40,0 bis 75,0 Gew.-%, noch weiter bevorzugt von 45,0 bis 70,0 Gew.-% und ganz besonders bevorzugt von 50,0 bis 65,0 Gew.-% enthält.

8. Verfahren nach einem der Ansrpüche 1 bis 7, **dadurch gekennzeichnet**, das die erste Zusammensetzung (A) mindestens einen kosmetischen Inhaltsstoff aus der Gruppe aus Hexamethyldisiloxan. Octamethyltrisiloxan, Decamethyltetrasiloxan, Hexamethylcyclotrisiloxan, Octamethylcyclotetrasiloxan und Decamethylcyclopentasiloxan enthält.

9. Verfahren nach einem der Ansprüche 1 bis 8, **dadurch gekennzeichnet, dass** die erste Zusammensetzung (A) - bezogen auf das Gesamtgewicht der Zusammensetzung (A) - 10,0 bis 50,0 Gew.-%, bevorzugt 15,0 bis 45,0 Gew.-%, weiter bevorzugt 20,0 bis 40,0 Gew.-%, noch weiter bevorzugt 25,0 bis 35,0 Gew.-% und ganz besonders bevorzugt 31,0 bis 34,0 Gew.-% Hexamethyldisiloxan enthält.

10. Verfahren nach einem der Ansprüche 1 bis 9, **dadurch gekennzeichnet, dass** die zweite Zuammensetzung (B) - bezogen auf das Gesamtgewicht der Zusammensetzung (B) - 10,0 bis 90,0 Gew.-%, bevorzugt 30,0 bis 90,0 Gew.-%, weiter bevorzugt 50,0 bis 90,0 Gew.-% , nocht weiter bevorzugt 70,0 bis 90,0 Gew.-% und ganz besonders bevorzugt 75,0 bis 90,0 Gew.-% Wasser (B1) enthält.

11. Verfahren nach einem der Ansprüche 1 bis 10, **dadurch gekennzeichnet, dass** die zweite Zusammensetzung (B) einen oder mehrere Fettbestandteile (B2) aus der Gruppe der C₁₂-C₃₀-Fettalkohole, der C₁₂-C₃₀-Fettsäuretriglyceride, der C₁₂-C₃₀-Fettsäuremonoglyceride, der C₁₂-C₃₀-Fettsäurediglyceride und/oder der Kohlenwasserstoffe enthält.

12. Verfahren nach einem der Ansprüche 1 bis 11, **dadurch gekennzeichnet, dass** die zweite Zusammensetzung (B) ein oder mehrere C₁₂-C₃₀-Fettalkohole (B2) aus der Gruppe aus Dodecan-1-ol, Tetradecan-1-ol, Hexadecan-1-ol, Octadecan-1-ol, Eicosan-1-ol, Heneicosan-1-ol, Docosan-1-ol, (9*Z*)-Octadec-9-en-1-ol, (9*E*)-Octadec-9-en-1-ol, (9*Z*,12*Z*)-Octadeca-9,12-dien-1-ol, (9*Z*,12*Z*,15*Z*)-Octadeca-9,12,15-trien-1-ol, (9*Z*)-Eicos-9-en-1-ol, (5*Z*,8*Z*,11*Z*,14*Z*)-Eicosa-5,8,11,14-tetraen-1-ol, (13*Z*)-Docos-13-en-1-ol), (13*E*)-Docosen-1-ol), 2-Octyl-dodecanol, 2-Hexyl-Dodecanol und/oder 2-Butyl-dodecanol enthält.

13. Verfahren nach einem der Ansprüche 1 bis 12, **dadurch gekennzeichnet, dass** die zweite Zusammensetzung (B) - bezogen auf das Gesamtgewicht der Zusammensetzung (B) - einen oder mehrere C₁₂-C₃₀-Fettalkohole (B2) in einer Gesamtmenge von 2,0 bis 50,0 Gew.-%, bevorzugt von 3,0 bis 40,0 Gew.-%, weiter bevorzugt von 4,0 bis 30,0 Gew.-%, noch weiter bevorzugt von 5,0 bis 20,0 Gew.-% und ganz besonders bevorzugt von 6,0 bis 15,0 Gew.-% enthält.

14. Verfahren nach einem der Ansprüche 1 bis 13, **dadurch gekennzeichnet, dass** die zweite Zusammensetzung (B) mindestens ein Tensid, besonders bevorzugt ein nichtionisches Tensid, enthält.

15. Verfahren nach einem der Ansprüche 1 bis 14, **dadurch gekennzeichnet, dass** die zweite Zusammensetzung (B) mindestens ein nichtionisches Tensid der Formel (T-I) enthält, 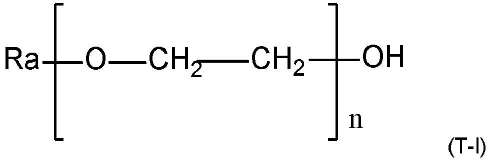 worin
Ra für eine gesättigte oder ungesättigte, unverzweigte oder verzweigte C₈-C₂₄-Alkylgruppe, bevorzugt für eine gesättigte, unverzeigte C₁₆- bis C₁₈- Alkylgruppe, steht und
n für eine ganze Zahl von 10 bis 40, bevorzugt für eine ganze Zahl von 20 bis 35 und besonders bevorzugt für die Zahl 30, steht.

16. Verfahren nach einem der Ansprüche 1 bis 15, **dadurch gekennzeichnet, dass** die zweite Zusammensetzung (B) mindestens eine farbgebende Verbindung aus der Gruppe aus der Gruppe der anorganischen Pigmente enthält, die ausgewählt ist aus der Gruppe der farbigen Metalloxide, Metallhydroxide, Metalloxidhydrate, Silicate, Metallsulfide, komplexen Metallcyanide, Metallsulfate, Bronzepigmente, Metalle, Metalllegierungen und/oder aus farbigen Pigmenten auf Mica- oder Glimmerbasis, die mit mindestens einem Metalloxid und/oder einem Metalloxychlorid beschichtet sind.

17. Verfahren nach einem der Ansprüche 1 bis 16, **dadurch gekennzeichnet, dass** die Zusammensetzung (B) mindestens eine farbgebende Verbindung (B3) aus der Gruppe der organischen Pigmente enthält, die ausgewählt ist aus der Gruppe aus Carmin, Chinacridon, Phthalocyanin, blauen Pigmente mit den Color Index Nummern CI 42090, CI 69800, CI 69825, CI 73000, CI 74100, CI 74160, gelben Pigmenten mit den Color Index Nummern CI 11680, CI 11710, CI 15985, CI 19140, CI 20040, CI 21100, CI 21108, CI 47000, CI 47005, grünen Pigmenten mit den Color Index Nummern CI 61565, CI 61570, CI 74260, orangen Pigmenten mit den Color Index Nummern CI 11725, CI 15510, CI 45370, CI 71105, roten Pigmenten mit den Color Index Nummern CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915 und/oder CI 75470.

18. Verfahren nach einem der Ansprüche 1 bis 17, **dadurch gekennzeichnet, dass** die Zusammensetzung (B) mindestens eine farbgebende Verbindung (B3) aus der Gruppe der organischen Pigmente enthält, die ausgewählt ist aus der Gruppe aus
- dem blauen Pigment mit den Color Index Nummer CI 74160,
- dem gelben Pigment mit der Color Index Nummer CI 11680, und
- dem roten Pigmenten mit der Color Index Nummer CI 12490.

19. Verfahren nach einem der Ansprüche 1 bis 18, **dadurch gekennzeichnet, dass** die zweite Zusammensetzung (B) - bezogen auf das Gesamtgewicht der Zusammensetzung (B) - ein oder mehrere farbgebende Verbindungen (B3) in einer Gesamtmenge von 0,1 bis 6,0 Gew.-%, bevorzugt von 0,3 bis 5,0 Gew.-%, weiter bevorzugt von 0,5 bis 4,0 Gew.-% und ganz besonders bevorzugt von 0,7 bis 1,5 Gew.-% enthält.

20. Verfahren nach einem der Ansprüche 1 bis 19, **gekennzeichnet durch** die (3) Lagerung der Zusammensetzung (A) für einen Zeitraum von mindestens 5 Tagen, bevorzugt von mindestens 10 Tagen, weiter bevorzugt von mindestens 14 Tagen und ganz besonders bevorzugt von mindestens 21 Tagen.

21. Verfahren nach einem der Ansprüche 1 bis 20, **gekennzeichnet durch** die (3) Lagerung der Zusammensetzung (B) für einen Zeitraum von mindestens 24 Stunden, bevorzugt von mindestens 36 Stunden, besonders bevorzugt von mindestens 48 Stunden.

22. Verfahren nach einem der Ansprüche 1 bis 21, **gekennzeichnet durch** das Vermischen der Zusammensetzungen (A) und (B) im Gewichtsmengenverhältnis (A)/(B) von 1:20 bis 20:1, bevorzugt von 1:5 bis 1:20, und ganz besonders bevorzugt von 1:6 bis 1:14.

23. Verfahren nach einem der Ansprüche 1 bis 22, **dadurch gekennzeichnet, dass** die Mischung aus (A) und (B) innerhalb eines Zeitraums von 1 bis 60 Minuten, bevorzugt von 1 bis 45 Minuten und ganz besonders bevorzugt innerhalb eines Zeitraums von 1 bis 30 Minunten nach ihrem Vermischen auf dem keratinischen Material angewendet wird.

24. Verfahren nach einem der Ansprüche 1 bis 23, **gekennzeichnet durch** die
(5) die Anwendung der Mischung aus (A) und (B) auf dem keratinischen Material, einwirkenlassen Mischung auf dem keratinischen Material für einen Zeitraum von 1 bis 10 Minuten, und auswaschen der Mischung mit Wasser.

25. Verfahren nach einem der Ansprüche 1 bis 24, umfassend die
(6) Anwendung einer Zusammensetzung (C) auf dem keratinischen Material, wobei die Zusammensetzung (C) enthält:
(C1) mindestens ein filmbildendes Polymer.

## Claims

1. Process for the preparation and use of an agent for coloring keratinous material, in particular human hair, comprising the following steps:
(1) preparing a first composition (A) containing
(A1) less than 10% by weight of water and
(A2) one or more organic C1-C6 alkoxy silanes and/or their condensation products, and
(2) preparation of a second composition (B) comprising
(B1) water and
(B2) one or more fatty components and
(B3) one or more colorant compounds selected from the group consisting of pigments and/or direct dyes,
(3) storage of the composition (A) and/or (B) for a period of at least 24 hours,
(4) mixing of the compositions (A) and (B),
(5) application of the mixture of (A) and (B) to the keratinous material.

2. Process according to claim 1, **characterized in that** the first composition (A) contains - based on the total weight of composition (A) - 0.01 to 9.5% by weight, preferably 0.01 to 8.0% by weight, further preferably 0.01 to 6.0 and most preferably 0.01 to 4.0% by weight of water (A1).

3. Process according to one of claims 1 to 2, **characterized in that** the first composition (A) comprises one or more organic C₁-C₆ alkoxy silanes (A2) of the formula (S-I) and/or (S-II),
R₁R₂N-L-Si(OR₃)ₐ(R₄)_{b} (S-I)
where
- R₁, R₂ independently of one another represent a hydrogen atom or a C₁-C₆ alkyl group,
- L is a linear or branched, divalent C₁-C₂₀ alkylene group,
- R₃, R₄ independently of one another represent a C₁-C₆ alkyl group,
- a is an integer from 1 to 3, and
- b represents the integer 3 - a, and
(R₅O)_{c}(R₆)_{d}Si-(A)ₑ-[NR₇-(A')]f-[O-(A")]_{g}-[NR₈-(A")]ₕ-Si(R₆')_{d}'(OR₅')_{c}' (S-II),
wherein
- R₅, R₅', R₅", R₆, R₆' and R₆" independently represent a C₁-C₆ alkyl group,
- A, A', A", A‴ and Aʺʺ independently represent a linear or branched C₁-C₂₀ divalent alkylene group
- R₇ and R₈ independently represent a hydrogen atom, a C₁-C₆ alkyl group, a hydroxy-C₁-C₆ alkyl group, a C₂-C₆ alkenyl group, an amino-C₁-C₆ alkyl group or a grouping of the formula (S-III),
- (Aʺʺ)-Si(R₆")_{d}"(OR₅")_{c}" (S-III),
- c stands for an integer from 1 to 3,
- d stands for an integer from 3 to c,
- c' stands for an integer from 1 to 3
- d' stands for the integer 3 - c',
- c" stands for an integer from 1 to 3
- d" stands for the integer 3 - c",
- e is 0 or 1
- f is 0 or 1
- g is 0 or 1
- h is 0 or 1
- with the proviso that at least one of the radicals from e, f, g and h is other than 0,
and/or condensation products thereof.

4. Process according to any one of claims 1 to 3, wherein the first composition (A) comprises at least one organic C₁-C₆-alkoxysilane (A2) of the formula (S-I) selected from the group consisting of
- (3-aminopropyl)triethoxysilane
- (3-aminopropyl)trimethoxysilane
- (2-aminoethyl)triethoxysilane
- (2-aminoethyl)trimethoxysilane
- (3-dimethylaminopropyl)triethoxysilane
- (3-dimethylaminopropyl)trimethoxysilane
- (2-dimethylaminoethyl)triethoxysilane,
- (2-dimethylaminoethyl)trimethoxysilane
and/or condensation products thereof.

5. Process according to any one of claims 1 to 4, **characterized in that** the first composition (A) comprises one or more organic C₁-C₆ alkoxy silanes (A2) of the formula (S-IV),
R₉Si(OR₁₀)ₖ(R₁₁)ₘ (S-IV),
where
- R₉ stands for a C₁-C₁₂ alkyl group
- R₁₀ stands for a C₁-C₆ alkyl group
- R₁₁ stands for a C₁-C₆ alkyl group
- k is an integer from 1 to 3, and
- m is an integer of 3 - k
and/or condensation products thereof.

6. Process according to any one of claims 1 to 5, **characterized in that** the first composition (A) comprises at least one organic C₁-C₆-alkoxysilane (A2) of the formula (S-IV) selected from the group consisting of
- methyltrimethoxysilane
- methyltriethoxysilane
- ethyltrimethoxysilane
- ethyltriethoxysilane
- hexyltrimethoxysilane
- hexyltriethoxysilane
- octyltrimethoxysilane
- octyltriethoxysilane
- dodecyltrimethoxysilane,
- dodecyltriethoxysilane,
and/or condensation products thereof.

7. Process according to any one of claims 1 to 6, **characterized in that** the first composition (A) comprises - based on the total weight of the composition (A) - one or more organic C₁-C₆-alkoxysilanes (A2) and/or the condensation products thereof in a total amount of from 30.0 to 85.0 wt. % by weight, preferably from 35.0 to 80.0 % by weight, more preferably from 40.0 to 75.0 % by weight, still more preferably from 45.0 to 70.0 % by weight and most preferably from 50.0 to 65.0 % by weight.

8. Process according to one of claims 1 to 7, **characterized in that** the first composition (A) comprises at least one cosmetic ingredient selected from the group consisting of hexamethyldisiloxane. octamethyltrisiloxane, decamethyltetrasiloxane, hexamethylcyclotrisiloxane, octamethylcyclotetrasiloxane and decamethylcyclopentasiloxane.

9. Process according to any one of claims 1 to 8, **characterized in that** the first composition (A) contains - based on the total weight of the composition (A) - 10.0 to 50.0% by weight, preferably 15.0 to 45.0% by weight, further preferably 20.0 to 40.0% by weight, still further preferably 25.0 to 35.0% by weight and very particularly preferably 31.0 to 34.0% by weight of hexamethyldisiloxane.

10. Process according to any one of claims 1 to 9, **characterized in that** the second composition (B) contains - based on the total weight of the composition (B) - 10.0 to 90.0% by weight, preferably 30.0 to 90.0% by weight, more preferably 50.0 to 90.0% by weight, still more preferably 70.0 to 90.0% by weight and very particularly preferably 75.0 to 90.0% by weight of water (B1).

11. Process according to any one of claims 1 to 10, wherein the second composition (B) comprises one or more fat constituents (B2) from the group consisting of C₁₂-C₃₀ fatty alcohols, C₁₂-C₃₀ fatty acid triglycerides, C₁₂-C₃₀ fatty acid monoglycerides, C₁₂-C₃₀ fatty acid diglycerides and/or hydrocarbons.

12. Process according to any one of claims 1 to 11, **characterized in that** the second composition (B) comprises one or more C₁₂-C₃₀ fatty alcohols (B2) selected from the group consisting of dodecan-1-ol, tetradecan-1-ol, hexadecan-1-ol, octadecan-1-ol, eicosan-1-ol, heneicosan-1-ol, docosan-1-ol, (9Z)-octadec-9-en-1-ol, (9E)-Octadec-9-en-1-ol, (9Z,12Z)-Octadeca-9,12-dien-1-ol, (9Z,12Z,15Z)-Octadeca-9,12,15-trien-1-ol, (9Z)-Eicos-9-en-1-ol, (5Z,8Z,11Z,14Z)-Eicosa-5, 8,11,14-tetraen-1-ol, (13Z)-docos-13-en-1-ol), (13E)-docosen-1-ol), 2-octyl-dodecanol, 2-hexyl-dodecanol and/or 2-butyl-dodecanol.

13. Process according to any one of claims 1 to 12, **characterized in that** the second composition (B) contains - based on the total weight of the composition (B) - one or more C₁₂-C₃₀ fatty alcohols (B2) in a total amount of from 2.0 to 50.0% by weight, preferably from 3.0 to 40.0% by weight, more preferably from 4.0 to 30.0% by weight, still more preferably from 5.0 to 20.0% by weight and very particularly preferably from 6.0 to 15.0% by weight.

14. Process according to any one of claims 1 to 13, **characterized in that** the second composition (B) comprises at least one surfactant, particularly preferably a nonionic surfactant.

15. Process according to any one of claims 1 to 14, **characterized in that** the second composition (B) comprises at least one nonionic surfactant of the formula (T-I), 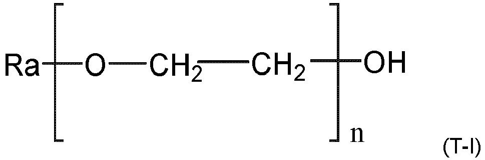 wherein
Ra represents a saturated or unsaturated, linear or branched C₈-C₂₄ alkyl group, preferably a saturated, linear C₁₆ to C₁₈ alkyl group, and
n stands for an integer from 10 to 40, preferably for an integer from 20 to 35 and particularly preferably for the number 30.

16. Process according to any one of claims 1 to 15, **characterized in that** the second composition (B) comprises at least one coloring compound selected from the group consisting of inorganic pigments, which is selected from the group consisting of colored metal oxides, metal hydroxides, metal oxide hydrates, silicates, metal sulfides, complex metal cyanides, metal sulfates, bronze pigments, metals, metal alloys and/or mica- or mica-based colored pigments coated with at least one metal oxide and/or a metal oxychloride.

17. Process according to any one of claims 1 to 16, **characterized in that** the composition (B) comprises at least one colorant compound (B3) from the group of organic pigments selected from the group consisting of carmine, quinacridone, phthalocyanine, blue pigments having the color index numbers CI 42090, CI 69800, CI 69825, CI 73000, C! 74100, C! 74160, yellow pigments having the color index numbers CI 11680, C! 11710, C! 15985, CI 19140, CI 20040, CI 21100, C! 21108, C! 47000, C! 47005, green pigments with Color Index numbers CI 61565, C! 61570, C! 74260, orange pigments with Color Index numbers Cl 11725, C! 15510, C! 45370, C! 71105, red pigments with Color Index numbers CI 12085, CI 12120, CI 12370, CI 12420, CI 12490, C! 14700, CI 15525, CI 15580, CI 15620, CI 15630, CI 15800, CI 15850, Cl 15865, Cl 15880, Cl 17200, CI 26100, C! 45380, C! 45410, C! 58000, CI 73360, CI 73915 and/or C! 75470.

18. Process according to any one of claims 1 to 17, **characterized in that** the composition (B) comprises at least one colorant compound (B3) from the group of organic pigments selected from the group consisting of
- the blue pigment having the Color Index number CI 74160,
- the yellow pigment having the Color Index number CI 11680, and
- the red pigment having the Color Index number C! 12490.

19. Process according to any one of claims 1 to 18, **characterized in that** the second composition (B) comprises - based on the total weight of the composition (B) - one or more color-imparting compounds (B3) in a total amount of from 0.1 to 6.0% by weight, preferably from 0.3 to 5.0% by weight, more preferably from 0.5 to 4.0% by weight and very preferably from 0.7 to 1.5% by weight.

20. Process according to any one of claims 1 to 19, **characterized by** the (3) storing the composition (A) for a period of at least 5 days, preferably at least 10 days, more preferably at least 14 days and most preferably at least 21 days.

21. Process according to any one of claims 1 to 20, **characterized by** the (3) storing the composition (B) for a period of at least 24 hours, preferably at least 36 hours, more preferably at least 48 hours.

22. Process according to any one of claims 1 to 21, **characterized by** mixing compositions (A) and (B) in a weight ratio (A)/(B) of from 1:20 to 20:1, preferably from 1:5 to 1:20, and most preferably from 1:6 to 1:14.

23. Process according to any one of claims 1 to 22, **characterized in that** the mixture of (A) and (B) is applied to the keratinous material within a period of from 1 to 60 minutes, preferably from 1 to 45 minutes, and most preferably within a period of from 1 to 30 minutes after mixing thereof.

24. Process according to any one of claims 1 to 23, **characterized by** the (5) applying the mixture of (A) and (B) to the keratinous material, allowing the mixture to act on the keratinous material for a period of 1 to 10 minutes, and washing out the mixture with water.

25. Process according to any one of claims 1 to 24, comprising.
(6) applying a composition (C) to the keratinous material, wherein the composition (C) comprises:
(C1) at least one film-forming polymer.

## Revendications

1. Procédé de préparation et d'utilisation d'une composition pour la coloration de matières kératiniques, en particulier de cheveux humains, comprenant les étapes suivantes:
(1) préparation d'une première composition (A) contenant
(A1) moins de 10 % en poids d'eau et
(A2) un ou plusieurs alcoxy-silanes organiques en C₁-C₆ et/ou leurs produits de condensation, et
(2) préparation d'une deuxième composition (B) contenant
(B1) de l'eau et
(B2) un ou plusieurs ingrédients gras et
(B3) un ou plusieurs composés colorants choisis dans le groupe des pigments et/ou des colorants directs,
(3) stockage de la composition (A) et/ou (B) pendant une période d'au moins 24 heures,
(4) mélange des compositions (A) et (B),
(5) application du mélange de (A) et (B) sur la matière kératinique.

2. Procédé selon la revendication 1, **caractérisé en ce que** la première composition (A) contient
- par rapport au poids total de la composition (A) - de 0,01 à 9,5 % en poids, de préférence de 0,01 à 8,0 % en poids, plus préférentiellement de 0,01 à 6,0 et tout particulièrement de 0,01 à 4,0 % en poids d'eau (A1).

3. Procédé selon l'une quelconque des revendications 1 à 2, **caractérisé en ce que** la première composition (A) contient un ou plusieurs alcoxy-silanes organiques en C₁-C₆ (A2) de formule (S-I) et/ou (S-II),
R₁R₂N-L-Si(OR₃)ₐ(R₄)_{b} (S-I)
où
- R₁, R₂ représentent indépendamment l'un de l'autre un atome d'hydrogène ou un groupe alkyle en C₁-C₆,
- L représente un groupe alkylène divalent en C₁-C₂₀, linéaire ou ramifié,
- R₃, R₄ représentent indépendamment un groupe alkyle en C₁-C₆,
- a, représente un nombre entier de 1 à 3, et
- b représente le nombre entier 3 - a, et
(R₅O)_{c}(R₆)_{d}Si-(A)ₑ-[NR₇-(A')]f-[O-(A")]_{g}-[NR₈-(A")]ₕ-Si(R₆')_{d}'(OR₅')_{c}' (S-II),
où
- R₅, R₅', R₅", R₆, R₆' et R₆" représentent indépendamment un groupe alkyle en C₁-C₆,
- A, A', A", A'" et A"" représentent indépendamment un groupe alkylène divalent en C₁-C₂₀, linéaire ou ramifié,
- R7 et R8 représentent indépendamment un atome d'hydrogène, un groupe alkyle en Ci-Ce, un groupe hydroxyalkyle en Ci-Ce, un groupe alcényle en C₂-C₆, un groupe aminoalkyle en Ci-Ce ou un groupement de formule (S-III),
- (Aʺʺ)-Si(R₆")_{d}"(OR₅")_{c}" (S-III),
- c représente un nombre entier de 1 à 3,
- d représente un nombre entier de 3 - c,
- c' représente un nombre entier de 1 à 3,
- d' représente le nombre entier 3 - c',
- c" représente un nombre entier de 1 à 3,
- d" représente le nombre entier 3 - c",
- e est égal à 0 ou 1,
- f est égal à 0 ou 1,
- g est égal à 0 ou 1,
- h est égal à 0 ou 1,
- à condition qu'au moins l'un des radicaux parmi e, f, g et h soit différent de 0,
et/ou leurs produits de condensation.

4. Procédé selon l'une quelconque des revendications 1 à 3, **caractérisé en ce que** la première composition (A) contient au moins un alcoxysilane organique en Ci-Ce (A2) de formule (S-I) choisi dans le groupe constitué de
- (3-aminopropyl)triéthoxysilane
- (3-aminopropyl)triméthoxysilane
- (2-aminoéthyl)triéthoxysilane
- (2-aminoéthyl)triméthoxysilane
- (3-diméthylaminopropyl)triéthoxysilane
- (3-diméthylaminopropyl)triméthoxysilane
- (2-diméthylaminoéthyl)triéthoxysilane,
- (2-diméthylaminoéthyl)triméthoxysilane
et/ou leurs produits de condensation.

5. Procédé selon l'une quelconque des revendications 1 à 4, **caractérisé en ce que** la première composition (A) contient un ou plusieurs alcoxy-silanes organiques en Ci-Ce (A2) de formule (S-IV),
R9Si(OR10)k(R11)m (S-IV),
où
- R9 représente un groupe alkyle en C₁-C₁₂,
- R10 représente un groupe alkyle en Ci-Ce,
- R11 représente un groupe alkyle en Ci-Ce
- k représente un nombre entier de 1 à 3, et
- m représente le nombre entier 3 - k,
et/ou leurs produits de condensation.

6. Procédé selon l'une quelconque des revendications 1 à 5, **caractérisé en ce que** la première composition (A) comprend au moins un alcoxysilane organique en Ci-Ce (A2) de formule (S-IV) choisi dans le groupe constitué par
- méthyltriméthoxysilane
- méthyltriéthoxysilane
- éthyltriméthoxysilane
- éthyltriéthoxysilane
- hexyltriméthoxysilane
- hexyltriéthoxysilane
- octyltriméthoxysilane
- octyltriéthoxysilane
- le dodécyltriméthoxysilane,
- le dodécyltriéthoxysilane,
et/ou leurs produits de condensation.

7. Procédé selon l'une quelconque des revendications 1 à 6, **caractérisé en ce que** la première composition (A) contient - par rapport au poids total de la composition (A) - un ou plusieurs alcoxysilanes organiques en Ci-Ce (A2) et/ou les produits de condensation de ceux-ci en une quantité totale de 30,0 à 85,0 % en poids. % en poids, de préférence de 35,0 à 80,0 % en poids, de préférence encore de 40,0 à 75,0 % en poids, de préférence encore de 45,0 à 70,0 % en poids et de préférence encore de 50,0 à 65,0 % en poids.

8. Procédé selon l'une quelconque des revendications 1 à 7, **caractérisé en ce que** la première composition (A) contient au moins un ingrédient cosmétique choisi dans le groupe constitué par l'hexaméthyldisiloxane. Octaméthyltrisiloxane, décaméthyltétrasiloxane, hexaméthylcyclotrisiloxane, octaméthylcyclotétrasiloxane et décaméthylcyclopentasiloxane.

9. Procédé selon l'une des revendications 1 à 8, **caractérisé en ce que** la première composition (A) contient - par rapport au poids total de la composition (A) - de 10,0 à 50,0 % en poids, de préférence de 15,0 à 45,0 % en poids, plus préférentiellement de 20,0 à 40,0 % en poids, encore plus préférentiellement de 25,0 à 35,0 % en poids et tout particulièrement de 31,0 à 34,0 % en poids d'hexaméthyldisiloxane.

10. Procédé selon l'une des revendications 1 à 9, **caractérisé en ce que** la deuxième composition (B) contient - par rapport au poids total de la composition (B) - de 10,0 à 90,0 % en poids, de préférence de 30,0 à 90,0 % en poids, de préférence encore de 50,0 à 90,0 % en poids, de préférence encore de 70,0 à 90,0 % en poids et de manière tout à fait préférée de 75,0 à 90,0 % en poids d'eau (B1).

11. Procédé selon l'une des revendications 1 à 10, **caractérisé en ce que** la deuxième composition (B) contient un ou plusieurs composants gras (B2) choisis dans le groupe des alcools gras en C₁₂-C₃₀, des triglycérides d'acides gras en C₁₂-C₃₀, des monoglycérides d'acides gras en C₁₂-C₃₀, des diglycérides d'acides gras en C₁₂-C₃₀ et/ou des hydrocarbures.

12. Procédé selon l'une quelconque des revendications 1 à 11, **caractérisé en ce que** la seconde composition (B) comprend un ou plusieurs alcools gras en C₁₂-C₃₀ (B2) choisis dans le groupe constitué par le dodécane-1-ol, le tétradécane-1-ol, l'hexadécane-1-ol, l'octadécane-1-ol, l'eicosane-1-ol, l'hénéicosane-1-ol, le docosane-1-ol, le (9Z)-octadéc-9-ène-1-ol, (9E)-Octadec-9-en-1-ol, (9Z,12Z)-Octadeca-9,12-dien-1-ol, (9Z,12Z,15Z)-Octadeca-9,12,15-trien-1-ol, (9Z)-Eicos-9-en-1-ol, (5Z,8Z,11Z,14Z)-Eicosa-5, 8,11,14-tétraen-1-ol, (13Z)-docos-13-én-1-ol), (13E)-docosen-1-ol), 2-octyl-dodécanol, 2-hexyl-dodécanol et/ou 2-butyl-dodécanol.

13. Procédé selon l'une des revendications 1 à 12, **caractérisé en ce que** la deuxième composition (B) contient - par rapport au poids total de la composition (B) - un ou plusieurs alcools gras en C₁₂-C₃₀ (B2) en une quantité totale de 2,0 à 50,0 % en poids, de préférence de 3,0 à 40,0 % en poids, plus préférentiellement de 4,0 à 30,0 % en poids, encore plus préférentiellement de 5,0 à 20,0 % en poids et tout particulièrement de 6,0 à 15,0 % en poids.

14. Procédé selon l'une des revendications 1 à 13, **caractérisé en ce que** la deuxième composition (B) contient au moins un agent tensioactif, de préférence un agent tensioactif non ionique.

15. Procédé selon l'une des revendications 1 à 14, **caractérisé en ce que** la deuxième composition (B) contient au moins un tensioactif non ionique de formule (T-I), 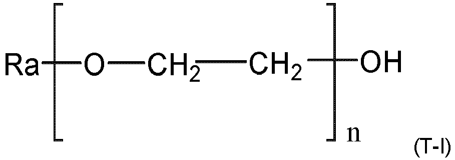 dans laquelle
Ra représente un groupe alkyle en C₈-C₂₄, saturé ou insaturé, linéaire ou ramifié, de préférence un groupe alkyle en C₁₆ à C₁₈, saturé et non ramifié, et
n représente un nombre entier de 10 à 40, de préférence un nombre entier de 20 à 35 et de manière particulièrement préférée le nombre 30.

16. Procédé selon l'une quelconque des revendications 1 à 15, **caractérisé en ce que** la deuxième composition (B) contient au moins un composé colorant choisi dans le groupe des pigments minéraux, choisi dans le groupe des oxydes métalliques colorés, des hydroxydes métalliques, des oxydes métalliques hydratés, des silicates, des sulfures métalliques, des cyanures métalliques complexes, des sulfates métalliques, des pigments de bronze, des métaux, des alliages métalliques et/ou des pigments colorés à base de mica ou de mica revêtus d'au moins un oxyde métallique et/ou d'un oxychlorure métallique.

17. Procédé selon l'une quelconque des revendications 1 à 16, **caractérisé en ce que** la composition (B) contient au moins un composé colorant (B3) appartenant au groupe des pigments organiques choisis dans le groupe constitué par le carmin, la quinacridone, la phtalocyanine, les pigments bleus ayant les numéros d'indice de couleur CI 42090, CI 69800, CI 69825, CI 73000, CI 74100, CI 74160, les pigments jaunes ayant les numéros d'indice de couleur CI 11680, CI 11710, CI 15985, CI 19140, CI 20040, CI 21100, CI 21108, CI 47000, CI 47005, des pigments verts avec les numéros Color Index CI 61565, CI 61570, CI 74260, des pigments oranges avec les numéros Color Index CI 11725, CI 15510, CI 45370, CI 71105, des pigments rouges avec les numéros Color Index CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915 et/ou CI 75470.

18. Procédé selon l'une quelconque des revendications 1 à 17, **caractérisé en ce que** la composition (B) comprend au moins un composé colorant (B3) choisi dans le groupe des pigments organiques parmi
- le pigment bleu portant le numéro d'indice de couleur CI 74160,
- le pigment jaune avec le numéro Color Index CI 11680, et
- le pigment rouge avec le numéro Color Index CI 12490.

19. Procédé selon l'une des revendications 1 à 18, **caractérisé en ce que** la deuxième composition (B) contient - par rapport au poids total de la composition (B) - un ou plusieurs composés colorants (B3) en une quantité totale de 0,1 à 6,0 % en poids, de préférence de 0,3 à 5,0 % en poids, plus préférentiellement de 0,5 à 4,0 % en poids et tout particulièrement de 0,7 à 1,5 % en poids.

20. Procédé selon l'une quelconque des revendications 1 à 19, **caractérisé en ce que** (3) le stockage de la composition (A) pendant une durée d'au moins 5 jours, de préférence d'au moins 10 jours, plus préférentiellement d'au moins 14 jours et tout particulièrement d'au moins 21 jours.

21. Procédé selon l'une quelconque des revendications 1 à 20, **caractérisé par** la (3) le stockage de la composition (B) pendant une durée d'au moins 24 heures, de préférence d'au moins 36 heures, plus préférentiellement d'au moins 48 heures.

22. Procédé selon l'une quelconque des revendications 1 à 21, **caractérisé par** le mélange des compositions (A) et (B) dans un rapport massique (A)/(B) de 1:20 à 20:1, de préférence de 1:5 à 1:20, et tout particulièrement de 1:6 à 1:14.

23. Procédé selon l'une quelconque des revendications 1 à 22, **caractérisé en ce que** le mélange de (A) et (B) est appliqué sur la matière kératinique dans un délai de 1 à 60 minutes, de préférence de 1 à 45 minutes et plus préférentiellement dans un délai de 1 à 30 minutes après son mélange.

24. Procédé selon l'une quelconque des revendications 1 à 23, **caractérisé en ce qu'**il consiste à (5) l'application du mélange de (A) et (B) sur la matière kératinique, en laissant agir le mélange sur la matière kératinique pendant une durée de 1 à 10 minutes, et en rinçant le mélange à l'eau.

25. Procédé selon l'une quelconque des revendications 1 à 24, comprenant .
(6) l'application d'une composition (C) sur la matière kératinique, ladite composition (C) comprenant:
(C1) au moins un polymère filmogène.
